# EUROPEAN PATENT SPECIFICATION

(11) **EP 3 493 682 B1**
(45) Date of publication and mention of the grant of the patent: **20.03.2024**
(21) Application number: 17836085.5
(22) Date of filing: 25.07.2017
(51) Int. Cl.: A01N 63/30, A61K 36/06, C12N 1/14, A01N 25/12, A23K 10/18, A23K 50/10, C12R 1/645, A61P 33/10

(54) **COMPOSITION COMPRISING DUDDINGTONIA FLAGRANS**
ZUSAMMENSETZUNG MIT DUDDINGTONIA FLAGRANS
COMPOSITION COMPRENANT DU DUDDINGTONIA FLAGRANS

(30) Priority: 05.08.2016 AU 2016903096
(43) Date of publication of application: 12.06.2019
(73) Proprietor: International Animal Health Products Pty Ltd, Huntingwood, New South Wales 2148 (AU)
(72) Inventor: HEALEY, Kevin, Ultimo, New South Wales 2007 (AU); LAWLOR, Chris, Kurmond, New South Wales 2575 (AU); KNOX, Malcolm, Armidale, New South Wales 2350 (AU)
(74) Representative: Vossius & Partner Patentanwälte Rechtsanwälte mbB
(86) International application number: PCT/AU2017/050764
(87) International publication number: WO 2018/023151

(56) References cited:
- WO-A1-2014/125154
- WO-A1-2017/125468
- CN-A- 105 343 879
- US-A- 5 643 568
- Anonymous: "BioWorma (Application Summary for Application No 106347)", AUSTRALIAN PESTICIDES & VETERINARY MEDICINES AUTHORITY, 20 July 2016 (2016-07-20), pages 1-40, XP055459672, Retrieved from the Internet: URL:https://archive.apvma.gov.au/applicati on_summaries/106347.pdf [retrieved on 2018-03-15]
- Anonymous: "Livamol with BioWorma (Application Summary for Application No 106348)", AUSTRALIAN PESTICIDES & VETERINARY MEDICINES AUTHORITY, 20 July 2016 (2016-07-20), pages 1-12, XP055459677, Retrieved from the Internet: URL:https://archive.apvma.gov.au/applicati on_summaries/106348.pdf [retrieved on 2018-03-15]
- Anonymous: "BioWorma and Livamol with BioWorma", EPA Application Summary APP202832, 3 August 2016 (2016-08-03), pages 1-19, XP055459684, Retrieved from the Internet: URL:https://www.epa.govt.nz/assets/FileAPI /hsno-ar/APP202832/APP202832-APP202832-Dec ision-FINAL-3August2016.pdf [retrieved on 2018-03-15]
- ANDRÉIA BUZATTI ET AL: "Duddingtonia flagrans in the control of gastrointestinal nematodes of horses", EXPERIMENTAL PARASITOLOGY, vol. 159, 22 July 2015 (2015-07-22), pages 1-4, XP055382311, US ISSN: 0014-4894, DOI: 10.1016/j.exppara.2015.07.006
- F.A. KHAN ET AL: "Evaluation of administering Duddingtonia flagrans through Complete Feed Block for Controlling Haemonchus contortus in Sheep", ANIMAL NUTRITION AND FEED TECHNOLOGY, vol. 15, no. 3, 1 January 2015 (2015-01-01), pages 447-456, XP055459692, ISSN: 0972-2963, DOI: 10.5958/0974-181X.2015.00045.1
- P.J WALLER ET AL: "The potential of nematophagous fungi to control the free-living stages of nematode parasites of sheep: feeding and block studies with Duddingtonia flagrans", VETERINARY PARASITOLOGY, vol. 102, no. 4, 1 December 2001 (2001-12-01), pages 321-330, XP055459697, NL ISSN: 0304-4017, DOI: 10.1016/S0304-4017(01)00542-8
- Anonymous: "Biological Control of Nematode Parasites of Small Ruminants in Asia - Final Proceedings of FAO Technical Co-operation Project in Malaysia TCP/MAL/0065 (T) 2002", Food and Agriculture Organization of the United Nations, 16 September 2002 (2002-09-16), pages 1-11, XP055589920, Retrieved from the Internet: URL:http://helminto.inta.gob.ar/pdf%20alte rnativos/fao_tcp_mal_0065(t)1.pdf [retrieved on 2019-05-20]
- BURKE J M ET AL: "Interaction between copper oxide wire particles and Duddingtonia flagrans in lambs", VETERINARY PARASITOLOGY, ELSEVIER SCIENCE, AMSTERDAM, NL , vol. 134, no. 1-2 25 November 2005 (2005-11-25), pages 141-146, XP027655618, ISSN: 0304-4017, DOI: 10.1016/J.VETPAR.2005.06.018 Retrieved from the Internet: URL:https://www.sciencedirect.com/science/ article/pii/S0304401705003419?via%3Dihub [retrieved on 2005-08-08]
- Anonymous: "BioWorma (Application Summary for Application No 106347)", AUSTRALIAN PESTICIDES & VETERINARY MEDICINES AUTHORITY, 20 July 2016 (2016-07-20), pages 1-40, XP055459672, Retrieved from the Internet: URL:https://archive.apvma.gov.au/applicati on_summaries/106347.pdf [retrieved on 2016-07-20]
- Anonymous: "Livamol with BioWorma (Application Summary for Application No 106348)", AUSTRALIAN PESTICIDES & VETERINARY MEDICINES AUTHORITY, 20 July 2016 (2016-07-20), pages 1-12, XP055459677, Retrieved from the Internet: URL:https://archive.apvma.gov.au/applicati on _ summaries/106348.pdf [retrieved on 2016-07-20]
- Anonymous: "BioWorma and Livamol with BioWorma", EPA Application Summary APP202832, 3 August 2016 (2016-08-03), pages 1-19, XP055459684, Retrieved from the Internet: URL:https://www.epa.govt.nz/assets/FileAPI /hsno-ar/APP202832/APP202832-APP202832-Dec ision-FINAL-3August2016.pdf [retrieved on 2016-08-03]
- BUZATTI, A. et al.: "Duddingtonia flagrans in the control of gastrointestinal nematodes of horses", Experimental Parasitology, vol. 159, 2015, pages 1-4, XP055382311,
- KHAN, F.A. et al.: "Evaluation of administering Duddingtonia flagrans through complete feed block for controlling Haemonchus contortus in sheep", Animal Nutrition and Feed Technology, vol. 15, 2015, pages 447-456, XP055459692,
- WALLER, P.J. et al.: "The potential of nematophagous fungi to control the free-living stages of nematode parasites of sheep: feeding and block studies with Duddingtonia flagrans", Veterinary Parasitology, vol. 102, 2001, pages 321-330, XP055459697,
- Anonymous: "Biological Control of Nematode Parasites of Small Ruminants in Asia - Final Proceedings of FAO Technical Co-operation Project in Malaysia TCP/MAL/0065 (T) 2002", Food and Agriculture Organization of the United Nations, 16 September 2002 (2002-09-16), pages 1-11, XP055589920, Retrieved from the Internet: URL:http://helminto.inta.gob.ar/pdf%20alte rnativos/fao_tcp_mal_0065(t)1.pdf [retrieved on 2017-08-11]
- BURKE, J.M. et al.: "Interaction between copper oxide wire particles and Duddingtonia flagrans in lambs", Veterinary Parasitology, vol. 134, 25 November 2005 (2005-11-25), pages 141-146, XP027655618,
- SANTURIO, J.M. et al.: "Improved method for Duddingtonia flagrans chlamydospores production for livestock use", Veterinary Parasitology, vol. 164, 2009, pages 344-346, XP055459724,
- WALLER, P.J.: "Sustainable nematode parasite control strategies for ruminant livestock by grazing management and biological control", Animal Feed Science and Technology, vol. 126, no. 3-4, 9 March 2006 (2006-03-09), pages 277-289, XP024934229,
- RAWTE, D. et al.: "Strain improvement of predacious fungi: screening for the ability of mutant strains of Duddingtonia flagrans to withstand ruminant gut passage", Journal of Veterinary Parasitology, vol. 26, no. 2, 2012, pages 104-107, XP9513377,
- LARSSON, A. et al.: "A 3-year field evaluation of pasture rotation and supplementary feeding to control parasite infection in first-season grazing cattle - Effects on animal performance", Veterinary Parasitology, vol. 142, no. 3-4, 2006, pages 197-206, XP027887855,

## Description

### Field of the Invention

The present invention relates to a composition comprising a biologically pure isolate of the micro-organism *Duddingtonia flagrans (D. flagrans)* having all of the identifying characteristics of the strain deposited under Accession No. V16/019156 at National Measurement Institute, 1/153 Bertie Street, Port Melbourne, Victoria 3207, Australia referred to herein as "IAH 1297", the production thereof by fermentation on a solid substrate that is a moistened grain, legume or oilseed, and the use thereof in a method of controlling the spread of a parasitic nematode in a grazing animal.

### Background of the Invention

Parasitic nematodes (intestinal worms) remain a major threat to the health and welfare of grazing animals (with a digestive system that is highly adapted to a grass diet) all over the world. In particular, grazing animals are exposed to parasites at very high frequency. Grazing animals become re-infected after treatment because they pick up nematodes by grazing on pasture which is infected with worm larvae. These larvae originate from the manure piles containing large numbers of nematode eggs deposited by wormy animals.

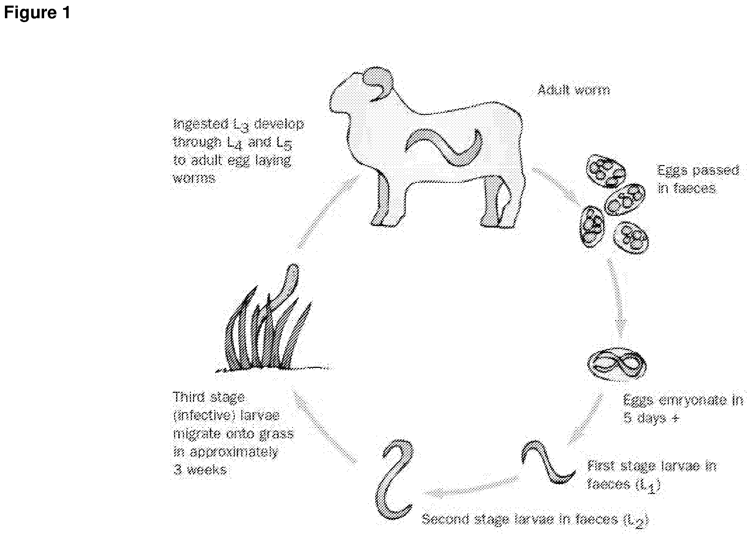

More particularly, as shown in Figure 1, the basic lifecycle of gastrointestinal nematodes (GIN) involve adult worms in the predilection site in the host's gastrointestinal tract producing large numbers of eggs that are passed from the host i.e., shed in the faeces. In the faecal pats or on the pasture the eggs typically undergo further development through two feeding stages (this development period varies between the different parasite species and is dependent on environmental conditions). The first stage larvae (L1) molt into second stage larvae (L2) and then into non-feeding infective third stage larvae (L3). The L1 and L2 larvae feed on organic matter in the faeces whereas the L3 larvae are unable to feed due to the retention of the cuticle from the L2 stage. The cuticle serves as a protective sheath from extreme environmental conditions and can extend the survival of the larvae until optimal conditions are present for further development (Pugh, D.G. et al., Comp Cont Educ Pract Vet 20 (1998): S112). The infective L3 subsequently migrate from the faeces onto surrounding pasture, where they are ingested by other grazing animals, thereby propagating the parasitic infection. Once inside the host, the L3 ex-sheath in response to changes in CO₂ concentration, temperature and pH before they reach their site of infection. They undergo two further molts (L4 and L5) and complete their development to adults at their preferred site in 15 - 21 days for most common species (Vlassoff, A. et al., (2001) New Zealand Veterinary Journal 49: p213-221).

During migration and establishment of the larvae/adults direct damage may be caused to the digestive tract. Damage may also be caused by inflammation at the site of infection as the host responds to the invading parasite. Several worldwide reports have suggested that such parasitic diseases inflict severe economic losses on the livestock industry and adversely affect the health of animals. Diarrhoea, anaemia, weight loss, decreased reproduction, decreased production (e.g. wool quality, milk production, etc.), and increased morbidity and mortality are just some of the devastating losses that gastrointestinal nematodes (GIN) can create. In the domestic small ruminant industry alone worldwide, there is an estimated production loss extending into the millions of dollars each year. Exact values are difficult to obtain due to the many factors that affect loss which include management, nutrition, environmental factors, stress, genetics and concurrent disease (Vlassoff, A. et al., (2001) New Zealand Veterinary Journal 49: p213-221). The estimated losses in sheep and cattle in Australia alone is $A1 billion and worldwide it extends to tens of billions of dollars (Roeber et al., (2013) Parasite & Vectors 6: p153). In another study published by Meat & Livestock Australia Limited (Lane et al., (2015) Report B.AHE.0010 ISBN 9781741918946) estimates of the total losses per annum in Australia due to internal parasites were:
Cattle $A93.6 million ($US70m/$EU66m) with production losses ranging from $0.44 - $3.59 per animal
Sheep $A436 million ($US327m/$EU305m) with production losses ranging from $1.29 - $28.29 per animal
Goats $A2.54 million ($US1.9m/$EU1.8m) with production losses ranging from $0 - $5.34 per animal.

Exotic or nondomestic ruminant species such as giraffe, antelope, deer, oryx, sable, blackbuck, bongo, okapi, and wildebeest are a few of the many species of artiodactylids that also exhibit GIN infections. GIN infections are not as severe and numerous in wild artiodactylids in their natural habitats as they are in captive artiodactylids in zoological facilities. Reasons for increased numbers of GIN and severity of infection in captive artiodactylids are due to stress, the lack of browse in enclosures, increased stocking rates, irrigation and the inability to close exhibits. Stressful conditions caused by captivity can decrease the function of the immune system and increase the body's exposure to parasites and diseases (Fagiolini, M. (2010) et al., Journal of Zoo and Wildlife Medicine, 41(4): p662-670). In zoological facilities, guests must be able to view animals in their enclosures. For that reason, there is a decreased availability of browse, which allows animals that are natural browsers in the wild, such as giraffe, to graze instead. However, it is difficult to predict the extent of infection in such forced "grazers" compared to domestic grazing animals, as it would depend upon the extent of actual grazing compared to browsing provided in such conditions.

Globally, among the most important gastrointestinal nematode (GIN) parasites of livestock include those which belong to the genera *Haemonchus, Ostertagia, Teladorsagia, Trichostrongylus, Ascaris, Nematodirus, Strongyloides, Trichuris, Chabertia, Oesophagostomum, Cooperia* and *Bunostomum* (Yong, W. K. 1992. In: Yong, W. K (Ed.) Animal Parasite Control Utilizing Biotechnology. pp. 115-134. CRC Press, Florida, USA). For ruminants, the major species are *Haemonchus* spp., *Teladorsagia* (*Ostertagia*) spp., *Cooperia* spp., and *Trichostrongylus* spp. while for horses the major species are cyathostomes, *Strongylus* spp., *Strongyloides westeri, Parascarus equorum, Trichostrongylus axei* and *Dictyocaulis arnfeldi.* The adult nematode will preferentially inhabit a particular segment of the host gastrointestinal tract (see Table 1).

**Table 1 The important nematodes of grazing animals in Australia, their common names and the anatomical site in which they are located**

| Anatomical site | Scientific name | Common name |
|---|---|---|
| Abomasum (ruminants) | *Haemonchus contortus* | barbers pole worm |
| | *H. placei* | barbers pole worm |
| | *Teladorsagia* (Ostertagia) spp. *Trichostrongylus axei* | small brown stomach worm stomach hair worm |
| Small intestine | *Cooperia* spp. | intestinal worm |
| | *Nematodirus* spp. | thin necked intestinal worm |
| | *Trichostrongylus* spp. | black scour worm |
| | *Bunostomum trigonocephalum* | hook worm |
| | *Strongyloides papillosis* | thread worm |
| | *Strongyloides westeri* | thread worm |
| | *Parascarus equorum* | round worms |
| Large intestine | *Chabertia ovina* | large mouthed bowel worm |
| | *Oesophagostomum columbianum* | nodule worm |
| | *Oe. Vendosum* | - |
| | *Trichuris ovis* | whipworm |
| | *Cyathostomins* | small strongyles |
| | *Strongylus* spp. | large strongyles |
| | *Oxyuris equi* | pin worms |

Of these parasitic species, *H. contortus,* a parasite of the abomasum of ruminants, is very significant with respect to global economic impact and clinical disease because it is a blood feeding parasite that can cause severe anaemia in animals and is prevalent around the world where there is a mean monthly temperature of 18°C and rainfall of 50mm. This particular parasite, for example, is endemic to the South-eastern United States, Europe and Australasia as well as other areas that have climates of high temperature and humidity. https://www.researchgate.net/publication/6904034_Haemonchus_contortus_parasite_proble m_No_1_from_tropics_-_Polar_Circle_Problems_and_prospects_for_control_based_on_epidemiology.

Presently, the conventional approach to management of intestinal parasites in grazing animals is chemotherapy i.e. administration of chemical agents intended to kill the parasitic nematodes which cause disease. These chemicals are known as anthelmintics. This approach has been used with great success around the world but has led to over-reliance on these drugs which, in turn, has led to the evolution of drug-resistance which is undermining the treatment paradigm.

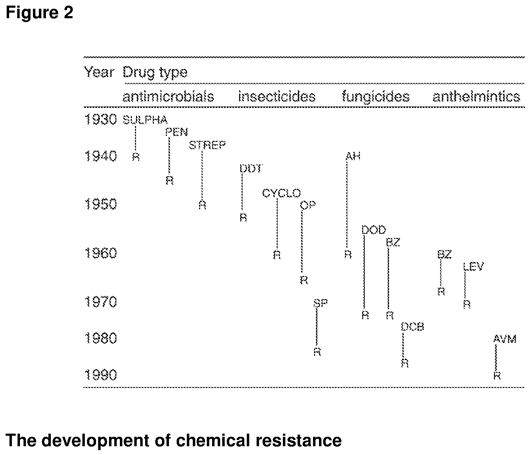

The 1960s and 1970s, when the regime of chemotherapy was established, was a period in which the general availability of drugs and the emergence of new drugs flourished. However, the pipeline has dried-up and the outlook for further innovations in drug therapy is bleak (Ihler, C. F., (2010) Acta Veterinaria Scandinavica, 52 (Suppl 1):S24). Generally, resistance to a new chemical becomes evident after just 5-10 years of use, as shown in the in Figure 2. There have been recent cases of resistance developing at a faster pace. For example, in 2013 it was reported that monepantel (a new anthelmintic drug) had become completely ineffective against *Teladorsagia circumcincta* and *Trichostrongylus colubriformis* after just two years use on a goat farm in New Zealand (Scott, I. et al, (2013) Veterinary Parasitology 198 (1-2) 166-171. Due to varying compliance of orally administered medications, estimated body weights, as well as unknown pharmacokinetic data, has led to the use of sub-therapeutic doses. This, along with overuse and poor pasture management has contributed to the current anthelmintic resistance problem worldwide.

Administration of the chemicals also gives rise to chemical residues in the meat and milk products obtained from the recipient animals. It also causes unintended contamination of the environment through spillages and excretion in urine and faeces. Although resistance in parasites is increasing, commercial farming continues to rely on the use of anthelmintics.

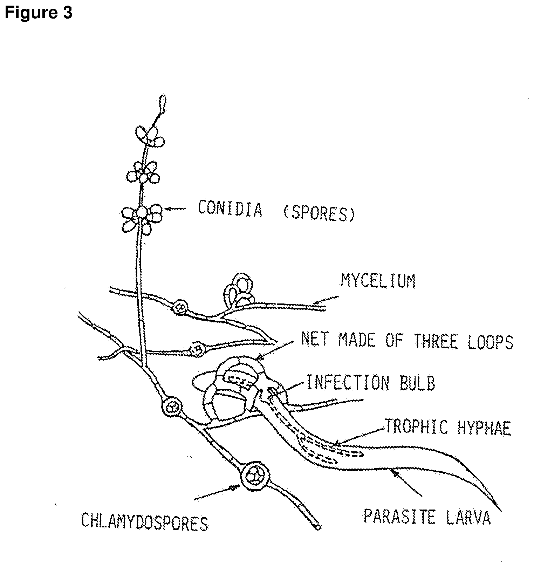

In view of the above problems with anthelmintic use, there remains an urgent need for other control methods in commercial settings e.g., on farms and/or zoos. Biological control methods provide a means for chemical-free control of parasitic nematodes by utilising the capabilities of microbial agents which have biological properties adapted for this purpose. Nematophagous fungi have emerged in recent years as promising biological control agents of parasitic nematodes of livestock (Larsen et al., (1997) Veterinary Parasitology 72: p479-85; Waller and Faedo, (1996) International Journal for Parasitology 26: p915-925). Many studies have shown *Duddingtonia flagrans (D. flagrans)* to have potential as a control agent. D. *flagrans* is a nematophagous fungus that is adapted to trapping and digesting the infective larvae of nematode (worm) species, which are parasitic to grazing animals. The spores of the fungus are particularly thick-walled and so when fed to grazing animals, can pass through their digestive tracts intact and are deposited in their manure. In particular, *D. flagrans* produces large chlamydospores (approx. 20 µm), which survive gut passage. As discussed above, the animals also shed the eggs of their parasites into their manure. In the manure pats, the fungus proliferates, traps and consumes the infective larvae that hatch from the worm eggs, as illustrated in Figure 3. This interrupts the worms' life cycle and thus, *D. flagrans* was considered to be potentially useful as a biological control for parasitic worms of grazing animals.

Despite the vast research that has been conducted on the use of *D. flagrans* in the biological control of parasitic worms in the last 20 years (Buzatti et al., (2015) Experimental Parasitology 159: p1-4; Khan et al., (2015) Animal Nutrition and Feed Technology 15(3): p447-456; Waller et al. (2001) Veterinary Parasitology 102(4): p321-330; Burke et al. (2005) Veterinary Parasitology 134(1-2): p141-146; US 5643568 A; WO 2014/125154 A1; CN 105343879 A; Anonymous: "Biological Control of Nematode Parasites of Small Ruminants in Asia - Final Proceedings of FAO Technical Co-operations Project in Malaysia TCP/MAL/0065 (T) 2002", Food and Agriculture Organization of the United Nations, (2002-09-16), pages 1-11.), the outcomes in this area have proved disappointing, so much so that no commercial products have emerged. In particular, a plethora of feeding studies using a variety of dosages of *D. flagrans* highlight numerous problems associated with the development of a commercial product comprising *D. flagrans* suitable for use in grazing animals. A study conducted in 2005 highlights the inconsistencies in results obtained from field trials, describing a high variability in efficacy obtained with voluntary ingestion, which may accentuate differences in efficacy between doses and which is a major pitfall with environmental use despite efficacy observed in only some groups (Paraud etal., Vet. Res. (2005) 36: p157-166). Waller et al. Acta. Vet. Scand. (2006) 47: p23-32) describe trials conducted on three commercial sheep farms in Sweden to evaluate the administration of *D. flagrans* under a range of commercial farming operations and whether or not commercial application of a biological product comprising *D. flagrans* would be feasible. These trials showed that there was no significant benefit in performance of lambs in the fungal treatment for all three farms used in this study.

It has further been reported by Terrill et al. (Veterinary Parasitology 186 (2012): p28-37) that work with nematode trapping fungi was discontinued by the Southern Consortium for Small Ruminant Parasite (SCSRPC) after an encouraging start because of a lack of commercial source of the spores. Moreover, whether the use of *D. flagrans* is feasible under grazing conditions with consistent reproducible efficacy, along with long-term use to reduce the gastrointestinal nematode larval numbers on pasture (thereby reducing infection rates and improving animal performance), remains to be seen. Indeed a further review published by Kumar et al., (J. Parasit. Dis. (2013) 37(2): p151-157) indicates there is an urgent need to design a sustainable parasite control strategy. Moreover, whilst recent experimental studies describe *D. flagrans* as being the "most promising" biological control agent for nematode parasites in grazing animals, researchers have begun to study combinations of *D. flagrans* with other nematophagous fungi (e.g., see Da Silva et al. Biomed Res. Intl. (2015) Article ID 474879). However, such combinations have yet to be tested in commercial farm settings and/or zoos and are likely to present even greater problems of reproducibility and efficacy under the varying field conditions compared with the use of one biological agent alone.

As discussed above, the use *D. flagrans* to control infective larvae on the pasture showing consistent reproducible efficacy has met with much difficulty due to high variance between results obtained under experimental conditions, those obtained in field trials and under commercial farm settings, as well as the difficulties associated with fermentation and scale up to produce commercial quantities of a usable product i.e., the spores. Accordingly, despite the existence of numerous studies describing the potential to use of *D. flagrans* as a biological control agent none of the strains used have successfully led to a commercially available product. No specific information about strain IAH 1297 are available (e.g., how to derive the strain or what are the characterizing properties) (Anonymous: "BioWorma (Application Summary for Application No 106347)", Australian Pesticides & Veterinary Medicines Authority, (2016-07-20), pages 1-40, Anonymous: "Livamol with BioWorma (Application Summary for Application No 106348)" Australian Pesticides & Veterinary Medicines Authority, 20 July 2016 (2016-07-20), pages 1-12, Anonymous: "BioWorma and Livamol with BioWorma", EPA NZ Decision APP202832, 3 August 2016 (2016-08-03), pages 1-19). There remains a need for a composition comprising *D. flagrans* that provides reproducible control of the spread and infectivity of livestock and pasture by parasitic nematodes and that is suitable for commercialisation and/or applicable for use in a sustainable commercial parasite control strategy.

Any discussion of the prior art throughout the specification should in no way be considered as an admission that such prior art is widely known or forms part of common general knowledge in the field.

It is an objective of the present invention to overcome or ameliorate at least one of the disadvantages of the prior art treatments, or to provide a useful alternative.

### Summary of the Invention

Despite the lack of success in the art with attempts to produce a biological control product comprising *D. flagrans,* in work leading up to the present invention, the inventors sought to identify an isolate of a *D. flagrans* strain for use in a biological control composition suitable for commercial application. The inventors have surprisingly found an isolated *D. flagrans* strain, referred to in the present application as "IAH 1297", that has characteristics suitable for commercial application. The *D. flagrans* strain of the present application provides numerous advantages over known strains as discussed below.

The present invention is directed to the embodiments as defined in the claims. The present invention provides a composition comprising a biologically pure isolate of the microorganism *Duddingtonia flagrans (D. flagrans)* having all of the identifying characteristics of the strain deposited under Accession No. V16/019156 at National Measurement Institute, 1/153 Bertie Street, Port Melbourne, Victoria 3207, Australia on 2 August 2016 (referred to herein as "strain IAH 1297").
The present invention further relates to a composition for use in a method of controlling the spread of a parasitic nematode in a grazing animal comprising a biologically pure isolate of the micro-organism *Duddingtonia flagrans (D. flagrans)* having all of the identifying characteristics of the strain deposited under Accession No. V16/019156 at National Measurement Institute, 1/153 Bertie Street, Port Melbourne, Victoria 3207, Australia referred to herein as "IAH 1297".
In addition, the present invention relates to a method of producing a biologically pure isolate of the micro-organism *Duddingtonia flagrans (D. flagrans)* by fermentation the method comprising fermenting an isolate of *D. flagrans* having all of the identifying characteristics of the strain deposited under Accession No. V16/019156 at National Measurement Institute, 1/153 Bertie Street, Port Melbourne, Victoria 3207, Australia referred to herein as "IAH 1297" on a solid substrate, wherein the solid substrate is a moistened grain, legume or oilseed.

It will be apparent to a person skilled in the art that the term "biologically pure isolate" as used herein means that the isolate is separated from an environment of one or more constituents with which it may be associated if found in nature or otherwise. The terms "biologically pure" or "isolate" as used herein does not indicate the extent to which the microorganism has been purified. In one embodiment, strain IAH 1297 is the only D. *flagrans* strain present in the isolate and in another embodiment, the strain IAH 1297 is the only microorganism present in the isolate.

It will be clear to the skilled addressee that the present invention relates to a composition comprising the strain IAH 1297 in any of its life forms (see discussion above regarding the various life-cycle stages of *D. flagrans*). Preferably, strain IAH 1297 is in the form of a spore and more preferably it is in the form of a chlamydospore.

The composition according to the invention may comprise any functional part of the *D. flagrans* microorganism strain IAH 1297, as described herein, including a spore. Preferably, the composition comprises a spore of said strain IAH 1297 and, more preferably, the spore is in the form of a chlamydospore.

The present invention provides a composition comprising an isolated *D. flagrans* strain IAH 1297 and/or a spore of said isolated *D. flagrans* strain (preferably a chlamydospore of said isolated *D. flagrans* strain), wherein said composition interrupts the life-cycle of a parasitic nematode in a grazing animal, controls the spread of the nematode in a grazing animal and is suitable for commercial application.

The compositions of the present invention are useful as biological control compositions to interrupt the life-cycle of a parasitic nematode that infects a grazing animal. The biological control compositions control the spread of the nematode and are suitable for commercial application.

Without being bound to any particular theory, it is thought that when a composition of the present invention is fed to a grazing animal, the thick-walled spore of *D. flagrans* (preferably the chlamydospore) remains inert, resists digestion and passes through the digestive tract and is eventually shed into manure. The *D. flagrans* spores of the composition germinate in the manure and form trapping organs trapping the larvae of the parasitic nematode and killing them thereby interrupting the life-cycle of the parasitic nematode in a grazing animal. This controls the spread of the nematode from the manure onto the pasture by reducing the number of larvae emerging from manure. By controlling the emergence of larvae from the manure, the composition of the invention controls the spread of parasitic nematodes of grazing animals thereby reducing infectivity of the parasitic nematode. The larvae include e.g., L1, L2 and/or L3 larvae. Preferably, the composition reduces L3 larvae emerging from manure and/or on the pasture. Accordingly, it will be apparent to the skilled artisan that the term "controls the spread of the nematode" includes controlling the spread of parasitic nematodes of grazing animals. It will also be understood that controlling the spread of a parasite in a grazing animal means reducing the infectivity by the parasitic nematode whereby the population of the pathogen is below a threshold level where clinical problems and/or economic losses of livestock (the grazing animals) are reduced.

For example, the composition according to the invention interrupts the life-cycle of a parasitic nematode in a grazing animal and controls the spread of the nematode when the reduction of larvae in manure and/or onto pasture is reduced in the range of 30% to 100%, or 35% to 100%, or 40% to 100%, or 45% to 100%, or 50% to 100%, or 55% to 100%, or 60% to 100%, or 65% to 100%, or 70% to 100%, or 75% to 100%, or 80% to 100%, or 85% to 100%, or 90% to 100%, or 95% to 100%, or about 100%. Preferably, the composition according to the invention interrupts the life-cycle of a parasitic nematode in a grazing animal and controls the spread of the nematode when the reduction of larvae in manure and/or onto pasture is reduced in the range of 50% to 100%, more preferably in the range of 70 to 100%, even more preferably in the range of 80 to 100%. In one example, the composition according to the invention interrupts the life-cycle of a parasitic nematode in a grazing animal and controls the spread of the nematode when the reduction of larvae in manure and/or onto pasture is reduced in the range of about 50%, or 55%, or 60%, or 65%, or 70%, or 75%, or 80%, or 85%, or 90%, or 95%, or 100%.

The composition of the invention is also one that is suitable for commercial application i.e., application to animals held on a farm including large commercial farms, and/or animals held in an enclosure and/or zoo setting. Accordingly, it will be understood that the composition of the invention is suitable for commercial application and may be fed directly to the grazing animal and/or by mixing into feed and/or provided with a feed supplement, or the composition of the invention may be formulated further and provided as a feed, feed supplement, bolus or veterinary medicinal composition. The feed or feed supplement may be in any form suitable for feeding grazing animals including, but not limited to; a loose mix, a liquid feed/supplement, pellet or lick block.

It is preferable that the composition of the invention is a commercial product having at least one or more of the following characteristics:
Characteristic 1: Able to be produced in sufficiently large quantities to allow deployment into commercial grazing systems
Characteristic 2: Efficacy after large-scale production and performance on-farm and/or animals held in an enclosure and/or zoo setting in accordance with the label claims.
Characteristic 3: Safety for recipient animals and their human handlers.
Characteristic 4: Stability and shelf life. Preferably, the biological control product has a shelf life of at least 12 months, or at least 18 months or longer at room temperature (up to 30°C).
Characteristic 5: Suitable physical form. The physical form of the product should be suited to its intended end use i.e. free flowing and easy to blend with animal feed.
Characteristic 6: Palatability - Since the products are intended for routine oral administration in-feed, a taste and texture which is acceptable to the recipient animals.
Characteristic 7: Packaging / pack sizes - it is desirable to have robust packaging, easy to handle on farm and pack sizes appropriate for the end-use.
Characteristic 8: Purity - the microbial agents should be produced in a form sufficiently pure for safe administration to animals.
Characteristic 9: Suitability for use in either individual feeding or group feeding situations. Sometimes, grazing animals will be fed on an individual basis. More generally, grazing animals are fed in a group feeding situation such as a shared trough. This can lead to situations where feed intake of timid animals is inhibited and more aggressive animals can consume more than their share.

Despite significant research with *D. flagrans,* until the present invention, a commercial product has not been provided that has at least the characteristics 1 and 2 above. In one embodiment, the composition comprises at least two, or at least three, or at least four, or at least five, or at least six, or at least seven, or at least eight or at least nine of the above characteristics 1 to 9.

Preferably, the composition comprises each of the above characteristics 1, 2, 3, 6 and 7. Even more preferably, the composition comprises each of the above characteristics 1 to 9.

Efficacy after large scale production and performance on farm and/or animals held in an enclosure and/or zoo in accordance with the label claims in-part relates to the amount of viable spores of *D*. *flagrans* in the composition. As such, the composition according to the invention may comprise a viable count of *D*. *flagrans* spores of at least about 1 × 10² viable spores per gram of composition.

In one embodiment, the composition according to the invention may comprise a viable count of *D. flagrans* spores in the range of 1 × 10² to 1 × 10¹⁰ viable spores per gram of composition. For example, the composition according to the invention may comprise a viable count of *D*. *flagrans* spores of:
1 × 10² to 1 × 10³ viable spores per gram of composition, or
1 × 10³ to 1 × 10⁴ viable spores per gram of composition, or
1 × 10⁴ to 1 × 10⁵ viable spores per gram of composition, or
1 × 10⁵ to 1 × 10⁶ viable spores per gram of composition, or
1 × 10⁶ to 1 × 10⁷ viable spores per gram of composition, or
1 × 10⁷ to 1 × 10⁸ viable spores per gram of composition, or
1 × 10⁸ to 1 × 10⁹ viable spores per gram of composition, or
1 × 10⁹ to 1 × 10¹⁰ viable spores per gram of composition.

Preferably, the composition according to the invention may comprise a viable count of *D. flagrans* spores in the range of 1 × 10² to 1 × 10⁸ viable spores per gram of composition.

In another embodiment, the composition according to the invention may comprise a viable count of *D. flagrans* spores of
(a)
   1 × 10² to 5 × 10⁶ viable spores per gram of composition, or
   1 × 10² to 1 × 10⁶ viable spores per gram of composition, or
   1 × 10³ to 1 × 10⁶ viable spores per gram of composition, or
   1 × 10³ to 5 × 10⁵ viable spores per gram of composition, or
   1 × 10⁴ to 5 × 10⁵ viable spores per gram of composition, or
   1 × 10⁴ to 1 × 10⁵ viable spores per gram of composition, or
(b)
   1 × 10⁴ to 1 × 10⁸ viable spores per gram of composition, or
   1 × 10⁴ to 5 × 10⁷ viable spores per gram of composition, or
   1 × 10⁴ to 1 × 10⁷ viable spores per gram of composition, or
   1 × 10⁵ to 5 × 10⁶ viable spores per gram of composition, or
   1 × 10⁵ to 1 × 10⁶ viable spores per gram of composition, or
(c)
   1 × 10⁵ to 1 × 10⁸ viable spores per gram of composition, or
   1 × 10⁵ to 1 × 10⁷ viable spores per gram of composition, or
   1 × 10⁶ to 1 × 10⁷ viable spores per gram of composition.

In another example, the composition according to the invention may comprise a viable count of *D. flagrans* spores of
(a) about 1 × 10², or about 1.5 × 10², or about 2 × 10², or about 2.5 × 10², or about 3 × 10², or about 3.5 × 10², or about 4 × 10², or about 4.5 × 10², or about 5 × 10², or about 5.5 × 10², or about 6 × 10², or about 6.5 × 10², or about 7 × 10², or about 7.5 × 10², or about 8 × 10², or about 8.5 × 10², or about 9 × 10², or about 9.5 × 10²; or;
(b) about 1 × 10³, or about 1.5 × 10³, or about 2 × 10³, or about 2.5 × 10³, or about 3 × 10³, or about 3.5 × 10³, or about 4 × 10³, or about 4.5 × 10³, or about 5 × 10³, or about 5.5 × 10³, or about 6 × 10³, or about 6.5 × 10³, or about 7 × 10³, or about 7.5 × 10³, or about 8 × 10³, or about 8.5 × 10³, or about 9 × 10³, or about 9.5 × 10³; or;
(c) about 1 × 10⁴, or about 1.5 × 10⁴, or about 2 × 10⁴, or about 2.5 × 10⁴, or about 3 × 10⁴, or about 3.5 × 10⁴, or about 4 × 10⁴, or about 4.5 × 10⁴, or about 5 × 10⁴, or about 5.5 × 10⁴, or about 6 × 10⁴, or about 6.5 × 10⁴, or about 7 × 10⁴, or about 7.5 × 10⁴, or about 8 × 10⁴, or about 8.5 × 10⁴, or about 9 × 10⁴, or about 9.5 × 10⁴; or
(d) about 1 × 10⁵, or about 1.5 × 10⁵, or about 2 × 10⁵, or about 2.5 × 10⁵, or about 3 × 10⁵, or about 3.5 × 10⁵, or about 4 × 10⁵, or about 4.5 × 10⁵, or about 5 × 10⁵, or about 5.5 × 10⁵, or about 6 × 10⁵, or about 6.5 × 10⁵, or about 7 × 10⁵, or about 7.5 × 10⁵, or about 8 × 10⁵, or about 8.5 × 10⁵, or about 9 × 10⁵, or about 9.5 × 10⁵; or
(e) about 1 × 10⁶, or about 1.5 × 10⁶, or about 2 × 10⁶, or about 2.5 × 10⁶, or about 3 × 10⁶, or about 3.5 × 10⁶, or about 4 × 10⁶, or about 4.5 × 10⁶, or about 5 × 10⁶, or about 5.5 × 10⁶, or about 6 × 10⁶, or about 6.5 × 10⁶, or about 7 × 10⁶, or about 7.5 × 10⁶, or about 8 × 10⁶, or about 8.5 × 10⁶, or about 9 × 10⁶, or about 9.5 × 10⁶, or
(f) about 1 × 10⁷, or about 1.5 × 10⁷, or about 2 × 10⁷, or about 2.5 × 10⁷, or about 3 × 10⁷, or about 3.5 × 10⁷, or about 4 × 10⁷, or about 4.5 × 10⁷, or about 5 × 10⁷, or about 5.5 × 10⁷, or about 6 × 10⁷, or about 6.5 × 10⁷, or about 7 × 10⁷, or about 7.5 × 10⁷, or about 8 × 10⁷, or about 8.5 × 10⁷, or about 9 × 10⁷, or about 9.5 × 10⁷, or
(g) about 1 × 10⁸, or about 1.5 × 10⁸, or about 2 × 10⁸, or about 2.5 × 10⁸, or about 3 × 10⁸, or about 3.5 × 10⁸, or about 4 × 10⁸, or about 4.5 × 10⁸, or about 5 × 10⁸, or about 5.5 × 10⁸, or about 6 × 10⁸, or about 6.5 × 10⁸, or about 7 × 10⁸, or about 7.5 × 10⁸, or about 8 × 10⁸, or about 8.5 × 10⁸, or about 9 × 10⁸, or about 9.5 × 10⁸, or
(h) about 1 × 10⁹, or about 1.5 × 10⁹, or about 2 × 10⁹, or about 2.5 × 10⁹, or about 3 × 10⁹, or about 3.5 × 10⁹, or about 4 × 10⁹, or about 4.5 × 10⁹, or about 5 × 10⁹, or about 5.5 × 10⁹, or about 6 × 10⁹, or about 6.5 × 10⁹, or about 7 × 10⁹, or about 7.5 × 10⁹, or about 8 × 10⁹, or about 8.5 × 10⁹, or about 9 × 10⁹, or about 9.5 × 10⁹, or about 1 × 10¹⁰
viable spores per gram of composition.

More preferably, the composition according to the invention may comprise a viable count of *D. flagrans* spores of
(a) about 1 × 10⁴, about 3 × 10⁴, or about 1 × 10⁵ viable spores per gram of composition; or
(b) about 1 × 10⁵, about 5 × 10⁵ or about 1 × 10⁶ viable spores per gram of composition; or
(c) about 1 × 10⁶, about 3 × 10⁶ or about 1 × 10⁷ viable spores per gram of composition; or
(d) about 3 × 10⁶, about 1 × 10⁷ or about 1 × 10⁸ viable spores per gram of composition.

The composition is prepared according to any method suitable to produce the *D. flagrans* strain IAH 1297 and/or spore of said isolated *D. flagrans* strain. In addition, the present invention provides a method of producing a biologically pure isolate of the micro-organism *Duddingtonia flagrans (D. flagrans)* by fermentation the method comprising fermenting an isolate of *D. flagrans* having all of the identifying characteristics of the strain deposited under Accession No. V16/019156 at National Measurement Institute, 1/153 Bertie Street, Port Melbourne, Victoria 3207, Australia referred to herein as "IAH 1297" on a solid substrate, wherein the solid substrate is a moistened grain, legume or oilseed. Preferably the spore is a chlamydospore. Preferably, the method is a fermentation method that provides the *D. flagrans* viable spores per gram of composition according to the invention. In one example, the *D. flagrans* strain and/or spore of said isolated *D. flagrans* strain is produced by a solid substrate fermentation method, wherein the solid substrate is a moistened wholegrain, legume or oilseed. The wholegrain used may be any suitable wholegrain useful for such a purpose including, but not limited to, barley, rye, millet, rice, wheat, sorghum, triticale, oat, buckwheat, amaranth, corn, quinoa, teff or lupin. The solid substrate may also include moistened legume including, but not limited to, chickpea, faba/broadbean, field pea, lentil,lupin or mungbean; or the substrate may be oilseed including, but not limited to soybean, canola, safflower, sunflower and sesame seed. For example, the method may be the method described in Examples 2 and 3.

It will also be understood that the composition may be in any form such that the composition is suitable for commercial application as described herein. For example, the composition may be in the form of a spore concentrate including mycelium of the *D. flagrans* and/or spores of the *D. flagrans,* wherein preferably the spores are chlamydospores. For example, when solid substrate fermentation is used, the wholegrain may be dried and ground to produce the composition of the invention, wherein the wholegrain includes mycelium of the *D. flagrans* and/or spores of the *D. flagrans,* wherein preferably the spores are chlamydospores.

It is also contemplated that the composition according to the invention, including the spore concentrate described herein, may be mixed with or prepared as a feed, feed supplement, bolus or veterinary medicinal composition. The feed or feed supplement may be in any form suitable for feeding grazing animals including, but not limited to; a loose mix, a liquid feed/supplement, pellet or lick block.

In another aspect, the present invention provides use of the composition according to the invention in the manufacture of a feed in any form, feed supplement, bolus or veterinary medicinal composition for controlling the spread of a parasitic nematode in a grazing animal.

In another aspect, the present invention provides use of the composition according to the invention in controlling the spread of a parasitic nematode in a grazing animal.

The composition according to the invention may also be formulated for administration to easily provide an amount of viable *D. flagrans* spores per Kg bodyweight of the grazing animal per day. In one embodiment the composition may be formulated for administration to provide at least about 1 × 10³ viable *D. flagrans* spores per Kg bodyweight of the grazing animal per day. In another embodiment, the composition may be formulated for administration to provide
1 × 10³ to 5 × 10⁸ viable *D. flagrans* spores per Kg bodyweight of the grazing animal per day, or
1 × 10³ to 2.5 × 10⁸ viable *D. flagrans* spores per Kg bodyweight of the grazing animal per day, or
1 × 10³ to 1 × 10⁸ viable *D. flagrans* spores per Kg bodyweight of the grazing animal per day, or
1 × 10³ to 5 × 10⁷ viable *D. flagrans* spores per Kg bodyweight of the grazing animal per day, or
1 × 10³ to 2.5 × 10⁷ viable *D. flagrans* spores per Kg bodyweight of the grazing animal per day, or
1 × 10³ to 1 × 10⁷ viable *D. flagrans* spores per Kg bodyweight of the grazing animal per day, or
1 × 10³ to 5 × 10⁶ viable *D. flagrans* spores per Kg bodyweight of the grazing animal per day, or
1 × 10³ to 2.5 × 10⁶ viable *D. flagrans* spores per Kg bodyweight of the grazing animal per day, or
1 × 10³ to 1 × 10⁶ viable *D. flagrans* spores per Kg bodyweight of the grazing animal per day, or
1 × 10³ to 5 × 10⁵ viable *D. flagrans* spores per Kg bodyweight of the grazing animal per day, or
1 × 10³ to 2.5 × 10⁵ viable *D. flagrans* spores per Kg bodyweight of the grazing animal per day, or
1 × 10³ to 1 × 10⁵ viable *D. flagrans* spores per Kg bodyweight of the grazing animal per day, or
1 × 10³ to 5 × 10⁴ viable *D. flagrans* spores per Kg bodyweight of the grazing animal per day, or
1 × 10³ to 2.5 × 10⁴ viable *D. flagrans* spores per Kg bodyweight of the grazing animal per day, or
1 × 10³ to 1 × 10⁴ viable *D. flagrans* spores per Kg bodyweight of the grazing animal per day, or
1 × 10³ to 9 × 10³ viable *D. flagrans* spores per Kg bodyweight of the grazing animal per day.
Preferably the composition may be formulated for administration to provide 1 × 10⁴ to 1 × 10⁵ viable *D. flagrans* spores per Kg bodyweight of the grazing animal per day.

For example, the composition may be formulated for administration to provide:
about 1 × 10⁴ viable *D. flagrans* spores per Kg bodyweight of the grazing animal per day, or
about 1.5 × 10⁴ viable *D. flagrans* spores per Kg bodyweight of the grazing animal per day, or
about 2 × 10⁴ viable *D. flagrans* spores per Kg bodyweight of the grazing animal per day, or
about 2.5 × 10⁴ viable *D. flagrans* spores per Kg bodyweight of the grazing animal per day, or
about 3 × 10⁴ viable *D. flagrans* spores per Kg bodyweight of the grazing animal per day, or
about 3.5 × 10⁴ viable *D. flagrans* spores per Kg bodyweight of the grazing animal per day, or
about 4 × 10⁴ viable *D. flagrans* spores per Kg bodyweight of the grazing animal per day, or
about 4.5 × 10⁴ viable *D. flagrans* spores per Kg bodyweight of the grazing animal per day, or
about 5 × 10⁴ viable *D. flagrans* spores per Kg bodyweight of the grazing animal per day, or
about 5.5 × 10⁴ viable *D. flagrans* spores per Kg bodyweight of the grazing animal per day, or
about 6 × 10⁴ viable *D. flagrans* spores per Kg bodyweight of the grazing animal per day, or
about 6.5 × 10⁴ viable *D. flagrans* spores per Kg bodyweight of the grazing animal per day, or
about 7 × 10⁴ viable *D. flagrans* spores per Kg bodyweight of the grazing animal per day, or
about 7.5 × 10⁴ viable *D. flagrans* spores per Kg bodyweight of the grazing animal per day, or
about 8 × 10⁴ viable *D. flagrans* spores per Kg bodyweight of the grazing animal per day,
   or
about 8.5 × 10⁴ viable *D. flagrans* spores per Kg bodyweight of the grazing animal per day, or
about 9 × 10⁴ viable *D. flagrans* spores per Kg bodyweight of the grazing animal per day.
   or
about 1 × 10⁵ viable *D. flagrans* spores per Kg bodyweight of the grazing animal per day.

More preferably, the composition may be formulated for administration to provide about 1 × 10⁴, about 3 × 10⁴ or about 1 × 10⁵ viable *D. flagrans* spores per Kg bodyweight of the grazing animal per day. Most preferably, the composition or biological control product may be formulated for administration to provide an amount of viable *D. flagrans* spores of about 3 × 10⁴ viable spores per Kg bodyweight of the grazing animal per day. It will also be understood that the composition of the invention may be formulated for administration to provide a standard amount, as desired, of composition wherein the above amounts of viable spores are provided per day.

It is also contemplated that "additional components" may be added to the composition of the invention which also control the spread of a parasite in a grazing animal e.g., components that provide a "double-strike" effect. Such additional components include, but are not limited to, anthelmintics (such as abamectin, ivermectin, pyrantel pamoate, pelletierine sulphate) which are effective against nematodes; or such as benzimidazoles (BZ), which are also ovicidal (with activity against eggs being passed by intestinal nematodes and tapeworms), macrocyclic lactones (ML), imidazothiazoles and tetrahydropyrimidines (LV), salicylanilides, nitazoxanide, praziquantel, octadesipeptides (eg. emodepside), derquantel (spiroindole) and aminoacetonitrile derivatives (AAD) which are effective against other worms, other nematophagous fungi (such as *Purpureocillium lilacinum, Stopharia rugosoannulata* or *Coprinus comatus),* tannins, minerals, diatomaceous earth, herbs, agricultural by-products, vaccines or copper oxide wire particles (COWP) or combinations thereof. The invention provides a composition that may further comprise one or more of the components from the following group: one or more anthelmintics, one or more other nematophagous fungi, copper oxide wire particles (COWP), a tannin, a mineral, diatomaceous earth, a herb, an agricultural by-product, one or more bacteria or a vaccine. Suitable dosages of these additional components will be clear to the skilled addressee based on the available scientific and commercial (product-specific) literature as well as the skilled addressee's own common general knowledge. In one embodiment, the composition further comprises COWP. For example, the COWP may be present in the composition of the invention in an amount suitable for administration to an animal at a dose of about 10g per 100 Kg bodyweight of the animal to be treated.

In another aspect, the present invention provides a composition for use in a method of controlling the spread of a parasitic nematode in a grazing animal comprising administering the composition of the invention in an amount and for a time sufficient to reduce the parasitic nematode (worm) burden on pasture by at least about 30%. For example, the composition may be administered in an amount and for a time sufficient to reduce the parasitic nematode (worm) burden on pasture in the range of 30% to 100%, or 35% to 100%, 40% to 100%, or 45% to 100%, or 50% to 100%, or 55% to 100%, or 60% to 100%, 65% to 100%, or 70% to 100%, or 75% to 100%, or 80% to 100%, or 85% to 100%, or 90% to 100%, 95% to 100%, or about 100%. Preferably, the composition may be administered in an amount and for a time sufficient to reduce the parasitic nematode (worm) burden on pasture in the range of 50% to 100%, more preferably in the range of 60 to 100%.

It will be apparent to the skilled artisan that the time sufficient to reduce parasitic nematode (worm) burden on pasture may vary according to the type of grazing animal, the animal's health condition and/or weight, and/or conditions of pasture, time of year, temperature and/or climate and will be determined by the user or their technical advisors.

Due to the development of resistance and multi-resistance to chemical anthelmintics, scientists worldwide recommend using faecal egg counts (FEC) to indicate which grazing animals need worming and when. It is also recommended that a faecal egg-count reduction test (FECRT) be performed from time to time to check whether the anthelmintics being used are effective and/or for identifying worm species by using faecal larval cultures (FLC). http://journals.plos.org/plosone/article?id=10.1371/journal.pone.0037327.

For example, the time sufficient to reduce parasitic nematode (worm) burden on pasture may be at least one week, or at least 2 weeks, or at least 3 weeks, or at least 4 weeks, or at least 5 weeks, or at least 6 weeks, or at least 7 weeks, or at least 8 weeks, or at least 9 weeks, or at least 10 weeks, or in the range of 1 to 26 weeks, or 1 to 15 weeks, or 1 to 10 weeks, or 1 to 8 weeks, or 1 to 6 weeks, or 1 to 5 weeks, or 1 to 4 weeks, or 1 to 3 weeks, or 2 to 15 weeks, or 2 to 10 weeks, or 2 to 8 weeks, or 2 to 6 weeks, or 2 to 5 weeks, or 2 to 4 weeks, or 2 to 3 weeks. Preferably, the time sufficient to reduce parasitic nematode (worm) burden on pasture may be in the range of 2 to 26 weeks. In one embodiment of the invention, the time may be in the range 12 to 20 weeks, and in particular 12 to 16 weeks.

It is contemplated that the composition of the invention may be administered for any length of time, even after the parasitic nematode (worm) burden on pasture is reduced. For example, the composition may be administered for at least one week, or can be continued indefinitely.

It will also be apparent to the skilled artisan that an amount sufficient to reduce parasitic nematode (worm) burden on pasture according to the composition for use of the invention may be an amount of viable *D. flagrans* spores contained in the composition that is administered to the grazing animal and will vary according to the animal's weight, and will be determined by the user or their technical advisors.

In one embodiment, according to the composition for use of the invention, the amount administered may comprise viable *D. flagrans* spores of at least about 1 × 10³ viable spores per Kg bodyweight of the grazing animal per day. In another embodiment, according to the composition for use of the invention, the amount administered may comprise viable *D. flagrans* spores in the range of
1 × 10³ to 5 × 10⁸ viable spores per Kg bodyweight of the grazing animal per day, or
1 × 10³ to 2.5 × 10⁸ viable spores per Kg bodyweight of the grazing animal per day, or
1 × 10³ to 1 × 10⁸ viable spores per Kg bodyweight of the grazing animal per day, or
1 × 10³ to 5 × 10⁷ viable spores per Kg bodyweight of the grazing animal per day, or
1 × 10³ to 2.5 × 10⁷ viable spores per Kg bodyweight of the grazing animal per day, or
1 × 10³ to 1 × 10⁷ viable spores per Kg bodyweight of the grazing animal per day, or
1 × 10³ to 5 × 10⁶ viable spores per Kg bodyweight of the grazing animal per day, or
1 × 10³ to 2.5 × 10⁶ viable spores per Kg bodyweight of the grazing animal per day, or
1 × 10³ to 1 × 10⁶ viable spores per Kg bodyweight of the grazing animal per day, or
1 × 10³ to 5 × 10⁵ viable spores per Kg bodyweight of the grazing animal per day, or
1 × 10³ to 2.5 × 10⁵ viable spores per Kg bodyweight of the grazing animal per day, or
1 × 10³ to 1 × 10⁵ viable spores per Kg bodyweight of the grazing animal per day, or
1 × 10³ to 5 × 10⁴ viable spores per Kg bodyweight of the grazing animal per day, or
1 × 10³ to 2.5 × 10⁴ viable spores per Kg bodyweight of the grazing animal per day, or
1 × 10³ to 1 × 10⁴ viable spores per Kg bodyweight of the grazing animal per day.

For example, according to the composition for use of the invention, the amount administered may comprise viable *D. flagrans* spores of
about 1 × 10⁴ viable *D. flagrans* spores per Kg bodyweight of the grazing animal per day, or
about 1.5 × 10⁴ viable *D. flagrans* spores per Kg bodyweight of the grazing animal per day, or
about 2 × 10⁴ viable *D. flagrans* spores per Kg bodyweight of the grazing animal per day, or
about 2.5 × 10⁴ viable *D. flagrans* spores per Kg bodyweight of the grazing animal per day, or
about 3 × 10⁴ viable *D. flagrans* spores per Kg bodyweight of the grazing animal per day, or
about 3.5 × 10⁴ viable *D. flagrans* spores per Kg bodyweight of the grazing animal per day, or
about 4 × 10⁴ viable *D. flagrans* spores per Kg bodyweight of the grazing animal per day, or
about 4.5 × 10⁴ viable *D. flagrans* spores per Kg bodyweight of the grazing animal per day, or
about 5 × 10⁴ viable *D. flagrans* spores per Kg bodyweight of the grazing animal per day, or
about 5.5 × 10⁴ viable *D. flagrans* pores per Kg bodyweight of the grazing animal per day, or
about 6 × 10⁴ viable *D. flagrans* spores per Kg bodyweight of the grazing animal per day, or
about 6.5 × 10⁴ viable *D. flagrans* spores per Kg bodyweight of the grazing animal per day, or
about 7 × 10⁴ viable *D. flagrans* spores per Kg bodyweight of the grazing animal per day, or
about 7.5 × 10⁴ viable *D. flagrans* spores per Kg bodyweight of the grazing animal per day, or
about 8 × 10⁴ viable *D. flagrans* spores per Kg bodyweight of the grazing animal per day, or
about 8.5 × 10⁴ viable *D. flagrans* spores per Kg bodyweight of the grazing animal per day, or
about 9 × 10⁴ viable *D. flagrans* spores per Kg bodyweight of the grazing animal per day, or
about 1 × 10⁵ viable *D. flagrans* spores per Kg bodyweight of the grazing animal per day.

Preferably, according to the composition for use of the invention, the amount administered may comprise about 1 × 10⁴ to 1 × 10⁵ viable *D. flagrans* spores per Kg bodyweight of the grazing animal per day. More preferably, the amount administered may comprise about 1 × 10⁴, about 3 × 10⁴ or about 1 × 10⁵ viable *D. flagrans* spores per Kg bodyweight of the grazing animal per day. Most preferably, the amount administered may comprise about 3 × 10⁴ viable *D. flagrans* spores per Kg bodyweight of the grazing animal per day. I

In another aspect, the present invention provides the composition for use of the invention administered as part of an integrated parasite management program (IPM) for controlling the spread of a parasitic nematode of grazing animals, wherein the IPM program comprising the following:
a) Treating the grazing animal with an anthelmintic effective against the parasitic nematode;
b) Transferring the grazing animal to a clean pasture, if possible, which is minimally infected by or free of larvae of the parasitic nematode; and
c) Administering the composition of the invention to the grazing animal.

It will be clear to the skilled addressee that any anthelmintic useful in treating the parasitic nematode may be used in treating the grazing animal. In particular, for example, abamectin, ivermectin, pyrantel pamoate and pelletierine sulphate are useful anthelmintics in the present invention. Following the introduction of phenothiazines in the 1950's, the control of gastrointestinal parasites has been achieved using chemical anthelmintics and predominantly relies on the treatment with broad-spectrum parasiticides belonging to three main chemical classes: the benzimidazoles (BZ), the macrocyclic lactones (ML) and the imidazothiazoles/tetrahydropyrimidines (LV). Although some anthelmintics, including derquantel (spiroindole) and monepantel (an amino-acetonitrile derivative, AAD) have been developed, success in the discovery of novel anthelmintics has been limited over the last two decades. The above anthelmintics may be administered as a combination of one or more thereof. Suitable dosages of the anthelmintic will be clear to the skilled addressee and will depend on the usual parameters such as the nature of the anthelmintic, the animal species, the animal's bodyweight, the animal or group's chemical resistance status and climatic conditions etc.

It will be apparent to the skilled artisan that a clean pasture at b) of the previous aspect may be prepared according to any method. Preferably, if possible, the clean pasture at b) is prepared by not grazing the pasture with the same species for a period of about 6 weeks or more.

It will be understood by the skilled addressee that "biological control" means the control of a pest by the introduction of a natural enemy or predator.

In the context of the present invention, the term "biological control composition" includes, but is not limited to, agricultural biopesticides, veterinary biochemicals, feed additives, and products useful for agricultural pest control.

Consistent with the examples and text of the present application, the skilled addressee will understand that in the context of the present invention when reference is made to the singular form of a noun eg. a spore, the plural is also contemplated. Specifically, for example, a composition comprising a spore includes a composition comprising many spores. As a further example, reference to treatment of an animal includes treatment of a herd or flock of animals or any other collective group.

The term "grazing animal" according to any aspect, example, or embodiment herein includes any exotic (nondomestic) or domestic grazing animal and includes but is not limited to both exotic (nondomestic) and domestic ruminants. For example, the domestic grazing animal is selected from the group consisting of, but not limited to, horse, goat, sheep, cattle, deer and camelids (such as alpacas and llamas). In another example, the nondomestic or exotic animal is selected from species of artiodactylids including but not limited to giraffe, antelope, deer, oryx, sable antelope, blackbuck, bongo, okapi and wildebeest. Preferably, the exotic animal according to the invention is one that is held in captivity within an enclosure, e.g., in a zoological facility. In another example the grazing animal is selected from animals including, but not limited to, monograstric, ruminant, camelid and pseudo-ruminant animals (domestic or non-domestic or exotic). In another example, the grazing animal is selected from domestic or wild animals.

It will also be understood that the term "parasitic nematode" according to any aspect, example, or embodiment herein includes any parasitic nematode that is known, or subsequently discovered to infect a grazing animal. For example the parasitic nematode is any one or more of a nematode of the *Stronglyus spp., Cyathostomum spp., Triodontophorous spp., Trichonema spp., Oesophagodontus spp., Gyalocephalus spp., Cylicocylus spp., Cylicodonotophorus spp., Cylicostephanus spp., Gongylonema spp., Habronema spp., Mecistocirrus spp., Oxyuris spp., Parascaris spp., Skrjabinema spp., Strongyloides spp., Toxocara spp., Dictyocaulis spp., Muellerius spp., Protostrongylus spp., Onchocerca spp., Parafilaria spp., Setaria spp., Stephanofilaria spp., Thelazia spp., Trichostrongylus spp., Cooperia spp., Bunostomum spp., Teladosagia spp., Oesophagostomum spp., Nematodirus spp., Haemonchus spp., Chabertia spp., Trichuris spp.,* and/or *Ostertagia spp.*

Preferably, the parasitic nematode according to any aspect, example, or embodiment herein is one or more of Barber's Pole Worm or Wire Worm (*Haemonchus* spp.), Hairworm or Stomach Hairworm *(Trichostrongylus* spp), Intestinal Worm or Small Intestinal Worm (*Cooperia* spp.) and Hookworm (*Bunostomum* spp.), Black Scour Worm *(Trichostrongylus spp.),* Small and Medium Brown Stomach Worm (*Teladosagia (Ostertagia) spp.),* Nodule Worm (*Oesophagostomum* spp.), Stomach Hair Worm (*Trichostrongylus* spp.), Thread Worm (*Strongyloides* spp.), Large Mouthed Bowel Worm (*Chabertia* spp.), Whipworm (*Trichuris* spp.), Thin Necked Intestinal Worm or Thread Necked Worm (*Nematodirus* spp.), Redworms or Large Strongyles (including *Strongylus* spp., *Triodontophorus* spp.and *Oesophagodontus* spp.) and/or Cyathostomes / Small Strongyles (including *Cyathostomum* spp., *Trichonema* spp., *Gyalocephalus* spp., *Cylicocyclus* spp., *Cylicodontophorus* spp. and *Cylicostephanus* spp.), Ascarids (*Parascaris* spp.) and Pinworms (*Oxyuris* spp.).

In one or more embodiments, the composition and use thereof of the present invention provides the following advantages:
- *D. flagrans* IAH 1297, is not absorbed by the host animal.
- *D. flagrans* IAH 1297, survives passage through the gut and into the manure.
- *D. flagrans* IAH 1297, remains in the spore form as a chlamydospore until environmental conditions are suitable coinciding with the nematodes becoming active, then germinating in the manure where the worm larvae are trapped, paralysed and consumed.
- *D. flagrans* IAH 1297, contains low or no undesirable metabolites. It has very low levels of flagranone A, undetectable levels of flagranone B and undetectable levels of flagranone C. Hence the organism falls below the current European safety thresholds for such substances.
- Used according to the present invention, *D. flagrans* IAH 1297, effectively breaks the worm life cycle.
- *D. flagrans* IAH 1297, used according to the present invention is effective against anthelmintic-resistant and multi-resistant worms.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to".

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

Each aspect, embodiment and/or example of the invention described herein is to be applied *mutatis mutandis* to each and every aspect, embodiment and/or example unless specifically stated otherwise.

Those skilled in the art will appreciate that the invention includes all of the steps, features, compositions and compounds referred to or indicated in this specification, individually or collectively, and any and all combinations or any two or more of said steps or features.

The present invention is not to be limited in scope by the specific embodiments described herein, which are intended for the purpose of exemplification only. Functionally-equivalent products, compositions and methods are clearly within the scope of the invention, as described herein.

### List of Abbreviations:

GIN - Gastrointestinal nematodes
EP - End User product
MP - Mill Product
TGAI - Technical Grade Active Ingredient
LOQ - Limit of quantitation
LOD - Limit of detection
HPLC - high performance liquid chromatography
UV - ultraviolet
IPM - Integrated Pest Management
FEC - Faecal Egg Count
FECRT - Faecal Egg Count Reduction Test
FLC - Faecal Larval Cultures

### Brief Description of the Drawings

Figure 1: Schematic diagram showing the basic lifecycle of gastrointestinal nematodes in grazing animals.
Figure 2: Schematic diagram showing the development of chemical resistance. The diagram indicates the year of introduction of various chemicals and R denotes the year of first recorded resistance Waller, P.J., Acta Tropica 56(1994)233-243. Antimicrobials: SULHPA; sulphonamides, PEN, penicillin; STREP, streptomycin. Insecticides: DDT, dicophane; CYCLO, cyclodienes; OP, organophosphates; SP, synthetic pyrethroids. Fungicides: AH, aromatic hydrocarbons; DOD, dodine; BZ, benzimidazoles; DCB, dicarboxamides. Anthelmintics: BZ, benzimidazoles; LEV, levamisole; AVM, avermectins.
Figure 3: Schematic diagram showing a nematode larva captured by *D. flagrans.* The larva is captured in a sticky net consisting of a number of strong arcs. At the contact part, the parasite body is penetrated whereby the fungal hyphae extend out to grow and fill the body of the nematode to effectively kill the nematode. Inner organs are eventually dissolved and absorbed. The dotted lines show the fungal mycelium inside the parasite.
Figure 4a: Method for measuring faecal egg count (FEC).
Figure 4b: Method for faecal egg count reduction test (FECRT)
Figure 5: Product information for Livamol^{®}
Figure 6: Graphical representation of cattle field trial results (Cattle Trial 1) using the composition of the invention as described in Example 7 and Table 3.
Figure 7: Graphical representation of cattle field trial results (Cattle Trial 2) using the composition of the invention as described in Example 7 and Table 3.
Figure 8: Graphical representation of goat field trial results (Goat Trial 1) using the composition of the invention as described in Example 7 and Table 3.
Figure 9: Graphical representation of goat field trial results (Goat Trial 2) using the composition of the invention as described in Example 7 and Table 3.
Figure 10: Graphical representation of goat field trial results (Goat Trial 3) using the composition of the invention as described in Example 7 and Table 3.
Figure 11: Graphical representation of horse field trial results (Horse Trial 1) using the composition of the invention as described in Example 7 and Table 3.
Figure 12: Graphical representation of horse field trial results (Horse Trial 2) using the composition of the invention as described in Example 7 and Table 3.
Figure 13: Graphical representation of horse field trial results (Horse Trial 3) using the composition of the invention as described in Example 7 and Table 4.
Figure 14: Graphical representation of sheep field trial results (Sheep Trial 1) using the composition of the invention as described in Example 7 and Table 4.
Figure 15: Graphical representation of sheep field trial results (Sheep Trial 2) using the composition of the invention as described in Example 7 and Table 4.
Figure 16: Graphical representation of sheep field trial results (Sheep Trial 3) using the composition of the invention as described in Example 7 and Table 4.
Figure 17: Graphical representation of sheep field trial results (Sheep Trial 4) using the composition of the invention as described in Example 7 and Table 4.

### Detailed Description of Preferred Embodiments

The present invention will now be described in more detail with reference to specific but non-limiting examples describing specific compositions and methods of use. It is to be understood, however, that the detailed description of specific procedures, compositions and methods is included solely for the purpose of exemplifying the present invention. It should not be understood in any way as a restriction on the broad description of the inventive concept as set out above.

### EXAMPLE 1

### Isolation, culturing and characterisation of D. flagrans

The inventors sought to identify a *D. flagrans* isolate that would be useful for further work in development of a composition or biological control product. Without being bound to any particular theory, it is desirable that a suitable isolate exhibits relatively rapid and abundant growth, produces large quantities of chlamydospores for ease of scale-up, has a high predatory ability for maximum effectiveness on release, ease of monitoring on release as a biological control agent, has no negative effects on non-target organisms.

Twenty-five isolates were examined and *D. flagrans* IAH 1297 was found to have good growth rate, and chlyamydospore production, a high trapping efficiency and good growth on a grain substrate compared to the others. Based on these characteristics, the *D. flagrans* IAH 1297 isolate was deposited at National Measurement Institute, 1/153 Bertie Street, Port Melbourne, Victoria 3207, Australia on 2 August 2016 in accordance with the Budapest Treaty requirements.

By way of non-limiting example only, the following assays were used to determine these parameters.

### Fungal Isolates

Fungal isolates were obtained from original cultures derived from a field survey to detect the presence of nematophagous fungi in faecal, compost and soil samples according to procedures as described in Larsen et al., 1994 (Veterinary Parasitology 53:275-281) and subsequent additions to the CSIRO culture collection. Subcultures were taken from parent stock and maintained on Potato Dextrose Agar (PDA) for purity and viability using the procedure outlined below.

### Culturing Procedure

Long-term cultures were maintained in 100% PDA McCartney bottle slopes. These were prepared using commercially available PDA Difco^{™} according to the manufacturer's instructions. 10 mL of PDA mixture was then dispensed into McCartney bottles. Lids were loosely placed on bottles and autoclaved at 121°C for 21 min. While still hot, lids were tightened and bottles placed on a 45° angle and then allowed to cool and solidify. Spore inoculum was then transferred from parent cultures to the McCartney bottle agar surface using a sterile bacterial loop. The McCartney bottles were labelled in accordance with media type, fungal culture ID and date of inoculation, and stored in a refrigerator at 4°C for future use.

Short-term cultures derived from the long-term cultures, were maintained on PDA plates (6 cm diameter). Potato dextrose agar was prepared as above and autoclaved in the 1 L pyrex bottle at 121°C for 21 minutes. While still hot, 2 mL of antibacterial agent ENGEMYCINO (which contains 100 mg/mL of oxytetracycline hydrochloride) was added to the agar and mixed by inverting. Plates were then poured in a Laminar flow cabinet ensuring complete coverage of petri dish to a depth of approximately 6 mm. These were then allowed to cool and solidify before storage at 4°C until use. Using a sterile surgical blade, 3 mm × 3 mm sections of agar containing the fungal culture were cut and placed in the centre of a new PDA plate. Plates were then sealed with Parafilm^{®} to prevent desiccation and were incubated at 27°C for 7 - 10 days. Plates were labelled with fungal ID and dated.

### Temperature

*D. flagrans* isolates were evaluated. Five constant temperature regimes were tested: 12°C, 17°C, 22°C, 27°C and 32°C. Three replicate 1% Potato-Dextrose Agar (PDA) plates for each treatment (6 cm diameter) were inoculated with 2 mm × 2 mm agar plugs cut with a sterile scalpel blade from the colony margin of 1-2 week old fungal cultures grown on 1% PDA and placed in the centre of the test agar plates. Each treatment was incubated for 14 days (or days otherwise indicated herein below) in a Gallenkamp controlled temperature incubator that had been calibrated to the desired temperature. Throughout the experiments the temperature within each cabinet varied by +/- 2°C. Radial growth measurements and chlamydospore enumeration was calculated daily.

### Radial Growth

Radial growth was measured daily under a stereo dissecting microscope at 10 X magnification. Radial growth was calculated by measuring the distance (mm) from the edge of the agar plug to the edge of the mycelial growth twice in opposite directions. One of these directions was chosen randomly. Growth rate was calculated over the number of days it took for the mycelia to reach the outer perimeter of the plate.

### Chlamydospore Enumeration

Chlamydospore numbers were determined daily by placing a 6 cm² grid divided into 2 mm² squares beneath the agar plate under a stereo dissecting microscope at 10 X magnification. The grid contained five randomly marked 2 mm² squares that were approximately 12-14 mm from the centre plug and the chlamydospore numbers in these five areas were counted and the number of chlamydospores per cm² was calculated.

### Statistical Analysis

Analysis of variance with Tukey's post hoc testing using Statgraphic software (Statgraphics Centurion XV, 2006) was performed at each temperature to assess the differences between the number of days taken to reach the perimeter of the petri dish and also to determine differences between the rate of growth between the isolates. The same process was used to determine the differences between the rate of spore production between the isolates, in addition to the number of days taken to the first spore production.

### Trapping Efficiency

### Fungal inoculum for measurement of trapping efficiency

Fungal isolates of *D. flagrans* were obtained from original cultures derived from a field survey (Larsen et al., 1994 Veterinary Parasitology 53:275-281), and subsequent additions to the CSIRO. Subcultures were taken from parent stock and maintained on Potato Dextrose Agar (PDA) using the procedure outlined above. The spore material used was grown on barley substrate as outlined below in Example 2 and added to faecal cultures at a concentration of 1 × 10⁶ chlamydospores/g of faeces.

### Faecal cultures

Faecal material was collected from worm-free and *Trichostrongylus colubriformis* infected sheep kept in the CSIRO animal house at F.D. McMaster Laboratory-Chiswick, Armidale, NSW, for the purpose of maintaining helminth stocks. Faeces were collected in a bag attached to the rear end of the sheep by a faecal collection harness and left overnight. Sheep were fed 800 g/day feed pellets with a composition of (g/kg): lucerne 500, wheat 100, pollard 200, bran 175, salt 20 and ammonium chloride 5. Worm infected sheep were infected with a single oral dose of 20,000 third stage *7. colubriformis* larvae. Infected sheep were dosed weekly with 0.25-0.5 mg per 10 kg of the corticosteroid "Ilium Trimedexil^{©}, Troy Laboratories Pty Ltd" by intramuscular injection, to partially suppress the immune system to help maintain worm infection.

Faecal egg counts were estimated using a modified McMaster technique (Whitlock, 1948 Journal of the Council for Scientific and Industrial Research 21:177-180). Worm egg containing faeces was mixed with worm free faeces to create a faecal mixture with an egg count of approximately 2,500 eggs/g.

Twenty-five grams of faeces was mixed in Styrofoam cups with approx 7-10 g vermiculite (depending on initial faecal consistency) to create a moist crumbly texture and this sheep faecal mix was then transferred to 500 g culture jars (M.R. Knox *pers. comm.*)*.* For each isolate, five replicate culture jars were used and chlamydospores of the isolate were added to each of the replicate culture jars containing the sheep faecal mix. After thorough mixing, the faecal mix was then lightly compressed to create an even consistency. Approximately 10 mL of distilled water was then added to each jar to maintain humidity. Five replicate control cultures were similarly prepared without the addition of spore material. The culture jars were then incubated at 20°C for 10 days.

After incubation, culture jars were filled with distilled water and a 20 cm diameter petri dish was placed on the top of the jar. Jars were then inverted, the petri dish half filled with distilled water and left at room temp for 48 hrs. All third stage larvae (L3) were then collected by decanting the water from the Petri dishes into 50 mL collection tubes and made up to a volume of 50 mL. Formalin was added at final concentration of 1-2 % and then the 50 mL larval solution was heated at 55- 57°C for approximately 1 min to kill the larvae (Van Wyk et al. 2004 Veterinary Parasitology 119:277-306). Larvae were enumerated by taking 5 replicate 100 µL aliquots of each sample on a microscope slide and counting under 100x magnification. If the larvae counted was less than 10 per 100 µL aliquot, then the larvae were left to settle for a minimum of 3 hrs before siphoning off excess liquid, concentrating to a volume of 5 mL, before enumeration was repeated as described.

### EXAMPLE 2

### Solid substrate fermentation method/analysis

By way of non-limiting example only, *D. flagrans* may be produced using the following solid substrate fermentation method.

### Preparation of liquid inoculum for grain substrates

Liquid broth was made using 1 % yeast extract, 0.5 % KH₂PO₄, 0.1 % NaNOs, 0.05 % MgSO₄.7H₂O and 2 % soluble starch. The pH was adjusted to 6.5 using 0.1M HCl and sterilised in an autoclave under standard liquid conditions. The broth was then left to cool to approximately 25°C. Once cool, 250 mL of broth contained in a 1.5 L conical flask, was inoculated with 8 equal pieces of a ¼ (10 cm diameter) plate of a 7 day PDA culture of D. *flagrans* and incubated on an orbital shaker at 200 rpm for 7 days at 28°C.

### Solid substrate preparation and fermentation

For each substrate, triplicate 50 g grain samples were weighed into 250 mL Erlenmeyer flasks. The samples were then moistened with water at a rate of 1/3 of the total volume required to achieve substrate moisture of 40 % (See Figures 3.1, 3.2 and 3.3) and mixed thoroughly. Samples were left standing at room temperature overnight (16 hrs). After 16 hrs, the flasks were plugged with cotton wool and covered with foil. The flasks were then autoclaved under standard liquid conditions. The flasks were then transferred to a laminar flow cabinet and cooled to room temperature. Once cool, the flasks were inoculated with the final volume of liquid inoculum to achieve 40 % substrate moisture using sterilised measuring cylinders. In the case of millet, this was 24 mL of liquid inoculum; for lupins it was 24 mL of liquid inoculum plus 1 mL of sterile H₂O; and for barley it was 24 mL of liquid inoculum plus 4 mL of sterile H₂O. The addition of sterile H₂O to the inoculum for lupins and barley was to ensure each flask had an equal amount of fungal inoculum added while maintaining the substrate moisture at 40 %. Inoculated cultures were allowed to stand before being gently mixed after 24 hrs to ensure that all free moisture had been absorbed by the grains. After one week's incubation, substrates were broken up by vigorously shaking flasks. They were further incubated for 25 days at 20°C.

### Drying Procedure

After incubation, fermented grains were broken up by thorough shaking in the flask. In the laminar flow cabinet, the fermented grains were then transferred to small, shallow Styrofoam trays, combining the replicate cultures once it was determined that there was no contamination. Remaining substrate clumps were aseptically broken up. Cultures were dried for 10 days in a room with controlled temperature set at 22°C to achieve a moisture content of <10 % (as determined by oven moisture analyses of a 5 g sub-sample after drying for 2 hrs at 130°C). Periodic, gentle agitation of the trays during this drying period ensured rapid and even drying. The dried samples were stored in tightly capped 250 mL screw cap containers at 20°C for subsequent analysis.

### Chlamydospore Enumeration

After drying, the samples were ground using a pre-cleaned coffee grinder. Triplicate 0.1 g samples of dried and ground cultures were weighed into 50 mL digestion tubes. Samples were digested for 72 hrs with constant shaking in an iso-tonic saline solution containing 1 % pepsin (1:2500 Pepsin A; Sigma chemicals, Sydney) and 1.7 % hydrochloric acid (AR grade). Chlamydospores were counted using a Spencer Brightline^{™} haemocytometer and the spore yield calculated as chlamydospores per gram of dried and ground culture material.

### Viability assessment - MTT staining procedure

From the above samples, 5 g of ground spore material was suspended in 50 mL of sterile 10 mM potassium phosphate buffer (pH 6.9). This suspension was used for plate analysis. Triplicate 1.0 mL aliquots of the diluted spore suspension were dispensed into 5 mL vials. To each vial 1.0 mL of filter sterilized 3-(4,5-dimethylthiazol-2-yl)-2,5-diphenyltetrazolium bromide (MTT) (2 mg/ mL) and 2 µL 2.5 mM of cobalt chloride solution was added. Vials were tightly capped and incubated at 27°C for 24 - 36 hrs. After incubation the viable spores were counted using a haemocytometer. Viable spores stain red or blue through blue/black and non-viable spores do not stain. Only spores showing complete cytoplasmic staining were counted as viable (Meier and Charvat, 1993 American Journal of Botany 80:1007-1015).

### Statistical analysis

The differences in viable spores/g were estimated using ANOVA and Tukey's post hoc testing (Statgraphics Centurion XV., 2006).

### EXAMPLE 3

### Production scale-up for commercial application

By way of non-limiting example only, the *D. flagrans* of the composition according to any aspect, embodiment or example herein may be scaled-up for commercial application as follows. Below, in the context of the present non-limiting examples, the following terms are used:
Technical Grade Active Ingredient (TGAI): this refers to the dried raw material comprising chlamydospores of *D. flagrans* strain IAH 1297, which is the product of the method described in the present example, Example 3. Typically, this product would contain 1 × 10⁶ or more viable spores per gram.
End-user Product (EP): this refers to a product comprising TGAI blended with feed ingredients including, but not limited to, protein meals, oils, fats, carbohydrates, antioxidants, preservatives, vitamins, minerals and colourants. EP is intended for use by farmers as an animal feed supplement or for mixing into animal feed.
Mill Product (MP): this refers to a product comprising TGAI blended with feed ingredients including, but not limited to, protein meals, oils, fats, carbohydrates, antioxidants, preservatives, vitamins, minerals and colourants. MP is intended for sale to third-party manufacturers, such as but not limited to, feed mills, veterinarians or others that may use it as an ingredient in their own branded products including animal feed supplements and feeds. In the context of the present non-limiting example, MP comprises about 15x more TGAI than EP.
Purified TGAI: this refers to a product derived from TGAI in which the density of *D. flagrans* chlamydospores is increased relative to the TGAI from which it is derived.

A dried spore concentrate was produced. As indicated above, the dried spore concentrate was called the Technical Grade Active Ingredient (TGAI). The TGAI was produced as follows: 
Scheme 1 Manufacturing D. flagrans

MANUFACTURING OF DUDDINGTONIA FLAGRANS

RECEIVING RAW MATERIAL

FORMULATION OF
FERMENTATION SUBSTRATE

STERILISATION

INOCULATION WITH
PURE CULTURE

FERMENTATION

DRYING

QUALITY CONTROL

PACKAGING
a. The *D. flagrans* was grown using a standard solid substrate fermentation method as in Example 2. The *D. flagrans* was grown on a moistened wholegrain; additional growth media was added, containing an energy source, a nitrogen source, as well as nutrients;
b. The production process is a 2-step process comprising sterilizing the moistened grain by heat or gamma irradiation, inoculating the moistened grain with liquid broth containing the fungus and possibly including some or all of the constituents listed at (a). Inoculation is performed under sterile conditions and fermentation is allowed to take place over several weeks, typically about 4 weeks. The fermentation step is carried out at a controlled temperature of 20-28 degrees Centigrade and the moisture content varies from as low as 20% to about 40 to 60% moisture. During the fermentation step, spores develop naturally, specific initiation methods for inducing spore formation are not being used at this stage.
c. There are currently two types of fermentation processes used. In one process, stainless steel trays are used to contain the growth substrate and air or oxygen is circulated in the headspace during fermentation. In the other process, the growth substrate is contained in pillow packs that comprise a plastic pouch with a breathing strip, and the fermentation process is allowed to take place on a shelf, thus no forced circulation of air or oxygen is used. The fermentation process is designed to exclude the introduction of foreign organisms and to allow the escape of carbon dioxide and ingress of air.
d. The second step comprises drying the fermented grain by exposure to air. This is done as rapidly as possible at low temperature to a moisture content of less than 10%. The spores of *D. flagrans* are not separated from the grain because the *D. flagrans* produces spores that are an integral part of the grain. Once dried, the grain including the spores produce the raw material of spore concentrate. The spore concentrate contains dried mycelium including spores. The viable count of viable spores per gram obtained ranges between 1 × 10⁵ to 1 × 10¹⁰ spores/g.
e. On completion of drying the product (TGAI) is packed into sealed bags, packaged and shipped for further manufacture.

### EXAMPLE 4

### End-user Product (EP) and Mill Product (MP)

By way of non-limiting example only, the TGAI was prepared as described in Example 3 and formulated to provide a composition according to the invention. Two products were prepared, an End-user Product (EP) and a Mill Product (MP) as described above.

As indicated above, to produce the EP and MP, the TGAI was blended with feed ingredients including, but not limited to, ingredients comprising protein meals, oils, fats, carbohydrates, antioxidants, preservatives, vitamins, minerals and colourants. In the present, non-limiting example, MP comprises about 15x or more TGAI than the EP. After blending, the mixture was milled to break-down the spore granules to ensure homogeneity. The finished products were packaged in bags or bulk containers. Typically, the products (both EP and MP) provide a minimum of 3 × 10⁴ viable spores per Kg bodyweight per day when in use (as measured for example in the viability method in Example 5).

### EXAMPLE 5

### Faecal egg count test (FEC) test

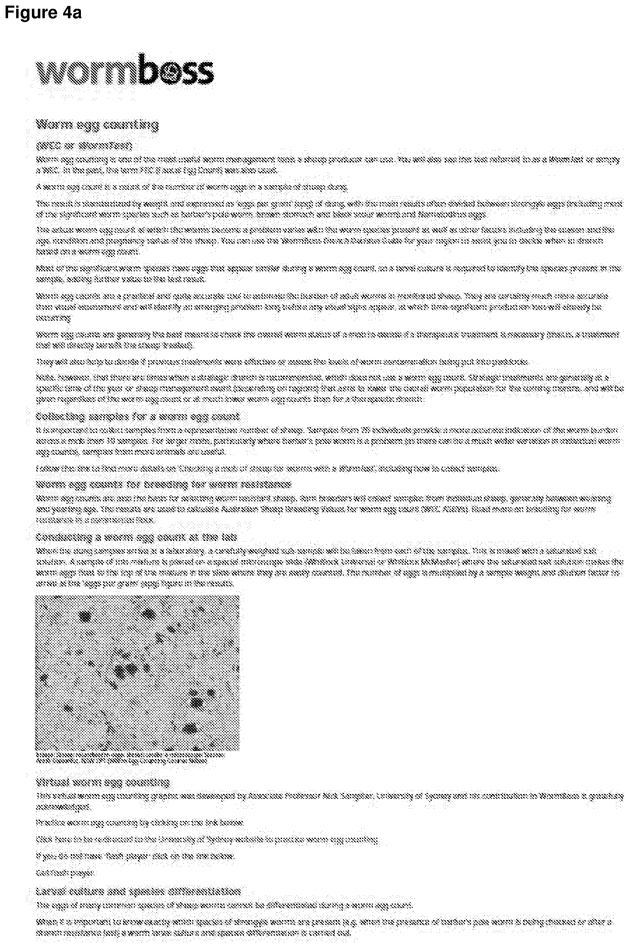

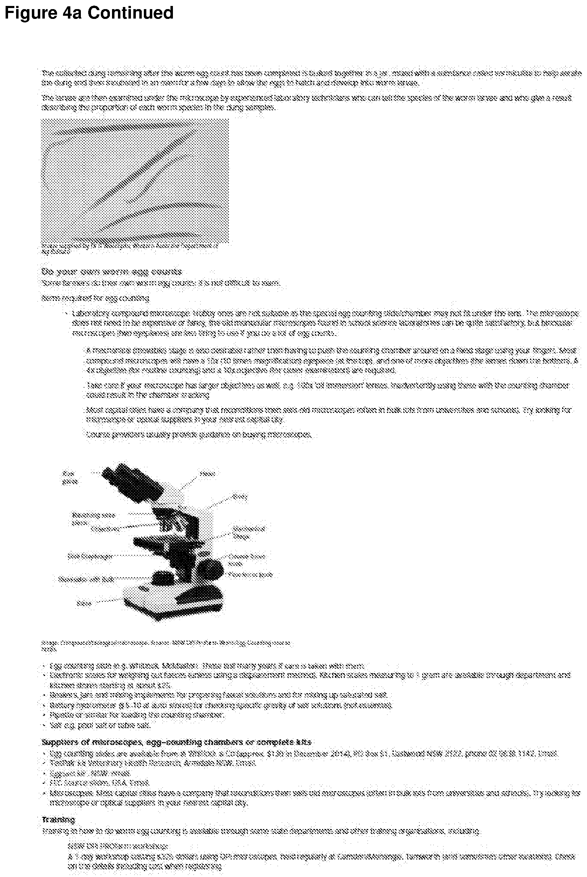

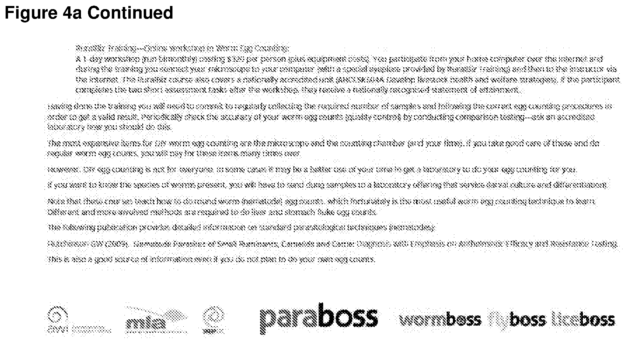

Methods for faecal egg counting (FEC) are well known in the art. Examples of techniques are widely available. An example of instructions for FEC is provided in Figure 4a. The skilled addressee will be aware of many other sources of methodologies.

### EXAMPLE 6

### Faecal egg count reduction test (FECRT)

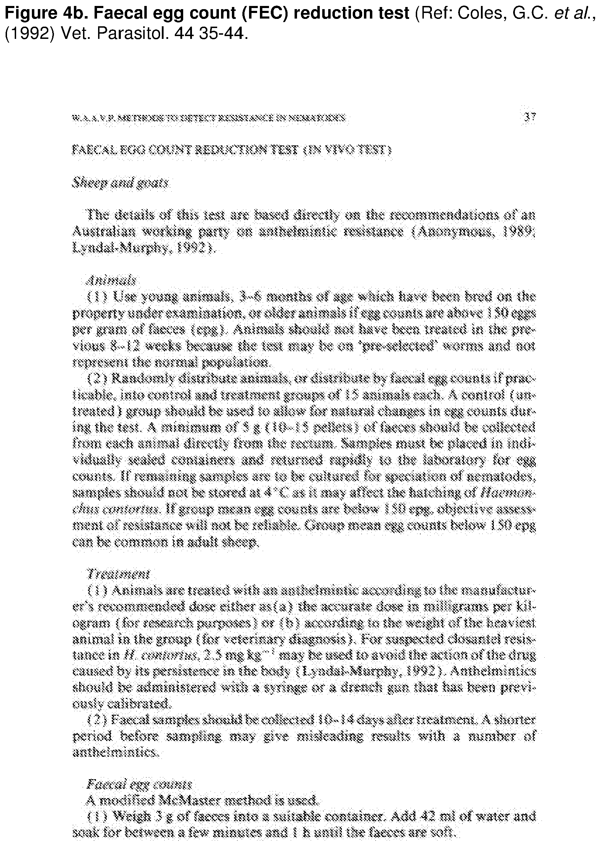

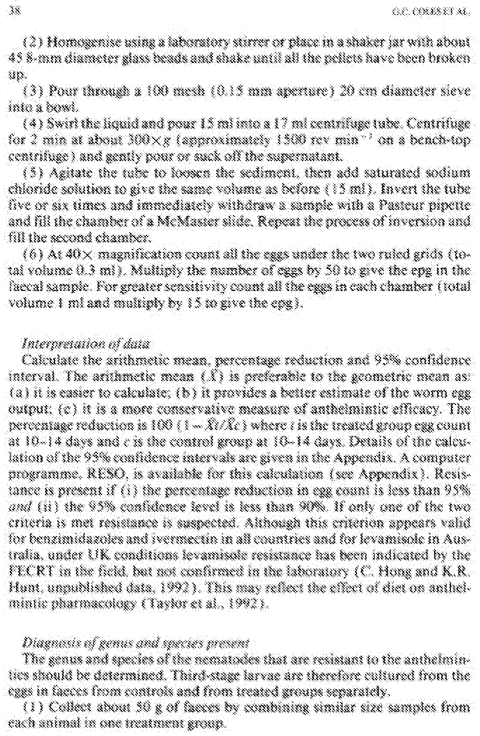

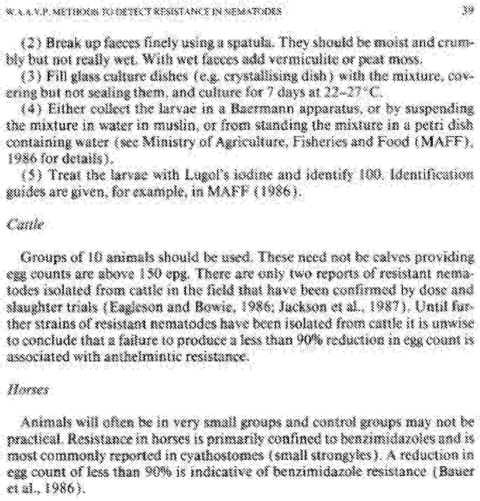

Methods for faecal egg count reduction test (FECRT) are well known in the art. Examples of techniques are widely available. An example of instructions for FECRT is provided in Figure 4b. The skilled addressee will be aware of many other sources of methodologies.

### EXAMPLE 7

By way of non-limiting example only, efficacy trials were conducted with the composition of the invention. The composition of the invention was prepared for example, according to Example 3 and further formulated to produce EP according to Example 4.

A series of placebo-controlled trials in cattle, sheep, goats and horses were conducted according to VICH (International Cooperation on Harmonisation of Technical Requirements for Registration of Veterinary Medicinal Products) Good Clinical Practice (GCP) in different Australian regional locations and seasons to evaluate the ability of the EP to reduce larval development and migration from faeces onto pasture.

Briefly, for the short-term trials with cattle, goats and horses, faeces were collected from the trial animals prior to treatment and placed on a pasture site (Control faeces). The same animals were then treated with the EP and faeces again collected after about 1 week (Treated faeces). These faeces were then also placed onto pasture sites. At 2 weekly intervals (from 2 through 8 weeks post treatment) samples from the pasture around randomly-selected faecal pats (Treated and Control) faecal samples were collected and examined for nematode larvae. The larvae found were then distinguished to species (as parasitic larvae for the host species involved) and the total parasite larval numbers for each sample were calculated. The treatment effect was measured by comparing the pasture larval counts of the two groups (Treated vs. Control).

For the long-term grazing studies in sheep, two groups of worm-infested sheep were grazed on separate paddocks, one group receiving EP and the other a placebo. The effect of treatment on the larval infectivity on the pasture was measured by co-grazing with groups of worm-free tracer lambs and the treatment effect was measured by comparing total worm counts of the two groups of tracers.

Worm species encountered in these studies included those shown in Table 2 below.

**Table 2. Worm species encountered in EP (containing chlamydospores of D. flagrans strain IAH 1297) efficacy trials**

| **Worm type** | **Cattle** | **Sheep** | **Goats** | **Horses** |
|---|---|---|---|---|
| *Cooperia spp.* | X | X | | |
| Cyathostomes | | | | X |
| *Haemonchus spp.* | X | X | X | |
| *Nematodirus spp.* | | X | X | |
| *Oesophagostomum spp.* | X | | X | |
| *Ostertagia spp.* | X | | | |
| *Strongylus spp.* | | | | X |
| *Teladorsagia spp.* | | X | X | |
| *Trichostrongylus spp.* | X | X | X | X |

Statistically-significant reductions (P<0.05) in total worm count in the tracer lambs were obtained in each of the sheep trials. For the cattle, goat and horse studies, the pooled data for each animal species were analysed, showing that the treatment effect was significant at P≤0.01 in each case, as shown below.

The studies with EP summarised below were conducted according to VICH GL9 GCP (June 2000) and World Association for the Advancement of Veterinary Parasitology (WAAVP) Guidelines for evaluating the efficacy of anthelmintics in ruminants.

### Short term efficacy studies with feed supplement (EP) (cattle, horses and goats)

A series of placebo-controlled trials were conducted to evaluate the ability of EP to reduce larval development and migration from faeces on pasture in cattle, horses and goats.

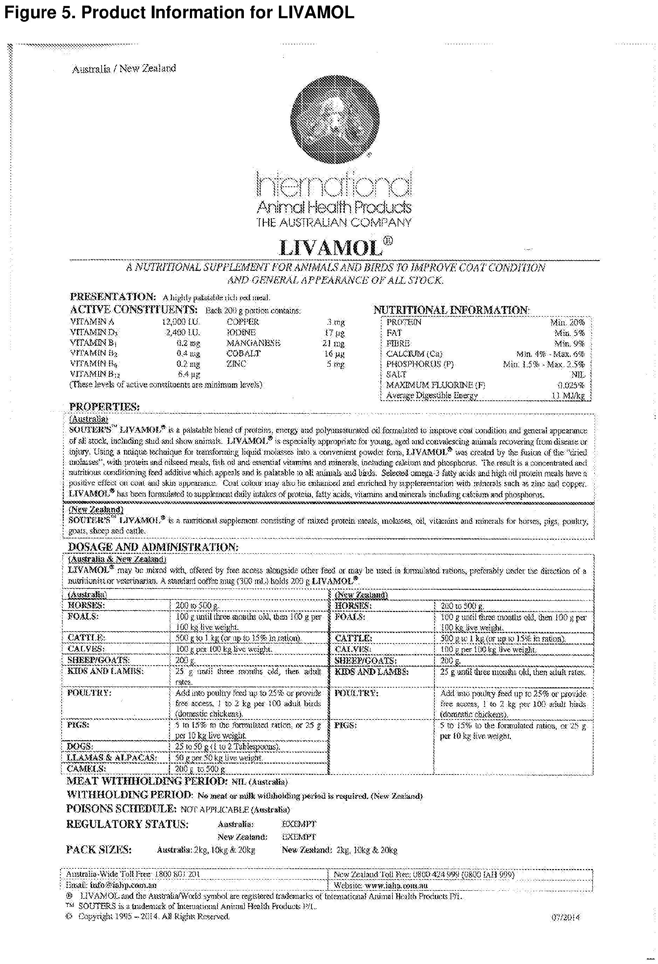

In these trials the following products were used:
1. Livamol^{®}: Placebo Product (see Product description at Figure 5) - a nutritious animal feed supplement manufactured by International Animal Health Products Pty Ltd, referred to as "Livamol^{®}" below.
2. EP - comprising TGAI dispersed in Livamol^{®} and referred to as "EP" below.

Briefly, the selected trial animals harboured a variety of parasitic nematodes, including anthelmintic-resistant strains, in some cases these were augmented via artificial infection. The animals were fed Livamol^{®} for about 1 week prior to collection of their faeces, which were then manually placed on a pasture at the trial site(s) (Control faeces). The same animals were then treated with EP (providing minimum 3 × 10⁴ viable chlamydospores of D. flagrans/kg bodyweight/day for about 1 week) and faeces again collected and placed onto pasture as above at the same sites (Treated faeces). At 2 weekly intervals (from 2 through 8 weeks post treatment) pasture samples from around the randomly-selected deposited faecal samples were collected and examined for nematode larvae. The larvae found were then distinguished into species (as parasitic larvae for the host species involved) and the total parasite larval numbers for each sample was calculated. The action of *D. flagrans* in the faecal pats of EP-treated faeces was evidenced in the reduced numbers of larvae found on the surrounding pasture.

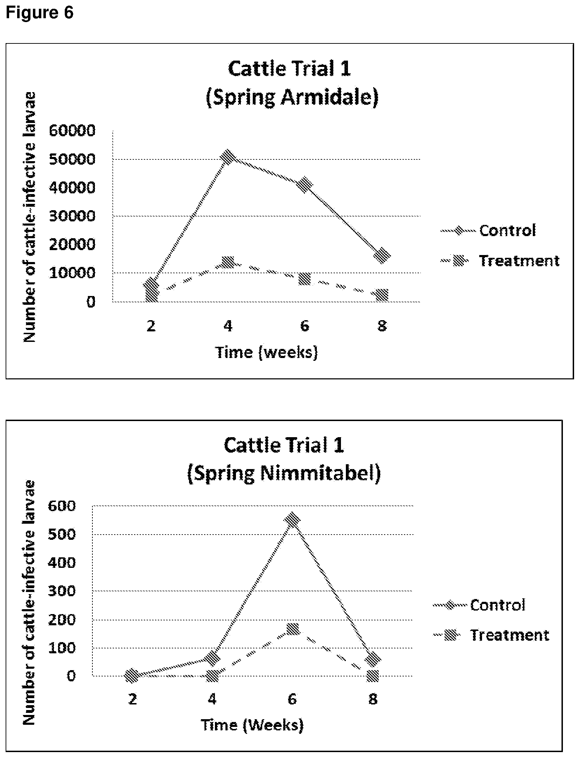

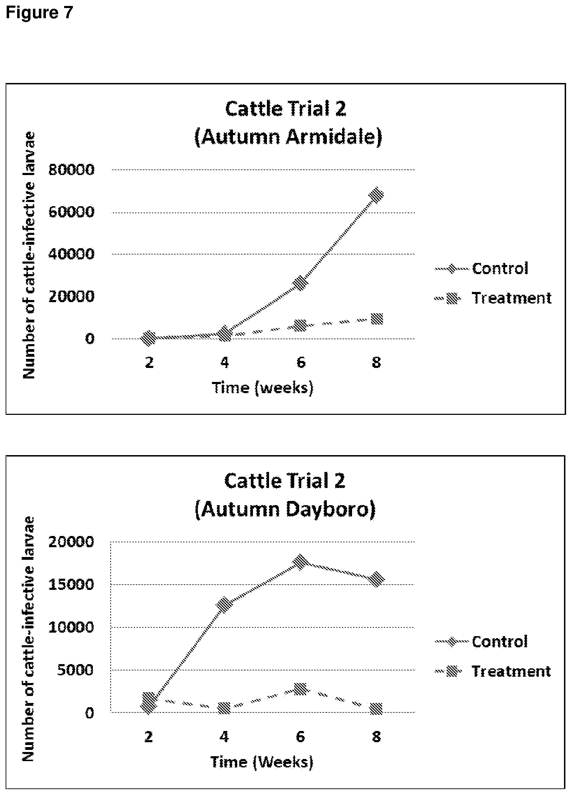

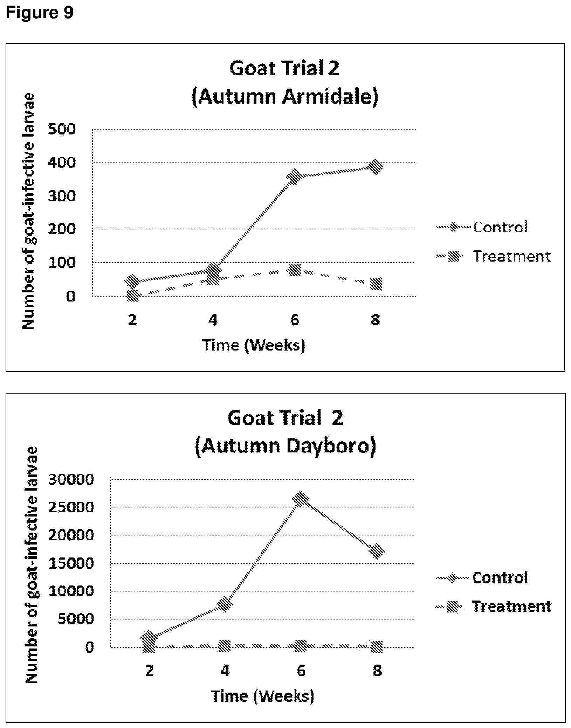

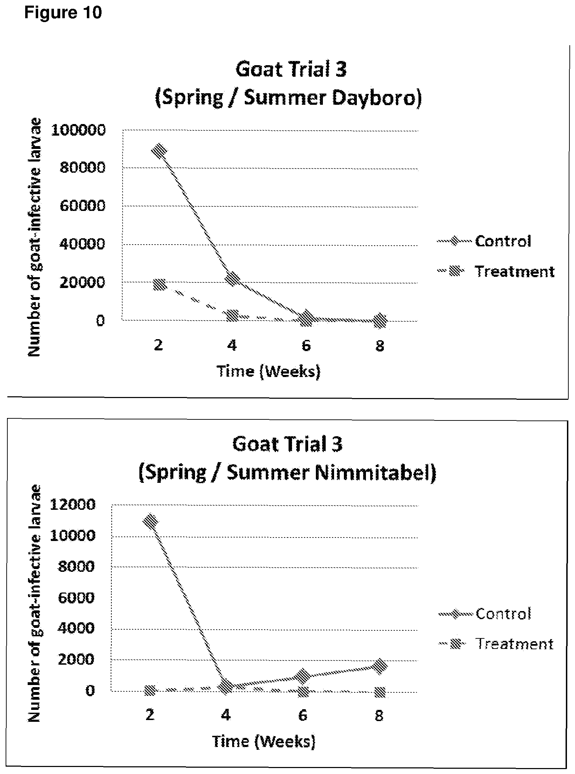

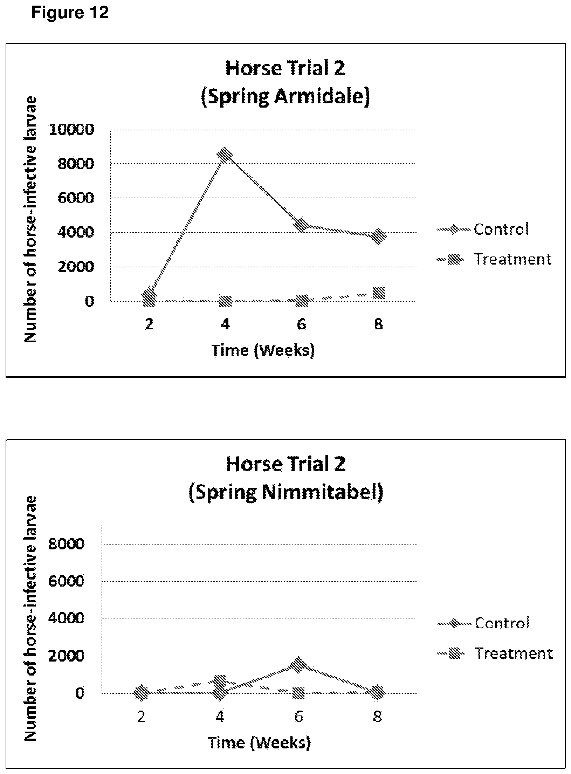

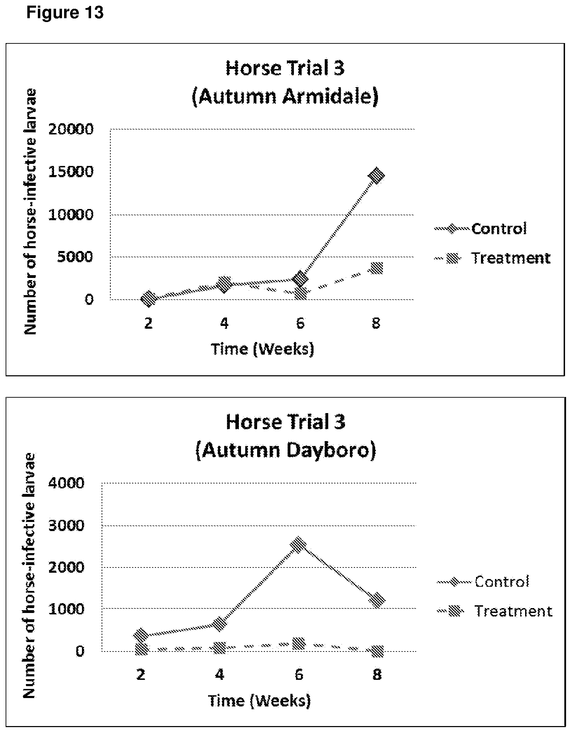

Studies summarised below were conducted according to VICH GL9 GCP (June 2000) and WAAVP Guidelines for evaluating the efficacy of anthelmintic in ruminants. Results are summarised in Table 3 and in Figures 6 to 13.

**Table 3. Overview of efficacy trials on supplementation with EP to reduce larval development and migration from faeces on pasture in cattle, goats and horses.**

| **Speci es** | **Number of animals** | **Dura tion of treat men t (day s)** | **Locatio n** | **Season** | **Result (% reductio n of larval emergen ce weeks 2-8)** | **Study Reference** |
|---|---|---|---|---|---|---|
| Cattle | 6 | 6 | Armidale NSW | Spring | 75 | Cattle Trial 1 |
| Cattle | 6 | 6 | Nimmita bel NSW | Spring | 75 | Cattle Trial 1 |
| Cattle | 6 | 7 | Armidale NSW | Autumn | 82 | Cattle Trial 2 |
| Cattle | 6 | 7 | Dayboro QLD | Autumn | 88 | Cattle Trial 2 |
| Goat | 6 | 7 | Armidale NSW | Spring | 85 | Goat Trial 1 |
| Goat | 6 | 7 | Nimmita bel NSW | Spring | 8* | Goat Trial 1 |
| Goat | 12 | 9 | Armidale NSW | Autumn | 81 | Goat Trial 2 |
| Goat | 12 | 9 | Dayboro OLD | Autumn | 99 | Goat Trial 2 |
| Goat | 12 | 7 | Dayboro QLD | Spring/Sum mer | 80 | Goat Trial 3 |
| Goat | 12 | 7 | Nimmita bel NSW | Spring/Sum mer | 98 | Goat Trial 3 |
| Horse | 5 | 7 | Armidale NSW | Autumn | 94 | Horse Trial 1 |
| Horse | 6 | 5 | Armidale NSW | Spring | 97 | Horse Trial 2 |
| Horse | 6 | 5 | Nimmita bel NSW | Spring | 53 | Horse Trial 2 |
| Horse | 6 | 5 | Armidale NSW | Autumn | 65 | Horse Trial 3 |
| Horse | 6 | 5 | Dayboro QLD | Autumn | 94 | Horse Trial 3 |

| | | | | | | |
|---|---|---|---|---|---|---|
| *Trial site affected by freezing conditions shortly after deposition of faeces. | | | | | | |

Statistical analysis (Groves, P 2015) shows that the treatment effect was statistically significant (p≤0.01) for all species (See Example 7).

In the cattle trials the nematode types encountered were *Cooperia* spp., *Trichostrongylus* spp., *Oesophagostomum* spp., *Ostertagia* spp. and *Haemonchus* spp, including multi-resistant strains.

In goats the nematodes were *Teladorsagia* spp., *Trichostrongylus* spp., *Nematodirus* spp., *Haemonchus* spp. and *Cooperia* spp. including multi-resistant strains. Goats were also challenged with a mixture of gastrointestinal strongyle larvae, including known multi-resistant strains of *Haemonchus contortus, Trichostrongylus colubriformis* and *Teladorsagia circumcincta* and recent field isolates of *Cooperia* spp. and *Nematodirus* spp.

In horses the dominant species were cyathostomes, however some *Strongylyus* spp. and *Trichostrongylus axei* were also present.

The pathogenic genera of gastrointestinal nematode parasites of domesticated livestock are cosmopolitan in their distribution although differences in prevalence and abundance occur due to climatic differences. The predominant genera in Australia are represented in most geographic locations where livestock production occurs throughout the world.

### Long term efficacy studies with EP (sheep)

A series of placebo-controlled trials were conducted to evaluate the ability of the EP to reduce larval development and migration from faeces on pasture in sheep in a range of geographic locations and seasons as outlined below.
1. Livamol^{®}: Placebo Product - a nutritious animal feed supplement manufactured by International Animal Health Products Pty Ltd, referred to as Livamol^{®} below.
2. EP - comprising TGAI dispersed in Livamol^{®} and referred to as "EP" below or "the test composition".

In each of these studies the groups of sheep used had different roles:
(1) "seeder" sheep, which harboured natural infections of a range of parasitic worms representative of the region (including multi-resistant strains), were used to contaminate the pasture by dropping faeces bearing worm eggs.
(2) "tracer" sheep, which were young worm-susceptible animals, free of any worm burden, which were used to assess the degree of worm-contamination of the pasture on which they grazed.

In each trial a pair of matched paddocks was used, where one paddock was grazed with a group of seeder sheep that received a daily supplement of the placebo (Control Group) and the other by a matching group that received a daily supplement of EP, providing a daily dose of minimum viable 3 × 10⁴ spores/kg bodyweight (Treatment Group).

After the seeder sheep had grazed the paddocks for two months, the degree of contamination of the pasture by infectious nematode larvae was assessed by grazing the paddocks with worm-susceptible tracers for a period of 3 weeks. One group of tracers grazed on the Control paddock and a matching group grazed the EP paddock. The tracer animals were then removed to raised pens to allow their worm burdens to mature and their degree of infection was determined by conducting a Total Worm Count (TWC) after sacrifice and gut washing. In three of the trials the seeder sheep continued to graze the trial paddocks for a further two months at which time another group of tracers were introduced and their TWC was determined.

The difference in TWC between the two groups of tracers demonstrated the ability of EP to prevent the emergence of infectious larvae from the droppings of the seeder sheep onto the pasture.

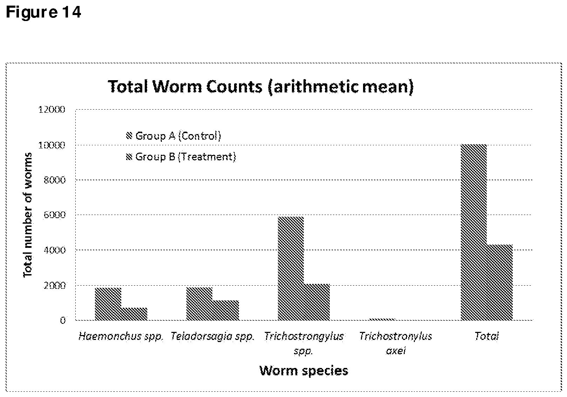

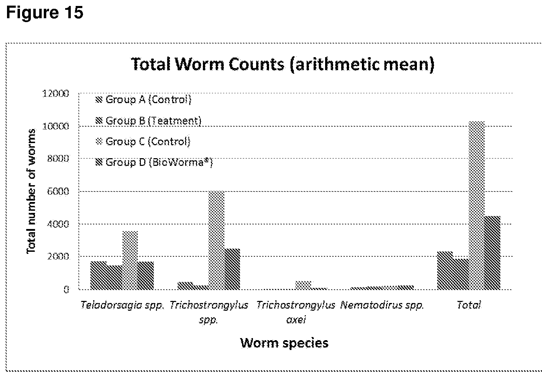

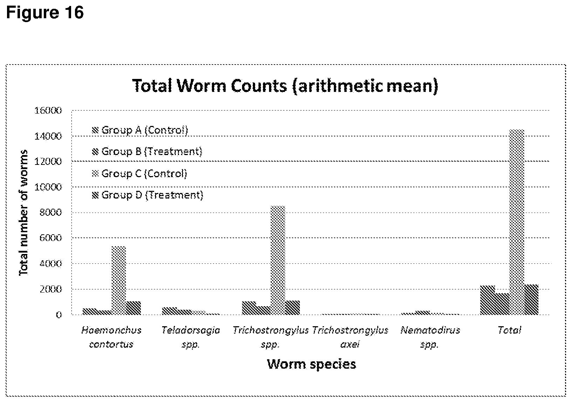

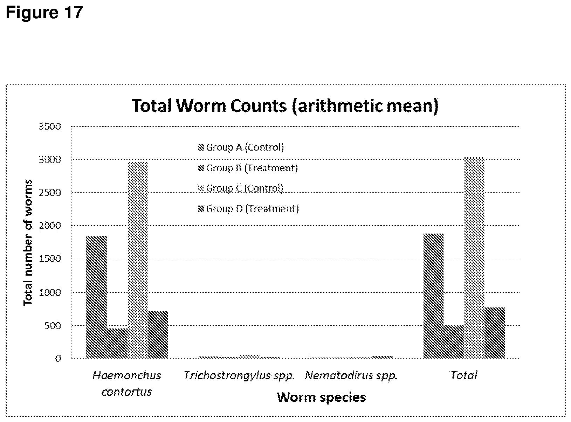

Results are summarised in Table 4 and in Figures 14 - 17. By naturally reducing the larval infectivity of the pasture, use of EP resulted in significantly lowered worm burdens for the most clinically-important species of worms encountered in the trials, including multi-resistant strains.

**Table 4 Overview of efficacy trials on supplementation with EP to reduce larval development and migration from faeces on pasture in sheep**

| **Species** | **Seeder animals** | | **Location** | **Season** | **Tracer animals** | | **Reference** |
|---|---|---|---|---|---|---|---|
| | No. of Animals | Duration of Treatment | | | No. of Animals* (duration) | Reduction Total Worm count§ | |
| Sheep | 20/20 | 57 days | Armidale NSW | Summer/ Autumn | 23/23 (21 days) | 57% (Week 14) | Sheep Trial 1 |
| Sheep | 30/30 | 119 days | Nimmitabel NSW | Spring/ Summer | 10/10 (21 days) | 20% (week 11) | Sheep Trial 2 |
| | | | | | | 57% (week 18) | |
| | | | | | 10/10 (21 days) | | |
| Sheep | 30/30 | 122 days | Armidale NSW | Spring/ Summer | 10/10 (21 days) | 26% (week 11) | Sheep Trial 3 |
| | | | | | | 84% (week 18) | |
| | | | | | 10/10 (21 days) | | |
| Sheep | 30/30 | 125 days | Gore, QLD | Summer/ Autumn | 10/10 (21 days) | 74% (week 11) | Sheep Trial 4 |
| | | | | | | 75% (week 18) | |
| | | | | | 10/10 (21 days) | | |

| | | | | | | | |
|---|---|---|---|---|---|---|---|
| *Number of control/treated animals, § arithmetic means # Days of treatment while on test paddocks | | | | | | | |

The worm genera encountered in these trials includes Haemonchus, Trichostrongylus, Teladorsagia and Nematodirus.

Other grazing animals that have been tested wherein EP provided large reductions in total worm counts include giraffe, antelope, zebra and blackbuck.

These studies confirm that *D. flagrans* of the invention survives gut passage and efficiently traps a wide variety of commercially-significant nematode parasites of grazing animals including cattle, sheep, horses, goats and zoo animals. These field studies have confirmed the composition of the invention is suitable for commercial application to reduce worm infestation on pasture and so manage the levels of infection in the animals themselves, as well as demonstrating economic benefit such as reduced disease & mortality, reduced need for use of chemical wormers and improved performance.

The required daily dose of *D. flagrans* strain IAH 1297 is approximately 10x lower than studies using other strains of *D. flagrans.* This represents a significant advantage over other strains of *D. flagrans.*

### EXAMPLE 8

### Statistical analysis of short-term studies of Example 7

At each time point at each trial site the mean numbers of parasite larvae for all the Control and test composition samples were calculated. In order to determine the impact of test composition treatment on larval numbers on pasture across trials, the data from each site for the multi-site trials were averaged and then, for each species of animal, the data from all studies were pooled for analysis by time period on pasture (2, 4, 6, 8 weeks and total for weeks 2-8). From this the overall mean larval numbers (Control and test composition) at each time point and total for weeks 2-8 were calculated.

The larval determinations at each time interval were not independent as for each trial the samples all came from a pooled composite from the same experimental animal. It is therefore appropriate to consider these are not completely independent and to use a repeated measure analysis (ANOVA) for comparison of the results. Hence the data were analysed using Repeated Measure ANOVA, with Non-treated faeces (Control) versus Treated faeces (test composition) and trial number as the main effect variables. All interactions were included in the analysis.

### Horse Trials

Three trials were compared, conducted using EP in horses over 5 geographic sites over 2 seasons. The sites differed between trials so only trial number was used as a variable in the analysis. The ANOVA results are shown in Table 5.

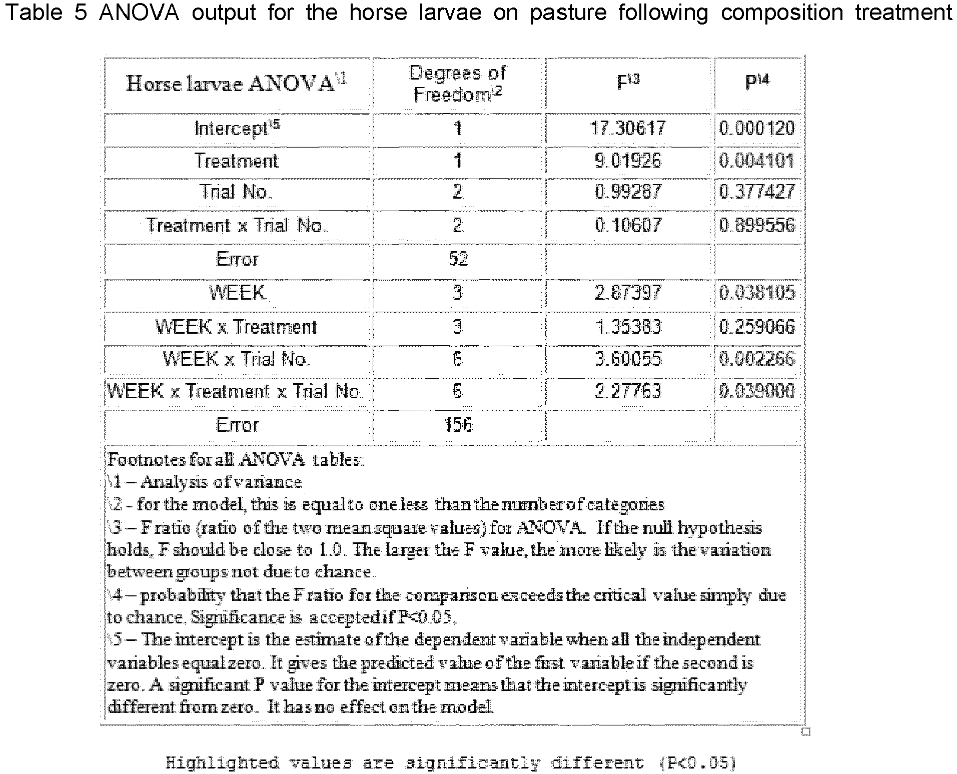

Table 5 shows that the effect of treatment with EP over the 8 week observation period was statistically-significant as was the week of sampling after treatment. There were significant interactions between the time after treatment and the trial and between time, trial and treatment.

Table 6 shows the mean numbers of horse parasite larvae in the pasture samples over the 8 week trial period for the 3 trials as well as the corresponding overall values. The overall mean larval count for the Control samples was 2248 larvae compared with only 363 larvae for the test composition treated samples (P=0.004).

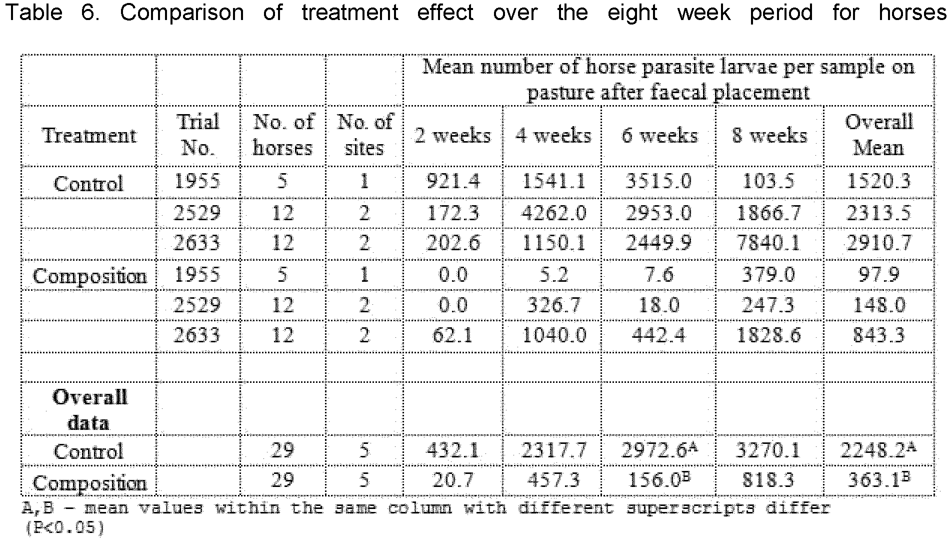

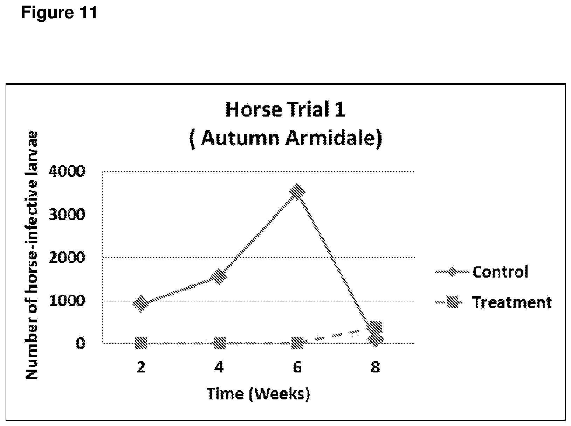

Figures 11 - 13 show that the burden of horse parasite larvae on pasture increased with time after placement of the faeces on pasture and this increased faster and maintained at much higher levels from the Control faeces compared with test composition treated faeces. Variability was marked across the trials but a significant difference was observed at 6 weeks post treatment (Table 6). Overall, the total number of larvae emerging from test composition treated faeces over the 8 week observation period was significantly reduced compared to the Control faeces (Tables 5 and 6).

This response varied across the three trials (Figs. 11 - 13) with more larvae found at 4 weeks post treatment in trial #2529, at 6 weeks in trial #1955 and a slower build up to a peak at 8 weeks observed in trial #2633. This may have been due to differing climatic and local conditions at each site.

### Conclusions from horse experiments

Overall, administration of EP to horses significantly (P=0.004) reduced the detection of horse parasite larvae on pasture surrounding faeces over an 8 week post treatment period.

### Cattle Trials

Two trials were compared, conducted using test composition in cattle over 4 geographic sites over 2 seasons. The sites differed between trials so only trial number was used as a variable in the analysis. The ANOVA results are shown in Table 7.

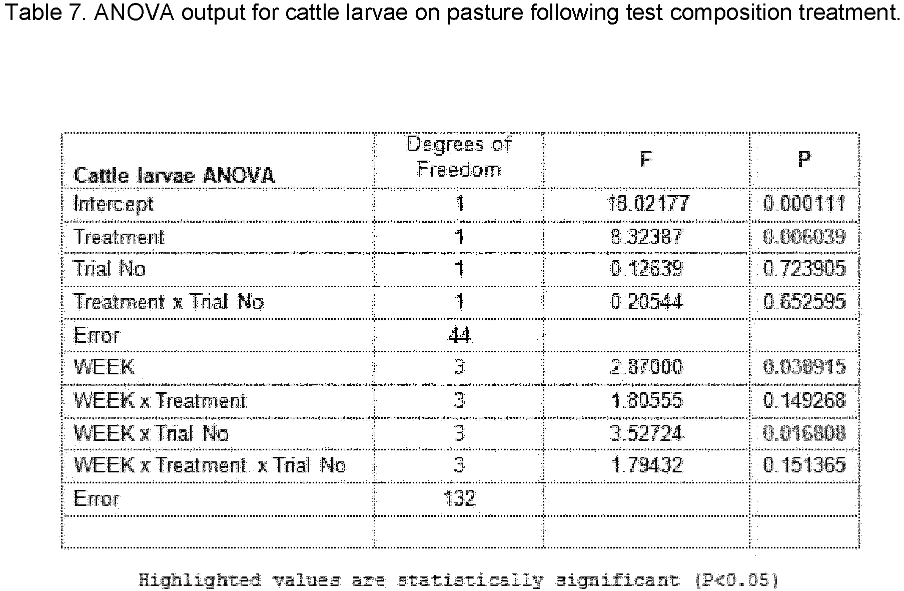

Table 7 shows that there were significant differences between EP treated and Control faeces over the 8 week study period and this varied over time. There was a significant interaction between the individual trials over time.

Table 8 shows the mean numbers of cattle parasite larvae in the pasture samples over the 8-week trial period for the 2 trials as well as the corresponding overall values. The overall mean larval count for the Control samples was 16,038 larvae compared to 3,059 larvae for the test composition treated samples (P= 0.006).

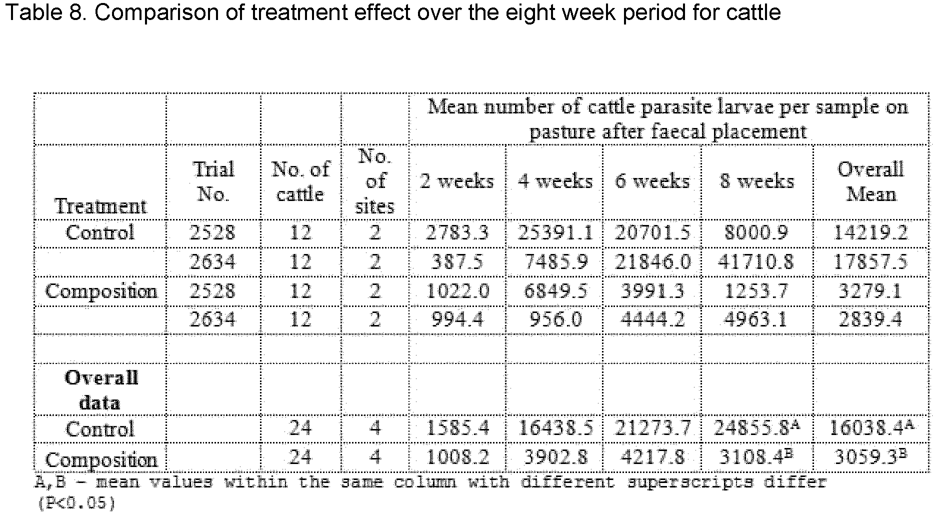

Figures 6 - 7 shows that larvae from the Control faeces increased rapidly by 4 weeks and thereafter remained at a high level through to 8 weeks post treatment. In comparison, larvae found emerging from test composition treated faeces rose only minimally over the entire observation period. Variability was marked across the trials but a significant difference was observed at 8 weeks post treatment. Overall, the total number of larvae emerging from EP-treated faeces over the 8 week observation period was significantly reduced compared to the Control faeces (Tables 7 & 8).

This response varied between the two trials (Figures 6 - 7), with larval values reaching a maximum at 4 weeks in trial # 2528, whereas in trial # 2634 there was a slower build up to a peak at 8 weeks. This may have been due to differing climatic and local conditions at each site.

### Conclusions from cattle experiments

Overall, treatment of cattle with EP significantly (P=0.006) reduced the detection of cattle parasite larvae on pasture surrounding faeces over an 8 week post treatment period.

### Goat Trials

Three trials were compared, conducted using EP in goats at 6 geographic sites over 3 seasons. Since goats have a relatively low faecal output, for two of the goat trials (#2635 and #2719) the faecal samples were collected from pairs of goats. The sites differed between trials so only trial number was used as a variable in the analysis. The ANOVA results are shown in Table 9

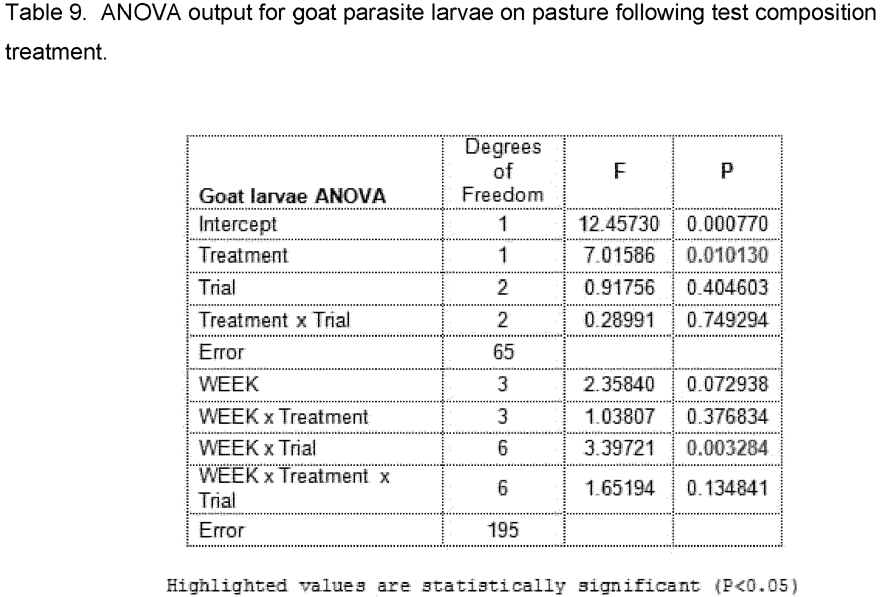

Table 9 shows that the effect of treatment with EP was statistically-significant over the 8 week observation period. There was also a significant interaction between time and trial.

Table 10 shows the mean numbers of goat parasite larvae in the pasture samples over the 8-week trial period for the 3 trials as well as the corresponding overall values. The overall mean larval count for the Control faeces was 12,866 larvae compared with only 1,834 larvae after test composition treatment (P=0.01).

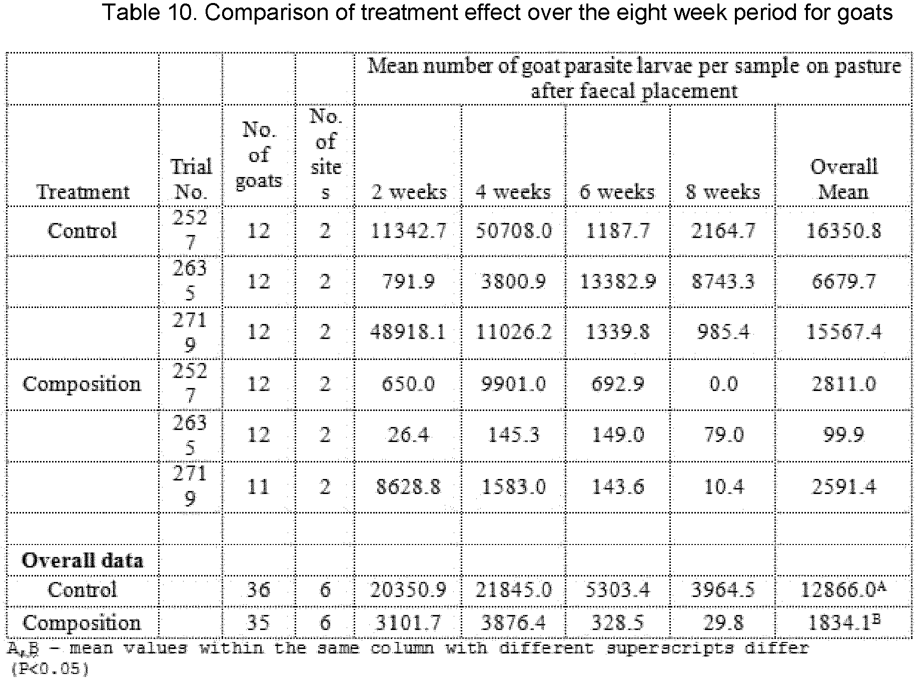

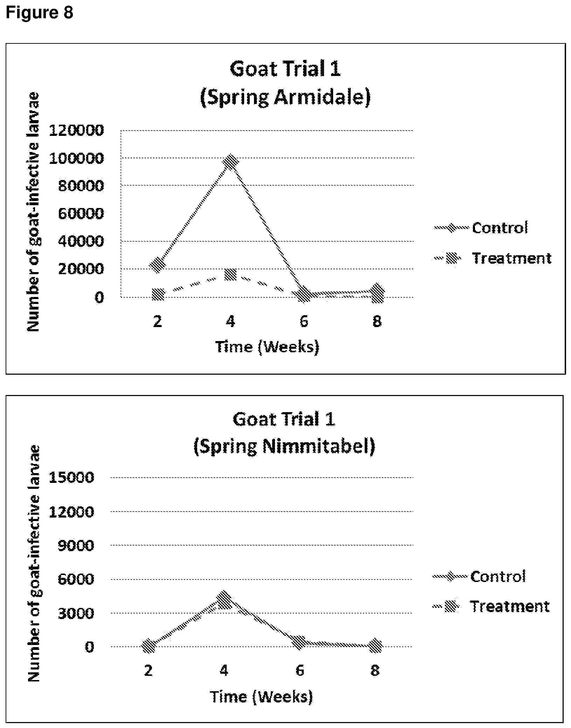

Figures 8-10 shows that the larval emergence seen differed in pattern to the horse and cattle studies in that for goat faeces, the parasitic larvae emergence was greater in the first 2 weeks post treatment and then declined. Variability was marked by week but overall, the total number of larvae emerging from composition treated faeces over the 8 week observation period was significantly reduced compared to the Control faeces (Tables 5 & 6).

This response varied across the three trials, with more larvae found at 2 weeks post treatment in #2719, 4 weeks in #2527 and 6 weeks in #2635.

### Conclusions

Overall, administration of the EP to goats significantly (p=0.01) reduced the detection of goat parasite larvae on pasture surrounding the faeces over an 8 week posttreatment period.

### Overall Conclusions

Treatment of horses, cattle and goats with the EP lead to significant (p≤0.01) reductions in parasite larvae of all three animal species on pasture surrounding their faeces over an 8 week post-treatment period.

### EXAMPLE 9

By way of non-limiting example only, the following table provides a summary of issues encountered in establishing safety and obtaining regulatory approval for a commercial biological control product of the invention.

**Table 11 Summary of Safety Issues**

| **Safety** | **Issue** | **How addressed** | **Time frame** |
|---|---|---|---|
| Infectivity/ pathogenicity | 1. Needed to prove that *D*. *flagrans strain* IAH 1297 was not infective / pathogenic. Lungs were most appropriate site for this investigation because they provide an aerobic environment. However, even individual spores were too big to enter the lungs by inhalation (20 microns) as they become trapped in nasal cavity and airways. | Method developed for direct instillation of powdered spore concentrate into trachea. Trial conducted to determine maximum tolerable dose. | Items 1-10 relate to the program of toxicology testing conducted in laboratory animals. |
| | | | Initial enquiries with a toxicology lab (RDDT Laboratories, Melbourne) began in May 2010. This lead to a series of rolling consultations with regulatory agencies, regulatory consultants and toxicologists to decide which tests were required, test protocol details and choice of test item. Testing began in December 2014 and last report was received in Jan 2016. |
| | | | Total time required 5 years 8 months. |
| | 2. Regulators required high dose (suggested 10⁸ spores). | Developed a process for purifying the TGAI to maximise the spore dose that could be administered | |
| | 3. Instillation of particulate matter induced physiological | Trial included control group with inactivated spores (gamma irradiation) | |
| | response that may be mistaken as adverse effect attributable to D. *flagrans* pathogenicity / toxicity (e.g. non specific pneumonia). | to demonstrate that symptoms were due to inhalation of particulate matter, not due to pathogenicity / toxicity of the spores | |
| | 4. Regulators required enumeration of spores in lung | Method developed and validated to enumerate spores in rat lung | |
| | 5. Regulators required demonstration of clearance of spores from the body, but clearance mechanisms in the lung aren't adept at clearance of 20 micron spores because particles of this size do not normally reach the lungs | Trial period was extended until clearance was finally achieved (6 weeks) | |
| Dustiness | 6. Needed to quantify the risk of inhalation of particulate matter by workers and animals | Dustiness test conducted showing feed supplement (MP) is "practically dust free" | |
| Particle size | 7. Needed to prove that finer particles are too large to be inhaled by workers and animals | Dry sieve analysis showed feed supplement (MP) has no particles less than 50 microns | |
| Acute oral toxicity | 8. Needed to show that *D*. *flagrans* strain IAH 1297 isn't toxic by oral administration. Regulators required as high dose as possible | Developed a process for purifying the (TGAI) to maximise the spore dose that could be administered at upper dose level used in such trials (5g/kg BW) | |
| Irritancy - Eye | 9. Needed to assess irritancy but EP and MP contain a known ocular irritant (calcium carbonate) | Eye irritancy studies (*in vitro* and *in-vivo*) conducted with EP | |
| Dermal irritancy and toxicity | 10. Calcium carbonate in EP and MP may also be a skin irritant | Dermal irritancy and toxicity studies conducted with EP | |
| Chronic oral toxicity | 11. Data from long term mammalian studies required | Addressed via long-term high-dose safety studies in target animals | Sheep study: Planning commenced July 2009, and study report received Feb. 2010. |
| | | | Total time required 7 months. |
| Target animal safety | 12. Need to demonstrate long-term safety under field conditions | Long-term (6-8 weeks) high-dose (5-10X) safety studies conducted in sheep, cattle and goats | Cattle and horse studies: Planning commenced March 2013, and study reports received Dec 2013. |
| | | | Total time required 9 months. |
| | 13. Large quantity of spores required (approx. 500kg feed supplement (EP) per trial with 10X spore inclusion rate, equivalent to 5 tonnes normal product) | Production of TGAI was scaled up. | Commenced January 2013, completed June 2013 |
| | | | Total time required 6 months |
| Residues | 14. Secondary metabolites (flagranones A, B and C) of D. *flagrans* were known, but no information was available about metabolite production by strain IAH 1297. | Conducted fermentation studies; showed that flagranone A is the single major metabolite of D. *flagrans* strain IAH 1297, that flagranones B and C are not produced and that other structurally-related materials are less than 5% of flagranone A | Project planning started October 2014, report received December 2015. |
| | | | Total time required 14 months |
| | 15. No published toxicology data was available for flagranones | Toxicological properties investigated by means of in-silico desktop studies using QSAR technology | Planning commenced April 2012, report received June 2016. |
| | | | Total time required 4 years. |
| | 16. No analytical reference material available for flagranone A | Purified flagranone A isolated and characterised by a specialist natural products chemist. | Phase 1: Project planning started April 2008, report received Oct 2008. |
| | | | Total time required 6 months |
| | | | Phase 2: Project planning started October 2014 report received December 2015. |
| | | | Total time required 14 months |
| | 17. Flagranone A pure reference material is unstable, but degraded material appears chromatographically pure, which results in inflated assay results | Stability study conducted with flagranone A. Pure material can be stabilised by storage in sealed ampoules in the dark at -80C, but not -20C | Project planning started October 2014, report received December 2015. |
| | | | Total time required 14 months |
| | 18. No analytical methods existed for assaying flagranone A in TGAI, MP and EP. | Methods developed and validated for assay of flagranone A in TGAI, MP and EP | Phase 1: Planning started April 2013, reports received March 2014. |
| | | | Total time required 11 months |
| | | | Phase 2: Planning started July 2015, report received December 2015. |
| | | | Total time required 5 months |
| | | Analysis of batches of TGAI showed that worst case daily intake by recipient animals were below the levels that trigger the requirement for feeding studies to assess residue levels in food products derived from recipient animals | Total time required 1 month, |
| | 19. No data was available on worker exposure to flagranone A | Using analytical data from TGAI and suitable exposure models it could be shown that worst-case exposure levels of manufacturing workers and farm workers handling feed supplement (EP) were below levels of concern | Total time required 1 month, |
| | 20. No data was available on intake levels for consumers eating products derived from feed supplement (EP)-treated animals | Using analytical data from TGAI it was shown that worst-case intake would be below levels of concern. | Total time required 1 month, |

### EXAMPLE 10

### Toxicology studies

By way of non-limiting example only, toxicology testing was conducted with the composition of the invention. The composition is also a biological control product. The composition of the invention was prepared for example, according to Example 3 and further formulated to produce EP and/or MP according to Example 4.

### Selection of test items

The test items for these studies were selected according to the objective and context of the test. The acute oral toxicity, and the acute pulmonary toxicity and infectivity studies were conducted with purified TGAI i.e. a concentrated preparation of the spores of D. *flagrans* strain IAH 1297 (the active constituent) to ensure that the highest possible dose of spores was administered in each case. Other studies, such as the ocular and dermal irritation tests, were conducted using EP, which was considered to be more relevant to users of the product.

### Data Summary and Overview

The suite of international standard Good Laboratory Practice toxicology studies that was carried out is summarised below; none of these studies indicated any cause for concern.

The acute oral toxicity study showed that the D. *flagrans* spores in the purified TGAI are not toxic. A dose of 5,000 mg/kg B.W. in rats did not cause any clinical signs and accordingly, the oral LD50 dose was greater than 5,000 mg/kg bodyweight.

In the acute pulmonary toxicity and infectivity study the spores of the purified TGAI were instilled directly into the lungs because the spore diameter of between 20-24 microns meant they were too large to be inhaled into the lungs. Instillation of spores into the lungs did not induce signs of toxicity or infectivity, apart from laboured breathing which was resolved in 3-4 days (the same result was obtained with attenuated product). The spores were eventually cleared from the lungs.

An acute dermal toxicity study conducted by application of EP to a dermal patch with the limit dose of 5,000 mg/kg bodyweight also indicated no toxicological effects.

An acute skin irritation test concluded that EP is classified as non-irritant when applied to rabbit skin. Testing of the feed supplement EP according the CIPAC test method TM171 for dustiness showed the product is defined as non-dusty, and this will contribute to the safety of the product.

An acute eye irritation study using EP concluded that conjunctival irritant effects occurred after 1 hour, but these effects were fully reversible within 72 hours. No corneal effects were observed. This information was supported by an in vitro eye irritation study with isolated chicken eyes where the product was not classified as either a severe irritant or non-irritant.

Long-term high-dose safety studies at 5x times the proposed dose in sheep and 10x times in cattle and horses have confirmed the safety of TGAI. Studies by other researchers support this finding.

The data provided supports regulatory approval for the composition or biological control product of the invention.

A number of relevant physical characteristics of MP were also determined, notably the dustiness according to the CIPAC test method TM171, and a sieve analysis according to CIPAC TM170. Using CIPAC criteria for dustiness, MP was found to be "nearly dust-free"; and the dry sieve analysis showed no material retained on, or passing through a 50 micron mesh. Also, a determination of the *D*. *flagrans* spore size is provided as there is a relationship between the aerodynamic size of any dust particle and its ability to be inhaled or respired. All of these test results provide evidence of the low-risk nature of the products.

Attention has also been paid to the secondary metabolites of *D*. *flagrans* strain IAH 1297. This strain produces a single major metabolite, flagranone A, and analysis has shown that this material is present at levels that are too low to be of toxicological concern.

A toxicological overview of *D*. *flagrans* strain IAH 1297 has been provided by a European Registered Toxicologist. It concludes:
*D. flagrans* acts in the faeces of the treated animals and is not absorbed into the systemic circulation; accordingly, no systemic toxicity due to the fungus is expected. This was confirmed through a series of toxicology studies in laboratory animals and long-term high-dose safety studies in target animals.
It was concluded that studies in laboratory animals and target species did not indicate any toxicological effects and that the risks posed by flagranone A (the secondary metabolite) to the consumer are negligible. Given the nature of the active constituent no further toxicological testing of the product or these substances is either practicable or necessary.

### Studies on the Active Constituent

### Infectivity and Pathogenicity:

These properties were assessed by means of the GLP acute pulmonary toxicity and infectivity study referred to below. The lung was considered to be the ideal organ for evaluation of infectivity and pathogenicity of *D*. *flagrans* because it has been shown that the spores require oxygen for germination and the lungs provide an aerobic environment, unlike the alternate test sites (gastrointestinal tract or peritoneal cavity).

Choice of test item was dictated by the need to maximise the spore dose that could be applied. The test item used was the purified TGAI, as it proved impossible to achieve a higher concentration via small scale laboratory trials. Since the spores have a diameter of approximately 20 microns it was decided to apply them by direct instillation into the trachea, bypassing the nose and throat where the spores might otherwise become trapped.

### Acute pulmonary toxicity & Infectivity Study.

An acute pulmonary toxicity and infectivity study was performed in the rat with animals treated with a single dose of *D*. *flagrans* spores IAH1297 in the form of a dry spore powder (purified TGAI), or an equivalent amount of inactivated spores, using an instillation method.

Necropsy observations confirmed that spore deposits spread within the lungs following insufflation, as required for this study. The only significant clinical signs observed were laboured respiration, noisy respiration, and increased respiration rate in all animals. These symptoms gradually resolved within the first 3-4 days.

Based on the results of this study, the test item, when administered to rats as a single intratracheal dose, does not induce signs of toxicity, infectivity, or pathogenicity. The results indicate that the maximum dose is determined by the size and physical surface of the spores, and not by their virulence. This is further confirmed by body weights increasing as clinical signs were reduced (after about 7 days) in animals treated with either active or inactivated spores. It is also noted, that after the 42-day period, living spores could not be detected in the lungs of the treated animals. The slow clearance of spores from the lung was considered to be related to the physical properties of the test material, particularly the large spore size.

### Acute Oral Toxicity.

An acute oral toxicity study was conducted with *D*. *flagrans* strain IAH 1297 spores in rats. In this study, 3 rats were given a single oral (gavage) dose of *D*. *flagrans* strain IAH 1297 spores (purified TGAI) at the limit dose of 5,000 mg/kg body weight (BW). The animals were fasted overnight prior to treatment and food was returned 3 hours after dosing.

Initially, one female was treated at a dose level of 5,000 mg/kg BW. No mortality was observed so two further rats (confirmatory group) were treated at 5,000 mg/kg BW. No mortality was observed in the confirmatory group.

Animals were observed individually after dosing for up to 6 hours post treatment and once daily for 14 days thereafter. Body weight was measured on Day -1, just before dosing (Day 0) and weekly thereafter. All animals were examined macroscopically at the end of the observation period. No specific pathogenicity evaluation was made, but close examination of animals was made for any adverse effects.

The study concluded:
- No mortality was observed.
- Treatment with *D*. *flagrans* spore (strain IAH 1297) at the dose level of 5,000 mg/kg bodyweight did not cause any clinical signs.
- There were no treatment related body weight changes. Body weights were within the range commonly recorded for this strain and age.
- There was no evidence of the macroscopic observations at a dose level of 5,000 mg/kg BW

Under the conditions of this study, the acute oral median lethal dose (LD₅₀) of the test item, *D. flagrans* spore strain IAH 1297 (purified TGAI), was greater than 5,000 mg/kg BW (limit dose) in female CRL:(WI) rats.

In this study, the quantity of spores fed to the animals is greater than 600-times the proposed commercial dose for TGAI in MP or EP as follows:
Study dose rate: 5 g of active constituent (the purified TGAI)/kg BW at 3.8 × 10⁶ spores/g (based on the assay result for the specific batch used in the trial) equals 1.9 × 10⁷ spores /kg BW
Proposed dose rate: 6 g MP/100 kg BW or 100g of EP/100kg bodyweight equals 30,000 spores/kg BW

Separately in 2006, the European Food Safety Authority (EFSA) published their opinion on the safety of *D*. *flagrans* as a feed additive for calves. The data considered included an acute oral toxicity study using 5 five female Sprague-Dawley-derived rats of 11-weeks old given a single dose of 2,000 mg/kg B.W. Animals were monitored for 14 days, killed by carbon dioxide inhalation and subject to necroscopy. The study concluded that no toxic effects were seen after dosing and at post mortem examination of organs examined except the lungs were of normal macroscopic appearance (lung abnormalities observed were attributed to the use of carbon dioxide to kill the rats).

EFSA concluded that the product was of low oral toxicity with an LD₅₀ greater than 2,000 mg/kg BW or 2 × 10⁷ spores/kg BW. EFSA concluded that the fungus has a very low oral toxicity.

### D.flagrans Spore Size

Determination of spore size gave results of 19 microns and 24 microns. This is relevant as this spore size is too large to be inspired into the lungs which mitigates any risk due to inhalation of the spores. Also, for this reason the pulmonary and infectivity study used separated spores as the test item, which was applied by direct intra-tracheal installation.

### Exposure Assessment

There is no exposure of consumers to *D*. *flagrans* because the fungus is not absorbed by the recipient animals.

Worker exposure to *D*. *flagrans* is minimised by ventilation, dust extractors fitted to manufacturing and packing equipment in the workplace and use of personal protective equipment.

A suite of toxicology and long-term safety studies have shown that *D*. *flagrans* strain IAH 1297 is a low risk material,

### Secondary metabolites of D. flagrans

Flagranones are secondary metabolites produced by *D. flagrans* and flagranones A, B and C have been reported. As shown in Example 11, It has been found that *D*. *flagrans* strain IAH 1297 (TGAI) does not produce flagranones Band C, and exposure of manufacturing workers, farmers and consumers to flagranone A from manufacturing and using TAGI, MP and EP, and from consuming products from treated animals have been determined. It was found that human exposure falls below the European Threshold of Toxicological Concern (TTC) value of 1.5 µg /kg BW/ day and animal exposure falls below the level above which residue studies are considered to be required (4 µg /kg BW/ day).

### Studies on the Product

### Acute Dermal Toxicity.

A dermal toxicity test was performed using EP as it was considered that the excipients might contribute to effects on the skin. In this study rats were treated with a single semi-occlusive dermal application of EP at the limit dose of 5,000 mg/kg BW. Sufficient water was used to dampen the test material to ensure good contact with the skin. The contact period was 24 hours, followed by a 14-day observation. Clinical observations along with a check of viability and mortality were performed on all animals at 1 hour and 5 hours after dosing and daily for 14 days thereafter. Body weight was measured prior to dosing on Day 0, Day 7 and 14. All animals were examined macroscopically at necropsy at the end of the observation period.

The results included:
- No mortality observed during the study.
- No adverse clinical signs were observed after treatment with the test item or during the 14 day observation period and no effects were observed at the site of application.
- There were no treatment related body weight changes.
- Body weights were within the range commonly recorded for this strain and age.
- There was no evidence of adverse effects at the dose level of 5,000 mg/kg BW at necropsy.

It was concluded that the median lethal dose (LD₅₀) EP after a single dermal administration was greater than 5,000 mg/kg bodyweight rats.

Acute eye irritation tests were performed using EP as it was considered as it represented the most realistic model of exposure. An *in vitro* eye irritation test was conducted using isolated chicken eyes with EP as the test item. The procedure involved applying the test item in a single dose onto the cornea of isolated eyes that have been obtained from slaughter animals.

After the zero reference measurements, the eye was held in horizontal position and test item was applied onto the centre of the cornea such that the entire surface of the cornea was covered. After 10 seconds, the surface was rinsed with saline. The positive control eyes were treated with 30 mg Imidazole. The negative control eye was treated with 30 µL of physiological saline. In the study, three test item treated eyes, three positive control treated eyes and one negative control treated eye were examined.

No significant corneal swelling was observed during the four hour observation period. Corneal opacity change (severity 0.5 or 1) and fluorescein retention change (severity 0.5 or 1) was noted on all test item treated eyes; furthermore, particles of test item were stuck to the cornea and could not be washed off during the study. Overall, the effects were clearly greater than a negative effect, but not sufficient to classify them as severe. The particles stuck to the cornea could potentially result in mechanical corneal damage in vivo.

The *in vitro* eye irritation indicated that EP is not classified as either a severe irritant or non-irritant. It was concluded further information would be required for classification.

### In-vivo eye irritation

Three young adult male New Zealand White rabbits were used in this trial and the test item (EP) was administered as a single dose into the conjunctival sac of the left eye with the untreated right eye serving as control. Irritation effects were scored at different times up to 72 hours as shown below after test item installation into the eye.

Observations with fluorescein staining were made approximately 24 hours before treatment and then 24, 48 and 72 hours after the treatment in all animals. Rabbits were treated with analgesic and anaesthetic as per the regulatory guideline. Results obtained from these three animals were used to classify the test item for irritation potential. No specific pathogenicity evaluation was made, but close examination of animals was made for any adverse effects. Irritation to the conjunctivae, iris and cornea are assigned a numerical score.

The following data was obtained:

### First animal (No: 1393) clinical observation:

At 1 hour after application, conjunctival redness (score 2), chemosis (score 1) and discharge (score 2) were noted in the rabbit. Test item remained in the eye sac at the one hour observation time point in the rabbit. At 24 hours after application, conjunctival redness (score 2), chemosis (score 1) and discharge (score 1) were noted in the rabbit.

At 48 hours after application, conjunctival redness (score 1) was noted in the rabbit. At 72 hours after application, no clinical signs and no conjunctival or corneal effects were observed.

### Second animal (No: 1398) clinical observation:

At 1 hour after application, conjunctival redness (score 2), chemosis (score 1) and discharge (score 2) were noted in the rabbit. Test item remained in the eye sac at the one hour observation time point in the rabbit. At 24 hours after application, conjunctival redness (score 2) and discharge (score 1) were noted in the rabbit.

At 48 hours after application, conjunctival redness (score 1) was noted in the rabbit. At 72 hours after application, no clinical signs and no conjunctival or corneal effects were observed.

### Third animal (No: 1390) clinical observation:

At 1 hour after application, conjunctival redness (score 2), chemosis (score 1) and discharge (score 2) were noted in the rabbit. Test item remained in the eye sac at the one hour observation time point in the rabbit. At 24 hours after application, conjunctival redness (score 2) and discharge (score 1) were noted in the rabbit.

At 48 hours after application, conjunctival redness (score 1) was noted in the rabbit. At 72 hours after application, no clinical signs and no conjunctival or corneal effects were observed.

Fluorescein staining was negative in all animals during the observation period. As no clinical signs were observed, the experiment was terminated after 72 hours' observation.

During the study, the control eye of each animal was symptom-free. The general state and behaviour of animals were normal throughout the experimental period. The bodyweights of all rabbits were considered to be within the normal range of variability. However, a slight body weight loss was noted in one animal (No.1393) during the observation period.

It was concluded that EP, applied to the rabbits' eye, caused conjunctival irritant effects at one hour after the treatment which were fully reversible within 72 hours. There were no corneal effects observed in the study.

### Acute Skin Irritation

An acute skin irritation study was undertaken using EP. Three (3) young adult New Zealand White rabbits were treated by topical, semi-occlusive application of test composition item (EP) to their intact shaved flanks. The test item was moistened with water to ensure good skin contact. The duration of treatment was 4 hours.

The scoring of skin reactions was performed at 1, 24, 48 and 72 hours after removal of the dressing. The primary irritation index (P.I.I.) was calculated by totalling the mean cumulative scores at 24, 48 and 72 hours and then dividing by the number of data points. No specific pathogenicity evaluation was made, but close examination of animals was made for any adverse effects.

In this study, the following results were obtained:
- The primary irritation index was 0.00.
- No local dermal signs were observed in the treated animals throughout the study.
- No clinical signs of systemic toxicity were observed in the animals during the study and no mortality occurred. As no clinical signs were observed at 72 hours after patch removal, the study was terminated after 72 hours observation.
- The body weights of all rabbits were considered to be within the normal range of variability.

According to the Draize classification criteria, EP is considered to be "not irritant" to rabbit skin (P.I.I. = 0.00).

A decrease of body weights was observed, but animals returned to their original body weights after approximately 7 days. After this period there were no treatment-related effects on body weight or body weight gain and body weights were within the range commonly observed for this strain and age of rat.

### Other Studies

### Dustiness study

A GLP dustiness study with MP according to the CIPAC test method MT171 showed that it was "nearly dust-free".

### Sieve Analysis

In addition, a GLP dry sieve analysis study with MP was conducted according to CIPAC TM170. The dry sieve analysis showed no material retained on, or passing through a 50 micron mesh.

Also, above the spore diameter for D. *flagrans* strain IAH 1297 was shown to be approximately 20 microns.

These results are significant as these characteristics will reduce the likelihood of exposure to the eye and skin and by inhalation.

### Long-term high-dose safety studies with TGAI in EP

Long term, high-dose safety studies for cattle, sheep and horses are summarised below.

### Long term safety study in cattle (10x dose)

A GCP study was conducted in cattle to confirm the safety of TGAI when administered to cattle at an elevated dose rate (ten times the normal dose) for eight weeks.

Twenty Angus cattle (female and male castrates, aged less than 12 months) were recruited from the same pool of animals and acclimatised to consumption of the placebo product (Livamol^{®}), 125 g/100 kg bodyweight/day). Following this, they were drafted into two groups of 10 animals based on sex and bodyweight. For the following 56 days Group 1 (Control Group) continued to receive the placebo product, while Group 2 (Test Group) were given 125 g/100 kg bodyweight/day of EP containing 10 times the usual amount of TGAI (equivalent to minimum 3 × 10⁵ spores/kg bodyweight/day). This dose is ten times that used for control of intestinal nematodes (3 × 10⁴ spores/kg bodyweight/day) in cattle.

Safety of TGAI was confirmed via repeated clinical examinations by veterinarians, bodyweight change over time and serum biochemistry and haematology, in comparison with untreated cattle. Detailed clinical examinations were conducted on Days -10, 3, 7, 14, 28 and 56, bodyweights were measured on Days -10, 7, 14, 28 and 56, blood samples were taken for serum biochemistry and haematology on days -10, 14 and 56.

No evidence of acute or chronic toxicity or infection (apart from theileriosis in two cattle in the control group) was observed during the course of the study. Overall, no adverse clinical, bodyweight gain, biochemical or haematological signs were observed during the study that could be associated with feeding of TGAI over an extended (56 day) period, the TGAI dose comprising 10x the number of *D*. *flagrans* spores required for control of intestinal nematodes in cattle.

### Long term safety study in horses (10x dose)

A GCP study was conducted in horses to confirm the safety of TGAI when administered to horses at an elevated dose rate (ten times the normal dose) for eight weeks.

Twenty horses (Warmblood, Thoroughbred and Australian Stock Horse; female, male castrate and male, aged 3 to 12 years) were recruited from the same pool of animals and acclimatised to consumption of the placebo product (Livamol^{®}, 125g/100 kg bodyweight/day). Following this, they were allocated to two groups of 10 animals based on bodyweight. For the following 56 days Group 1 (Control Group) continued to receive the placebo product, while Group 2 (Test Group) were given 125 g/100 kg bodyweight/day of EP containing 10 times the usual dose of TGAI (equivalent to minimum 3 × 10⁵ spores/kg bodyweight/day). This dose is ten times that used for control of intestinal nematodes (3 × 10⁴ spores/kg bodyweight/day) in horses.

Safety of TGAI was confirmed via repeated clinical examinations by veterinarians, bodyweight change over time and serum biochemistry and haematology, in comparison with untreated horses. Detailed clinical examinations were conducted on Days -1, 3, 7, 14, 27 and 56, bodyweights were measured on Days -1, 7, 14, 27 and 56, blood samples were taken for serum biochemistry and haematology on Days -1, 14 and 56.

No evidence of acute or chronic toxicity or infection was observed during the course of the study. Overall, no adverse clinical, biochemical or haematological signs were observed during the study that could be associated with feeding of TGAI over an extended (56 day) period, the TGAI dose comprising 10x the number of *D*. *flagrans* spores required for control of intestinal nematodes.

### Long term safety study in sheep (5x dose)

A GCP study was conducted in sheep to confirm the safety of TGAI when administered to sheep at an elevated dose rate (five times the normal dose) for six weeks.

Twenty (20) Merino ewes of approximately 12-13 months of age and weighing between 24.0 and 32.0 kg were acclimatised to consumption of the placebo product (Livamol^{®}, 100 g/100 kg/day). Following this they were allocated to two groups of 10 animals based on bodyweight. For the following 43 days Group 5 (Control Group) continued to receive the placebo product while Group 6 (Test Group) were given 100 g/head/day of EP containing 5 times the usual amount of TGAI (equivalent to minimum 1.5 × 10⁵ chlamydospores/kg bodyweight/day). This dose was five times the proposed usage rate for control of intestinal nematodes (3 × 10⁴ spores/ kg bodyweight/day) in sheep.

Safety of TGAI was confirmed via repeated clinical examinations by veterinarians, bodyweight change over time and serum biochemistry and haematology, in comparison with placebo treated sheep. Detailed clinical examinations were conducted, bodyweights were measured and, blood samples were taken for serum biochemistry and haematology on Days -1, 7, 15, 29 and 43.

No evidence of acute or chronic toxicity or infection was observed during the course of the study. Overall, no adverse clinical, biochemical or haematological signs were observed during the study that could be associated with feeding of EP over an extended (43 day) period, the EP dose comprising 5x the number of *D*. *flagrans* spores required for control of intestinal nematodes.

### Sub-chronic repeated dose oral toxicity studies

An Indian isolate of *D*. *flagrans* was screened for possible short-term toxicological effects using a mouse model. Following the feeding of 1 × 10⁶ spores per head daily for one month, no mortality of mice and no significant differences in body weights between fungus fed and control mice could be recorded. Fungus fed and unfed mice had similar general appearance, colour and reflex movements. No evidence of internal mycosis was observed in fungus fed and control mice and on tissue sections of liver, lungs and kidney.

### Determination of No Observed Adverse Effect Levels (NOAEL)

No adverse effects were noted in any acute toxicity study in laboratory animals nor in any medium term or chronic feeding study at intended dose levels of TGAI or at 10 fold the intended dose level of TGAI in cattle and horses and 5 times intended dose in sheep. These studies are supported by numerous studies in the public domain in cattle, sheep, goats and horses.

On this basis it was concluded that the NOAEL was greater than 60 g feed supplement (MP) per 100 kg BW/day corresponding to more than 3 × 10⁵ spores/kg BW/day of D. flagrans strain IAH 1297.

### Reproduction Studies (including prenatal developmental toxicity).

Since D. *flagrans* is not resorbed from the gastrointestinal tract of treated organisms or humans and in addition the spores do not germinate at 37 °C or in the absence of oxygen, there is no indication for reproductive toxicity.

### EXAMPLE 11

By way of non-limiting example only, residue testing was conducted with the composition of the invention. The composition of the invention was prepared for example, according to Example 3 and further formulated to produce EP and/or MP according to Example 4.

### Residues Data Summary

As a natural microorganism, *D. flagrans* IAH 1297 is orally administered e.g., as spores in the composition of the invention which are not absorbed and, thus, cannot form residues in edible tissues.

Anderson *et al.* (1999) reported on the identification of secondary microbial products, flagranones A, B and C which are produced by *D. flagrans.* The inventors have conducted further studies on the unique strain of *D*. *flagrans* IAH 1297. A study of the secondary metabolites of *D*. *flagrans* strain IAH 1297 found that it produced a single major metabolite identified as flagranone A. Other minor metabolites were found to be present at levels less than 5% of flagranone A and it is noteworthy flagranones B and C were not detectable. This study examined the chemistry of flagranone A and reported that the speed at which flagranone A is degraded under laboratory conditions means it is improbable the metabolite would have more than a brief transitory existence in an *in vivo* or environmental situation.

Due to a lack of available literature on toxicological properties of flagranones, an *in-silico* toxicological analysis using Derek Nexus and Leadscope was conducted on flagranones A, B and C across a range of endpoints and in a number of mammalian and bacterial species. This was required to characterise the potential toxicity of flagranones A, B and C and to assess the possibility that they may be present in food products so that any risk to consumers may be assessed. Flagranones B and C are not produced by *D. flagrans* strain IAH 1297 but were included for completeness. Also, a full Derek Nexus analysis was performed for all three flagranones. The genetic toxicity and rodent carcinogenicity suites of Leadscope were used for all three flagranones; in addition, flagranone A was analysed using the reproductive toxicity suite.

Flagranone A was predicted to be irritant and potentially sensitising but was not predicted to be mutagenic, carcinogenic or toxic to reproduction. *There were no structural alerts for genotoxicity.*

Following *in silico* analysis using Derek Nexus and Leadscope software(s), it was concluded that the potential presence of flagranones in MP or EP does not pose any undue toxicological hazard or risk to consumers. This is based on the following:
- That flagranones B and C, although predicted to be genotoxic and/or mutagenic, are not present in TGAI, MP or EP.
- The conclusion that flagranone A is unlikely to be mutagenic, carcinogenic or toxic to reproduction.
- That, although predicted to be irritant and potentially sensitising, the concentrations of flagranone A in tissues or animal products to be consumed are likely to be significantly below the concentrations at which such effects might be expressed in consumers.
- Review of the structures and calculated physical properties indicates that all three flagranones would be subject to rapid metabolism and elimination following absorption, meaning that it is highly unlikely that tissues consumed would have any residue of these compounds.

### Residue data

A range of commercial batches of *D*. *flagrans* strain IAH 1297 (TGAI) have been analysed for flagranone A using a validated analytical method, with results varying between 4.4 mg/kg and 73.6 mg/kg as shown below

**Table 12: Flagranone A Results in D. flagrans (TGAI) Spore Batches**

| **Analytical Report No.** | **Date** | **Batch Number** | **Flagranone A Result (mg/kg)** |
|---|---|---|---|
| 16-0021-6 | 19Jan 2015 | E03079A | 4.36 |
| 16-0021-1 | 19Jan 2016 | E03026 | 10.5 (10.1) |
| 16-0021-2 | 19Jan 2015 | E03039E | 14.4 |
| 16-0021-7 | 19Jan 2015 | E03079B | 15.6 |
| 16-0021-8 | 19Jan 2015 | E03105A | 53.2 |
| 16-0021-5 | 19Jan 2015 | E03064B | 48.1 |
| 16-0021-9 | 19Jan 2015 | E03105B | 71.6 |
| 16-0021-4 | 19Jan 2015 | E03064A | 73.9 |
| 16-0021-3 | 19 Jan 2015 | E03039F | 19.8 |
| Average (mg/kg) | | | 34.6 |

Based on the table above a level of 100 µg/g was selected as a conservative maximum concentration, being approximately three times the average value.

When the proposed doses of MP and EP are taken into consideration the calculations of Flagranone A are as shown below:

### Residues in Animals

### In MP

Flagranone A content in *D*. *flagrans* IAH 1297: Maximum level in TGAI: 100 mg/kg or 100 µg/g (conservative value) Dose rate of MP: 6 g/100 kg BW/day or 0.06 g/ kg BW/day. Therefore, this gives a maximum daily flagranone A intake by animals: 34.6 µg/g × 0.06 g/kg BW/day = 2.1 µg/kg BW/day.

### In EP

Flagranone A content in *D*. *flagrans* IAH 1297: 100 mg/kg or 100 µg/g (conservative maximum). Dose rate of EP: 100 g/100 kg BW/day or 1 g/kg BW/day. Therefore, this gives a maximum daily flagranone A intake by animals: 2.2 µg/g × 1 g/kg BW/day = 2.2 µg/kg BW/day.

In Regulation (EU) No 283/2013 on data requirements for pesticides in the EU, chapter 6.4., the trigger value for requirement of residue analysis in animal tissues (muscle, liver and milk) is 4 µg/kg BW/day of the substance in animal feed:
"The objective of feeding studies shall be to determine residues in products of animal origin which result from residues in feed. Feeding studies shall not be required where intake is below 0.004 mg/kg bw/day".

Thus, the exposure level for treated animals is well below the trigger value for residue analysis laid down in the pesticide Regulation (EU) No 283/2013. Hence, no residue studies are necessary.

Flagranone A is described as readily-degradable and the chemical structures indicate that these are prone to rapid metabolic degradation in mammalian organism, thus there is no potential for accumulation.

### Residue in consumers

Considering that the above calculated maximum intake of flagranone A for MP and EP treated animals of 2.2 µg/kg BW/day is distributed to the edible animal tissues (ignoring metabolic degradation and chemical instability of the flagranone). Considering in a conservative estimate that 12.74 g meat is consumed per kg BW per day, exposure would be:

2.2 µg/kg animal tissue/day × 0.0127 kg animal tissue/kg BW/day = 0.0279 µg/kg BW/day

This result is well below the threshold of toxicological concern (TTC), even for the most critical chemical structures (EFSA, 2012), based on the Cramer Classification system outlined below.

The following human exposure threshold values are conservative estimates used in EFSA's work:
- 0.0025 µg/kg B.W for substances with a structural alert for genotoxicity,
- 0.3 µg/kg B.W for organophosphate and carbamate substances with anticholinesterase activity,
- 1.5 µg/kg B.W for Cramer Class III and Cramer Class II substances,
- And 30 µg/kg B.W for Cramer Class I substances.

Taking into consideration that an *in-silico* analysis of flagranone A indicated no structural alerts for genotoxicity, the conservative estimated maximum intake of 0.0279 µg/kg BW/day is more than 53-fold below the relevant TTC value of 1.5 µg/kg BW/day. It is concluded that exposure to flagranones with EP or MP is of no toxicological concern for the consumer.

### Residue of unknown metabolites other than flagranones

Other than flagranones, unknown minor metabolites were detected in *D*. *flagrans* strain IAH 1297 at levels below 5% of the level of flagranones.¹⁸ The following maximum residue levels of these minor metabolites are calculated as 5% (maximum) of flagranone A:
In animal:
- 2.1 × 5% = 0.105 µg/kg BW/day, when MP is fed
- 2.2 × 5% = 0.11 µg/kg BW/day, when EP is fed

Both results are much lower than the 4 µg/kg BW/day trigger level, hence, no residue studies are necessary.

In consumers:
- 0.0279 × 5% = 0.001395 µg/kg BW/day
This is below the TTC value of 0.0025 µg /kg BW/ day even for substances with a structural alert for genotoxicity.

Consumer exposure to flagranone A via consumption of products from treated animals is less than 0.0279 µg /kg BW/day which is more than 53 fold below the relevant TTC value of 1.5 µg /kg BW/ day. As the exposures are 53 to 625 fold below the TTC, flagranone A can be presumed to present no appreciable human health risk.

### Residue exposure for manufacturing and farm workers

A summary is provided below:
Farm worker/user exposure to the secondary metabolite flagranone A was determined to be less than 0.0135 µg/kg BW/day which is more than 111 fold below the relevant TTC value of 1.5 µg /kg BW/day.

Manufacturing worker exposure to flagranone A was determined to be less than 0.0024 µg/ kg BW/day which is 625 fold below the relevant TTC value of 1.5 µg /kg BW/ day.

### Analytical Methodology

### Fungal Concentrates (TGAI)

The method development, validations and results for flagranone A in TGAI is summarised below.

The method has been developed and validated in accordance with SANCO/3030/99 rev.4 11/07/00, APVMA Guidelines for the Validation of Analytical Methods for Active Constituent and U.S. EPA Test Guidelines, OPPTS 830.1800 in a GLP study.

Scope: the method is applicable to the determination of flagranone A in TGAI. The method was validated over the linear range 0 -1000 mg/kg flagranone A in TGAI with recovery levels over the range of 10 - 600 mg/kg.

Reference standard of flagranone A had a purity of 99.5%. The reference material was stored in a freezer on dry ice until prepared as a solution.

Method of Analysis: Residues of flagranone A were extracted from 10 g of blended and homogeneous TGAI (fermented grain) samples. Samples were centrifuged and an aliquot of the supernatant is taken, diluted, filtered and analysed for flagranone A using high performance liquid chromatography (HPLC) with UV detection.

Selectivity/Interference: The amount of interference (if any) was evaluated by comparing the results from the analysis of control samples and reagent blanks with an LOQ standard. Chromatograms were considered acceptable if components present in control extracts and reagent blanks, which could potentially interfere with the analysis, were not present at levels of greater than 3% of the limit of quantitation. No interfering substances were observed greater than 3% of the Limit of Quantitation (LOQ).

Linearity: In order to establish the linearity of response of the analytical chromatography system to the test substance, ten flagranone A standards of increasing concentration were prepared. The concentration range spanned at least 80-120% of the expected concentrations in the recovery samples. The standard curve was constructed with the following concentrations of flagranone A: 0.005, 0.01, 0.05, 0.1, 0.5, 1, 5, 10, 50 and 100 mg/L. The system linearity was accepted where a statistical analysis showed significant linearity with r² > 0.99. Each calibration curve had a correlation coefficient (r²) of ≥ 0.99 thereby showing sufficient linearity.

Precision and Accuracy (Recovery): The 10, 50 and 150 mg/kg recovery samples were prepared by adding aliquots of the stock solution of flagranone A onto 10 g portions of each control grain. The 600 mg/kg recovery samples were prepared by weighing approximately 6 mg of flagranone A onto 10 g portions of each control grain. The 10, 50, 150 and 600 mg/kg concentration levels when converted to % w/w are 0.001, 0.005, 0.015 and 0.06%, respectively. Therefore, the acceptable mean recovery range for the data in this method validation study is 75-125%.

An aliquot of the extract was taken, filtered into LC vials and analysed according to the method. The recovery samples were fortified at 10, 50, 150 and 600 mg/kg in fungal concentrates. Results were in the range 98-105%.

The precision of the method was demonstrated by analysing five replicates at 10, 50 and 150 mg/kg validation levels in fungal concentrates. The relative standard deviations (RSD) were determined at the 10, 50 and 150 mg/kg fortification levels. Precision was considered acceptable if the RSD was ≤ 20%. The precision of the recovery data for this study met the acceptance criteria.

Limit of Quantitation (LOQ): The LOQ is determined by analysing the estimated LOQ concentration a minimum of 6 times. The average response (X) and standard deviation (SD) is calculated. The standard deviation should be < 20%. The LOQ is calculated as: X + (10 × SD). The estimated LOQ is 1.1 mg/L which is equivalent to 11 mg/kg in sample.

Limit of Detection (LOD): The LOD is determined by analysing the lowest calibration standard which produces a peak (minimum of 6 replicates). The average response (X) and standard deviation (SD) is calculated. The LOD is calculated as: X + (3 × SD). The estimated LOD is 0.005 mg/L which is equivalent to 0.04 mg/kg in sample.

System Precision: The 1 mg/L flagranone A standard solution was injected ten times to assess the system precision. The system precision was considered acceptable if the RSD ≤ 1% for retention time and ≤ 20% for peak area. The RSD for the retention time was found to be 0.1% and the RSD for the peak area was found to be 6.9%.

Method Precision: The method precision was evaluated by analysing at least five freshly prepared recoveries fortified at 10 mg/kg over two days with freshly prepared standards for each analysis. The within-run relative standard deviation and between- run relative standard deviation was considered acceptable if ≤ 20% for the 10 mg/kg fortification level. The relative standard deviation for the within-run was found to be 2.7% on day 1 and 0.8% on day 2 and the between-run relative standard deviation was found to be 1.9%.

Solution Stability: Aliquots of the final extracts of untreated control grains were fortified with flagranone A at concentrations of 1 mg/L and 15 mg/L (equivalent to the 10 mg/kg and 150 mg/kg recovery levels in the final extract). Aliquots were also fortified with flagranone A at concentrations of 1 mg/L and 15 mg/L. The concentration of the analyte was quantified against freshly prepared neat standards after 7 days storage at approximately -20 °C in the dark. Freshly prepared final extracts were fortified at equivalent concentrations to compare against the 7 day stability samples. Results were in the range 101-107%.

Method Robustness: The method ruggedness was tested by re-analysing a previously prepared set of standards and recoveries and varying the make-up of the mobile phase. The remainder of the HPLC-UV conditions remained unchanged. Adjusting the mobile phase composition had no major impact on the response to flagranone A

It was concluded that this method is fit for the purpose of analyzing flagranone A in TGAI. The parameters validated in this study meet the acceptance criteria according to SANCO/3030/99 rev.4 11/07/00 APVMA Guidelines for the Validation of Analytical Methods for Active Constituent and U.S. EPA Test Guidelines, OPPTS 830.1800.

### MP and EP.

The method development, validations and results for flagranone A in MP and EP are summarised below.

The method has been developed and validated in accordance with SANCO/3030/99 rev.4 11/07/00, APVMA Guidelines for the Validation of Analytical Methods for Active Constituent and U.S. EPA Test Guidelines, OPPTS 830.1800 in a GLP study.

Scope: the method is applicable to the determination of flagranone A in MP and EP. The method was validated over the linear range 0 - 100 mg/kg flagranone A in fungal concentrates with recovery levels over the range of 1 - 60 mg/kg.

Reference standard of flagranone A had a purity of 99.5%. The reference material was stored in a freezer on dry ice until prepared as a solution.

Method of Analysis: Residues of flagranone A were extracted from 10 g of homogeneous samples by tissumising and shaking. Samples were centrifuged and an aliquot of the supernatant is taken and diluted, filtered and analysed for flagranone A.

Selectivity/Interference: The amount of interference (if any) was evaluated by comparing the results from the analysis of control samples and reagent blanks with an LOQ standard. Chromatograms were considered acceptable if components present in control extracts and reagent blanks, which could potentially interfere with the analysis, were not present at levels of greater than 3% of the limit of quantitation. Interfering substances in the control samples were between 0.3-0.4% of the LOQ

Linearity: In order to establish the linearity of response of the analytical chromatography system to the test substance, ten flagranone A standards of increasing concentration were prepared. The concentration range spanned at least 80-120% of the expected concentrations in the recovery samples. The standard curve was constructed with the following concentrations of flagranone A: 0.0005, 0.001, 0.0025, 0.005, 0.01, 0.025, 0.05, 0.1, 0.25 and 0.5 mg/L. The system linearity was accepted where a statistical analysis showed significant linearity with r² > 0.99. Each calibration curve had a correlation coefficient (r²) of ≥ 0.99, thereby showing sufficient linearity

Precision and Accuracy (Recovery): The recovery samples were prepared by adding aliquots of a solution prepared from the reference standard of flagranone A onto 10 g portions of each control animal feed.. An aliquot of the extract was taken, diluted and filtered into LC vials and analysed according to the method. The recovery samples were fortified at 1, 5, 20 and 60 mg/kg in MP and EP. The acceptable mean recovery range for the data in this method validation study was 75-125% and the average value found was 105%.

The precision of the method was demonstrated by analysing five replicates at each validation level in MP and EP. The relative standard deviations (RSD) were determined at each fortification level. Precision was considered acceptable if the RSD was ≤ 20%. The precision of the recovery data for this study met the acceptance criteria

Limit of Quantitation (LOQ): The LOQ is determined by analysing the estimated LOQ concentration a minimum of 6 times. The average response (X) and standard deviation (SD) is calculated. The standard deviation should be < 20%. The LOQ is calculated as: X + (10 × SD). The estimated LOQ was 0.005 mg/L.

Limit of Detection (LOD): The LOD is determined by analysing the lowest calibration standard which produces a peak (minimum of 6 replicates). The average response (X) and standard deviation (SD) is calculated. The LOD is calculated as: X + (3 × SD). The estimated LOD is 0.0005 mg/L.

System Precision: The 1 mg/L flagranone A standard was injected ten times to assess the system precision. The instrument conditions are listed in Section 3.5. The system precision was considered acceptable if the RSD ≤ 1% for retention time and ≤ 20% for peak area. The relative standard deviation for the retention time was found to be 0.18% and the relative standard deviation for the peak area was found to be 3.8%.

Method Precision: The method precision was evaluated by analysing at least five freshly prepared recoveries fortified at 1 mg/kg over two days with freshly prepared standards for each analysis. The within-run relative standard deviation and between-run relative standard deviation was considered acceptable if ≤ 20% for the 1 mg/kg fortification level. The relative standard deviation for the within-run was found to be 6.4% on day 1 and 5.4% on day 2 and the between-run relative standard deviation was found to be 6.0%.

Solution Stability: Aliquots of the final extracts of untreated animal feeds were fortified with flagranone A at concentrations of 0.005 mg/L and 0.1 mg/L (equivalent to the 1 mg/kg and 20 mg/kg recovery levels in the final extract). Aliquots were also fortified with flagranone A at concentrations of 0.005 mg/L and 0.1 mg/L. The concentration of the analyte was quantified against freshly prepared neat standards after 7 days storage at approximately -20 °C in the dark. Freshly prepared final extracts were fortified at equivalent concentrations to compare against the 7 day stability samples. Samples were analysed using the conditions listed above. Results were in the range 95-102%.

Method Robustness: The method ruggedness was tested by re-analysing a previously prepared set of standards and recoveries and varying the make-up of the mobile phase 'A'. The remainder of the HPLC-UV conditions remained unchanged. Adjusting the mobile phase composition had no major impact on the response to flagranone A and the method was considered to be robust.

It was concluded that this method is fit for the purpose of analysing flagranone A in MP and EP. The parameters validated in this study meet the acceptance criteria according to SANCO/3030/99 rev.4 11/07/00APVMA Guidelines for the Validation of Analytical Methods for Active Constituent and U.S. EPA Test Guidelines, OPPTS 830.1800.

## Claims

1. A composition comprising a biologically pure isolate of the micro-organism *Duddingtonia flagrans (D. flagrans)* having all of the identifying characteristics of the strain deposited under Accession No. V16/019156 at National Measurement Institute, 1/153 Bertie Street, Port Melbourne, Victoria 3207, Australia referred to herein as "IAH 1297".

2. The composition of claim 1, further comprising one or more parasitic nematode reducing material(s).

3. A composition for use in a method of controlling the spread of a parasitic nematode in a grazing animal comprising a biologically pure isolate of the micro-organism *Duddingtonia flagrans (D. flagrans)* having all of the identifying characteristics of the strain deposited under Accession No. V16/019156 at National Measurement Institute, 1/153 Bertie Street, Port Melbourne, Victoria 3207, Australia referred to herein as "IAH 1297".

4. The composition for the use according to claim 3, wherein the composition is in the form of a spore concentrate including mycelium of the D. *flagrans,* and/or spores of the D. *flagrans,* wherein preferably the spores are chlamydospores.

5. The composition for the use according to claim 3 or claim 4, wherein said *D*. *flagrans* is present in the form of spores and wherein said composition comprises a viable count of *D*. *flagrans* spores of at least about 1 × 10² viable spores per gram of composition, a viable count of *D*. *flagrans* spores in the range of 1 × 10² to 1 × 10¹⁰ viable spores per gram of composition, a viable count of D. *flagrans* spores in the range of 1 × 10³ to 1 × 10⁸ viable spores per gram of composition, or a viable count of *D. flagrans* spores in the range of 1 × 10⁴ to 1 × 10⁶ viable spores per gram of composition.

6. The composition for the use according to claim 5, wherein the composition is formulated for administration to a grazing animal and wherein the composition is in unit dosage form that provides the amount of 1 × 10³ to 5 × 10⁸ viable *D*. *flagrans* spores per Kg bodyweight of the grazing animal per day.

7. The composition for the use according to any one of claims 3 to 6, further comprising one or more parasitic nematode reducing material(s).

8. The composition for the use according to claim 7, wherein the one or more materials is each selected from the group consisting of a tannin, one or more anthelmintics, one or more other nematophagous fungi, copper oxide wire particles (COWP), a mineral, diatomaceous earth, a herb, an agricultural by-product, one or more bacteria and a vaccine.

9. The composition for the use according to of claim 8, wherein the one or more material(s) is copper oxide wire particles (COWP), wherein the composition provides an amount of elemental copper in the range of 0.05 to 0.15 g per Kg bodyweight of the grazing animal per day or an amount of elemental copper of about 0.085 g per Kg bodyweight of the grazing animal per day.

10. The composition for the use according to any one of claims 3 to 9, wherein the composition is to be administered to a grazing animal for at least one week, wherein the composition is to be administered for 2 to 8 weeks, wherein the composition is to be administered for 8 weeks or more, or wherein the composition is to be administered for the life of the animal.

11. The composition for the use according to any one of claims 3 to 10, wherein the composition is to be administered one to four times daily.

12. The composition for the use according to any one of claims 3 to 11, wherein the composition is to be administered as part of an integrated parasite management program (IPM) for controlling the spread of a parasitic nematode to a grazing animal, wherein the IPM program comprises:
(a) treating the grazing animal with an anthelmintic effective against the parasitic nematode;
(b) optionally transferring the grazing animal to a clean pasture which is minimally infected by or free of larvae of the parasitic nematode; and
(c) administration of the composition to the grazing animal.

13. The composition for the use according to any one of claims 3 to 12, wherein
(a) the parasitic nematode is one or more of a nematode of the *Stronglyus* spp., *Cyathostomum* spp., *Triodontophorous* spp., *Trichonema* spp., *Oesophagodontus* spp., *Gyalocephalus* spp., *Cylicocylus* spp., *Cylicodonotophorus* spp., *Cylicostephanus* spp., *Gongylonema* spp., *Habronema* spp., Mecistocirrus spp., *Oxyuris* spp., *Parascaris* spp., *Skrjabinema* spp., *Strongyloides* spp., *Toxocara* spp., *Dictyocaulis* spp., *Muellerius* spp., *Protostrongylus* spp., *Onchocerca* spp., *Parafilaria* spp., *Setaria* spp., *Stephanofilaria* spp., *Thelazia* spp., *Trichostrongylus* spp., *Cooperia* spp., *Bunostomum* spp., *Teladosagia* spp., *Oesophagostomum* spp., *Nematodirus* spp., *Haemonchus* spp., *Chabertia* spp., *Trichuris* spp., and/or *Ostertagia* spp; or
(b) wherein the parasitic nematode is one or more of Barber's Pole Worm or Wire Worm (*Haemonchus* spp.), Hairworm or Stomach Hairworm (*Trichostrongylus* spp), Intestinal Worm or Small Intestinal Worm (*Cooperia* spp.) and Hookworm (*Bunostomum* spp.), Black Scour Worm (*Trichostrongylus* spp.), Small and Medium Brown Stomach Worm (*Teladosagia* (*Ostertagia)* spp.), Nodule Worm (*Oesophagostomum* spp.), Stomach Hair Worm (*Trichostrongylus* spp.), Thread Worm (*Strongyloides* spp.), Large Mouthed Bowel Worm (*Chabertia* spp.), Whipworm (*Trichuris* spp.), Thin Necked Intestinal Worm or Thread Necked Worm (*Nematodirus* spp.), Redworms or Large Strongyles (including *Strongylus* spp., *Triodontophorus* spp.and *Oesophagodontus* spp.) and/or Cyathostomes / Small Strongyles (including *Cyathostomum* spp., *Trichonema* spp., *Gyalocephalus* spp., *Cylicocyclus* spp., *Cylicodontophorus* spp. and *Cylicostephanus* spp.), Ascarids (*Parascaris* spp.) and/or Pinworms (*Oxyuris* spp.).

14. The composition for the use according to any one of claims 3 to 13, wherein the parasitic nematode has resistance or multi-chemical resistance to one or more anthelmintics.

15. A method of producing a biologically pure isolate of the micro-organism *Duddingtonia flagrans* (*D. flagrans*) by fermentation the method comprising fermenting an isolate of *D. flagrans* having all of the identifying characteristics of the strain deposited under Accession No. V16/019156 at National Measurement Institute, 1/153 Bertie Street, Port Melbourne, Victoria 3207, Australia referred to herein as "IAH 1297" on a solid substrate, wherein the solid substrate is a moistened grain, legume or oilseed.

16. The method of claim 15, wherein:
(a) the grain is rye, millet, rice, barley, wheat, sorghum, triticale or oat;
(b) the legume is lentil or lupin; or
(c) the oilseed soybean, canola, safflower, sunflower or sesame seed.

## Patentansprüche

1. Zusammensetzung, umfassend ein biologisch reines Isolat des Mikroorganismus *Duddingtonia flagrans* (*D. flagrans*), das alle identifizierenden Eigenschaften des unter der Zugangsnummer V16/019156 beim National Measurement Institute 1/153 Bertie Street, Port Melbourne, Victoria 3207, Australien hinterlegten Stamms aufweist, der hierin als "IAH 1297" bezeichnet wird.

2. Zusammensetzung nach Anspruch 1, des Weiteren umfassend eine oder mehrere Substanz(en), die parasitische Nematoden reduziert/reduzieren.

3. Zusammensetzung zur Verwendung bei einem Verfahren zur Bekämpfung der Übertragung von parasitischen Nematoden bei einem Weidetier, umfassend ein biologisch reines Isolat des Mikroorganismus *Duddingtonia flagrans* (*D. flagrans*), das alle identifizierenden Eigenschaften des unter der Zugangsnummer V16/019156 beim National Measurement Institute, 1/153 Bertie Street, Port Melbourne, Victoria 3207, Australien hinterlegten Stamms aufweist, der hierin als "IAH 1297" bezeichnet wird.

4. Zusammensetzung zur Verwendung nach Anspruch 3, wobei die Zusammensetzung in Form eines Sporenkonzentrats ist, das Myzel von *D. flagrans* und/oder Sporen von *D. flagrans* umfasst, wobei die Sporen bevorzugt Chlamydosporen sind.

5. Zusammensetzung zur Verwendung nach Anspruch 3 oder Anspruch 4, wobei *D. flagrans* in Form von Sporen vorliegt und wobei die Zusammensetzung eine Anzahl lebensfähiger Sporen von *D. flagrans* (viable count of *D. flagrans* spores) von mindestens etwa 1 × 10² lebensfähigen Sporen pro Gramm Zusammensetzung, eine Anzahl lebensfähiger Sporen von *D. flagrans* im Bereich von 1 × 10² bis 1 × 10¹⁰ lebensfähiger Sporen pro Gramm Zusammensetzung, eine Anzahl lebensfähiger Sporen von *D. flagrans* im Bereich von 1 × 10³ bis 1 × 10⁸ lebensfähiger Sporen pro Gramm Zusammensetzung oder eine Anzahl lebensfähiger Sporen von *D. flagrans* im Bereich von 1 × 10⁴ bis 1 × 10⁶ lebensfähiger Sporen pro Gramm Zusammensetzung umfasst.

6. Zusammensetzung zur Verwendung nach Anspruch 5, wobei die Zusammensetzung für die Verabreichung an Weidetiere formuliert ist, und wobei die Zusammensetzung in einer Einheitsdosisform ist, die 1 × 10³ bis 5 × 10⁸ lebensfähiger *D. flagrans-Sporen* pro kg Körpergewicht des Weidetieres pro Tag bereitstellt.

7. Zusammensetzung zur Verwendung nach einem der Ansprüche 3 bis 6, des Weiteren umfassend eine oder mehrere Substanz(en), die parasitische Nematoden reduziert/reduzieren.

8. Zusammensetzung zur Verwendung nach Anspruch 7, wobei die eine oder die mehreren Substanz(en) jeweils ausgewählt ist/sind aus der Gruppe bestehend aus einem Tannin, einen oder mehreren Anthelmintika, einem oder mehreren anderen nematophagen Pilzen, Kupferoxid-Drahtpartikel (copper oxide wire particles; COWP), einem Mineral, Diatomeenerde, einem Kraut, einem landwirtschaftlichen Nebenprodukt, einem oder mehreren Bakterium/Bakterien und einem Impfstoff.

9. Zusammensetzung zur Verwendung nach Anspruch 8, wobei die eine oder die mehreren Substanz(en) Kupferoxid-Drahtpartikel (COWP) ist/sind, wobei die Zusammensetzung eine Menge an elementarem Kupfer im Bereich von 0,05 bis 0,15 g pro kg Körpergewicht des Weidetieres pro Tag oder eine Menge an elementarem Kupfer von etwa 0,085 g pro kg Körpergewicht des Weidetieres pro Tag bereitstellt.

10. Zusammensetzung zur Verwendung nach einem der Ansprüche 3 bis 9, wobei die Zusammensetzung einem Weidetier mindestens eine Woche lang zu verabreichen ist, wobei die Zusammensetzung 2 bis 8 Wochen lang zu verabreichen ist, wobei die Zusammensetzung 8 Wochen lang oder länger zu verabreichen ist oder wobei die Zusammensetzung dem Tier für die Dauer seines Lebens zu verabreichen ist.

11. Zusammensetzung zur Verwendung nach einem der Ansprüche 3 bis 10, wobei die Zusammensetzung einmal bis viermal täglich zu verabreichen ist.

12. Zusammensetzung zur Verwendung nach einem der Ansprüche 3 bis 11, wobei die Zusammensetzung als Teil eines integrierten Parasitenmanagementprogramms (integrated parasite management program; IPM) zur Bekämpfung der Übertragung eines parasitischen Nematoden an ein Weidetier zu verabreichen ist, wobei das IPM-Programm umfasst:
(a) Behandeln des Weidetieres mit einem gegen den parasitischen Nematoden wirksamen Anthelmintikum;
(b) gegebenenfalls Überführen des Weidetieres auf eine saubere Weide, die minimal mit Larven des parasitischen Nematoden infiziert oder frei von diesen ist; und
(c) Verabreichung der Zusammensetzung an das Weidetier.

13. Zusammensetzung zur Verwendung nach einem der Ansprüche 3 bis 12, wobei
(a) der parasitische Nematode ein oder mehrere Nematode(n) von *Stronglyus* spp., *Cyathostomum* spp., *Triodontophorous* spp., *Trichonema* spp., *Oesophagodontus* spp., *Gyalocephalus* spp., *Cylicocylus* spp., *Cylicodonotophorus* spp., *Cylicostephanus* spp., *Gongylonema* spp., *Habronema* spp., *Mecistocirrus* spp., *Oxyuris* spp., *Parascaris* spp., *Skrjabinema* spp., *Strongyloides* spp., *Toxocara* spp., *Dictyocaulis* spp., *Muellerius* spp., *Protostrongylus* spp., *Onchocerca* spp., *Parafilaria* spp., *Setaria* spp., *Stephanofilaria* spp., *Thelazia* spp., *Trichostrongylus* spp., *Cooperia* spp., *Bunostomum* spp., *Teladosagia* spp., *Oesophagostomum* spp., *Nematodirus* spp., *Haemonchus* spp., *Chabertia* spp., *Trichuris* spp., und/oder *Ostertagia* spp. ist; oder
(b) wobei der parasitische Nematode einer oder mehrerer von Barber's Pole Worm oder Wire Worm (*Haemonchus* spp.), Hairworm oder Stomach Hairworm (*Trichostrongylus* spp.), Intestinal Worm oder Small Intestinal Worm (*Cooperia* spp.) und Hookworm (*Bunostomum* spp.), Black Scour Worm (*Trichostrongylus* spp.), Small und Medium Brown Stomach Worm (*Teladosagia* (*Ostertagia*) spp.), Nodule Worm (*Oesophagostomum* spp.), Stomach Hair Worm (*Trichostrongylus* spp.), Thread Worm (*Strongyloides* spp.), Large Mouthed Bowel Worm (*Chabertia* spp.), Whipworm (*Trichuris* spp.), Thin Necked Intestinal Worm oder Thread Necked Worm (*Nematodirus* spp.), Redworms oder Large Strongyles (einschließlich *Strongylus* spp., *Triodontophorus* spp. und *Oesophagodontus* spp.) und/oder Cyathostomes / Small Strongyles (einschließlich *Cyathostomum* spp., *Trichonema* spp., *Gyalocephalus* spp., *Cylicocyclus* spp., *Cylicodontophorus* spp. und *Cylicostephanus* spp.), Ascarids (*Parascaris* spp.) und/oder Pinworms (*Oxyuris* spp.) ist.

14. Zusammensetzung zur Verwendung nach einem der Ansprüche 3 bis 13, wobei der parasitische Nematode Resistenz oder multi-chemische Resistenz gegenüber einem oder mehreren Anthelmintika aufweist.

15. Verfahren zur Herstellung eines biologisch reinen Isolats des Mikroorganismus *Duddingtonia flagrans* (*D. flagrans*) durch Fermentation, wobei das Verfahren das Fermentieren eines Isolats von *D. flagrans* umfasst, das alle identifizierenden Eigenschaften des unter der Zugangsnummer V16/019156 beim National Measurement Institute, 1/153 Bertie Street, Port Melbourne, Victoria 3207, Australien hinterlegten Stamms aufweist, der hierin als "IAH 1297" bezeichnet wird, auf einem festen Substrat, wobei das feste Substrat ein angefeuchtetes Getreide, eine angefeuchtete Hülsenfrucht oder eine angefeuchtete Ölsaat ist.

16. Verfahren nach Anspruch 15, wobei:
(a) das Getreide Roggen, Hirse, Reis, Gerste, Weizen, Sorghum, Triticale oder Hafer ist;
(b) die Hülsenfrucht Linse oder Lupine ist; oder
(c) die Ölsaat Soja-, Raps-, Saflor-, Sonnenblumen- oder Sesamsaat ist.

## Revendications

1. Composition comprenant un isolat biologiquement pur du microorganisme *Duddingtonia flagrans* (*D. flagrans*) ayant toutes les caractéristiques identifiantes de la souche déposée avec le numéro d'accès V16/019156 au National Measurement Institute, 1/153 Bertie Street, Port Melbourne, Victoria 3207, Australie, à laquelle il est fait référence ici en tant que "IAH 1297".

2. Composition selon la revendication 1, comprenant en outre un ou plusieurs matériaux réduisant les nématodes parasites.

3. Composition pour une utilisation dans une méthode de contrôle de la propagation d'un nématode parasite chez un animal de pâturage comprenant un isolat biologiquement pur du microorganisme *Duddingtoniaflagrans* (*D. flagrans*) ayant toutes les caractéristiques identifiantes de la souche déposée avec le numéro d'accès V16/019156 au National Measurement Institute, 1/153 Bertie Street, Port Melbourne, Victoria 3207, Australie, à laquelle il est fait référence ici en tant que "IAH 1297".

4. Composition pour une utilisation selon la revendication 3, ladite composition étant sous la forme d'un concentré de spores contenant le mycélium de *D. flagrans,* et/ou de spores de *D. flagrans,* dans laquelle de préférence les spores sont des chlamydospores.

5. Composition pour une utilisation selon la revendication 3 ou la revendication 4, dans laquelle ledit *D. flagrans* est présent sous la forme de spores, et ladite composition comprenant un nombre viable de spores de *D. flagrans* d'au moins environ 1 × 10² spores viables par gramme de composition, un nombre viable de spores de *D. flagrans* dans la plage allant de 1 × 10² à 1 × 10¹⁰ spores viables par gramme de composition, un nombre viable de spores de *D. flagrans* dans la plage allant de 1 × 10³ à 1 × 10⁸ spores viables par gramme de composition, ou un nombre viable de spores de *D. flagrans* dans la plage allant de 1 × 10⁴ à 1 × 10⁶ spores viables par gramme de composition.

6. Composition pour une utilisation selon la revendication 5, ladite composition étant formulée pour une administration à un animal de pâturage, et ladite composition étant sous une forme posologique unitaire qui apporte la quantité de 1 × 10³ à 5 × 10⁸ spores viables de *D. flagrans* par kg de poids corporel de l'animal de pâturage par jour.

7. Composition pour une utilisation selon l'une quelconque des revendications 3 à 6, comprenant en outre un ou plusieurs matériaux réduisant les nématodes parasites.

8. Composition pour une utilisation selon la revendication 7, dans laquelle chacun desdits un ou plusieurs matériaux est choisi dans le groupe constitué par un tanin, un ou plusieurs anthelmintiques, un ou plusieurs autres champignons nématophages, des particules de fil d'oxyde de cuivre (COWP), un minéral, de la terre de diatomées, une herbe, un sous-produit agricole, une ou plusieurs bactéries, et un vaccin.

9. Composition pour une utilisation selon la revendication 8, dans laquelle le ou les matériaux sont des particules de fil d'oxyde de cuivre (COWP), ladite composition apportant une quantité de cuivre élémentaire dans la plage allant de 0,05 à 0,15 g par kg de poids corporel de l'animal de pâturage par jour ou une quantité de cuivre élémentaire d'environ 0,085 g par kg de poids corporel de l'animal de pâturage par jour.

10. Composition pour une utilisation selon l'une quelconque des revendications 3 à 9, ladite composition étant destinée à être administrée à un animal de pâturage pendant au moins une semaine, ladite composition étant destinée à être administrée pendant 2 à 8 semaines, ladite composition étant destinée à être administrée pendant 8 semaines ou plus, ou ladite composition étant destinée à être administrée pendant toute la vie de l'animal.

11. Composition pour une utilisation selon l'une quelconque des revendications 3 à 10, ladite composition étant destinée à être administrée une à quatre fois par jour.

12. Composition pour une utilisation selon l'une quelconque des revendications 3 à 11, ladite composition étant destinée à être administrée en tant que partie d'un programme de gestion intégrée des parasites (IPM) pour contrôler la propagation d'un nématode parasite chez un animal de pâturage, dans laquelle le programme IPM comprend :
(a) le traitement de l'animal de pâturage avec un anthelmintique efficace contre le nématode parasite ;
(b) éventuellement le transfert de l'animal de pâturage sur une pâture propre qui est minimalement infectée par ou exempte de larves du nématode parasite ; et
(c) l'administration de la composition à l'animal de pâturage.

13. Composition pour une utilisation selon l'une quelconque des revendications 3 à 12, dans laquelle
(a) le nématode parasite est un ou plusieurs nématodes de *Stronglyus* spp., *Cyathostomum* spp., *Triodontophorous* spp., *Trichonema* spp., *Oesophagodontus* spp., *Gyalocephalus* spp., *Cylicocylus* spp., *Cylicodonotophorus* spp., *Cylicostephanus* spp., *Gongylonema* spp., *Habronema* spp., *Mecistocirrus* spp., *Oxyuris* spp., *Parascaris* spp., *Skrjabinema* spp., *Strongyloides* spp., *Toxocara* spp., *Dictyocaulis* spp., *Muellerius* spp., *Protostrongylus* spp., *Onchocerca* spp., *Parafilaria* spp., *Setaria* spp., *Stephanofilaria* spp., *Thelazia* spp., *Trichostrongylus* spp., *Cooperia* spp., *Bunostomum* spp., *Teladosagia* spp., *Oesophagostomum* spp., *Nematodirus* spp., *Haemonchus* spp., *Chabertia* spp., *Trichuris* spp., et/ou *Ostertagia* spp ; ou
(b) le nématode parasite est un ou plusieurs parmi Barber's Pole Worm ou Wire Worm (*Haemonchus* spp.), Hairworm ou Stomach Hairworm *(Trichostrongylus* spp.), Intestinal Worm ou Small Intestinal Worm (*Cooperia* spp.) et Hookworm (*Bunostomum* spp.), Black Scour Worm (*Trichostrongylus* spp.), Small et Medium Brown Stomach Worm (*Teladosagia* (*Ostertagia*) spp.), Nodule Worm (*Oesophagostomum* spp.), Stomach Hair Worm (*Trichostrongylus* spp.), Thread Worm (*Strongyloides* spp.), Large Mouthed Bowel Worm (*Chabertia* spp.), Whipworm (*Trichuris* spp.), Thin Necked Intestinal Worm ou Thread Necked Worm (*Nematodirus* spp.), Redworms ou Large Strongyles (y compris *Strongylus* spp., *Triodontophorus* spp. ou *Oesophagodontus* spp.) et/ou *Cyathostomes* / Small Strongyles (y compris *Cyathostomum* spp., *Trichonema* spp., *Gyalocephalus* spp., *Cylicocyclus* spp., *Cylicodontophorus* spp. et *Cylicostephanus* spp.), Ascarids (*Parascaris* spp.) et/ou Pinworms (*Oxyuris* spp.).

14. Composition pour une utilisation selon l'une quelconque des revendications 3 à 13, dans laquelle le nématode parasite a une résistance ou une multirésistance chimique à un ou plusieurs anthelmintiques.

15. Méthode de production d'un isolat biologiquement pur du microorganisme *Duddingtonia flagrans* (*D. flagrans*) par fermentation, la méthode comprenant la fermentation d'un isolat de *D. flagrans* ayant les caractéristiques identifiantes de la souche déposée avec le numéro d'accès V16/019156 au National Measurement Institute, 1/153 Bertie Street, Port Melbourne, Victoria 3207, Australie, à laquelle il est fait référence ici en tant que "IAH 1297", sur un substrat solide, dans laquelle le substrat solide est une céréale, une légumineuse ou une graine oléagineuse humidifiée.

16. Méthode selon la revendication 15, dans laquelle :
(a) la céréale est le seigle, le millet, le riz, l'orge, le blé, le sorgho, la triticale ou l'avoine ;
(b) la légumineuse est la lentille ou le lupin ; ou
(c) la graine oléagineuse est une graine de soja, canola, carthame, tournesol ou sésame.
